# EUROPEAN PATENT APPLICATION

(11) **EP 1 764 413 A1**
(43) Date of publication of application: **21.03.2007**
(21) Application number: 05844884.6
(22) Date of filing: 28.12.2005
(51) Int. Cl.: C12N 15/09, A01K 67/027, A61K 38/00, A61P 19/10, A61P 43/00, C07K 14/435, C07K 16/18, C12P 21/02

(54) **PROTEIN PREPARATION CONTAINING S1-5**

(30) Priority: 01.07.2005 JP 2005012251
(71) Applicant: Locomogene, Inc., Tokyo 105-0001 (JP)
(72) Inventor: NAKAJIMA, Toshihiro, Kohoku Garden Hills A-503, Yokohama-shi, Kanagawa 224-0001 (JP); YAGISHITA, Noako, Cosmo Konandai 507, Yokohama-shi, Kanagawa 234-0053 (JP); AMANO, Tetsuya, Kawasaki-shi, Kanagawa 214-0037 (JP)
(74) Representative: Harding, Charles Thomas
(86) International application number: PCT/JP2005/024137
(87) International publication number: WO 2007/004325

(57) **Abstract**

The present inventors discovered that knockout mice whose S1-5 gene function is lost develop age-related diseases or symptoms. In such knockout mice, bone mineral content, bone mineral density, and bone strength were found to be decreased, and the number of osteoclasts in bone tissues was found to be increased. Analysis of osteoclast-forming ability using bone marrow cells derived from the knockout mice revealed that osteoclast-forming ability is enhanced and osteoclasts are larger in the knockout mice than in wildtype mice. When purified S1-5 protein was added to this *in vitro* system, osteoclast-forming ability was inhibited. Furthermore, administration of purified S1-5 protein to osteoporotic model mice showed that this protein has the effect of improving osteoporosis. The above findings demonstrate that S1-5 protein is useful for treating and preventing age-related diseases such as osteoporosis.

## Description

### Technical Field

The present invention relates to non-human knockout animals whose S1-5 gene has been made defective and which have developed age-related diseases or symptoms, and methods for producing such animals. The present invention also relates to methods of screening for preventive or therapeutic agents for age-related diseases or symptoms, in which the methods comprise administering candidate substances to the above-mentioned animals. Furthermore, the present invention relates to cells isolated from the non-human knockout animals, and their uses, as well as S1-5 proteins and their uses.

### Background Art

Rheumatoid arthritis (hereinafter abbreviated as RA) is a systemic chronic inflammatory disease that shows abnormal proliferation of synovial tissues in the joints. Synovial cells are fibroblast-like cells that form layers one to six of the epithelial-like layers in the synovial membrane in joints, and are thought to provide proteoglycans and hyaluronic acids to the synovial fluid. Synovial tissue proliferates in the joints of RA patients, causing various symptoms to be observed, including multilayered structures and the invasion of inflammatory cells.

In the process of conducting research to elucidate the molecular mechanisms behind the development and progress of RA, the present inventors discovered a gene strongly expressed in the synovial tissues of RA patients. The protein encoded by this gene was named synoviolin, after the synovial cells which are the tissues in which the protein is expressed (Patent Document 1).

From biochemical binding experiments on this protein, the present inventors elucidated the presence of S1-5 (also known as EFEMP-1, FBNL, FBLN-3, etc), which is a synoviolin binding factor. S1-5, isolated by the present inventors, is the first protein to be isolated as a synoviolin binding factor.

S1-5 was isolated as a gene overexpressed in human diploid fibroblasts (Leucka-Czernik, B. et al., Mol. Cell. Biol. 15: 120-128, 1995). In terms of structure, epidermal growth factor (EGF)-like domain and fibrin-like domain, which promote DNA synthesis (cell growth) were discovered in S1-5. Further, there are recent reports that S1-5 mutation is related to Malattia Leventinese (ML) and Doyne honeycomb retinal dystrophy (DHRD) (Non-Patent Document 1).

However, the detailed function of S1-5 is unknown, and the phenotype of individuals made S1-5 deficient is not clear.
[Patent Document 1] WO02/052007 pamphlet
[Non-Patent Document 1] Stone, E.M. et al., Nature Genetics 22: 199-202, 1999

### Disclosure of the Invention

### Problems to Be Solved by the Invention

An objective of the present invention is to provide non-human knockout animals whose S1-5 gene has been made defective and which have developed an age-related disease or symptom, and methods for producing such animals. A further objective of the present invention is to provide methods of screening for preventive or therapeutic agents for age-related diseases or symptoms, in which the methods comprise administering candidate substances to the above-mentioned animals. Another objective of the present invention is to provide cells isolated from the non-human knockout animals, and uses thereof, as well as S1-5 proteins and uses thereof.

### Means to Solve the Problems

Upon dedicated research to achieve the above-mentioned objective, the present inventors discovered that knockout mice whose S1-5 gene function is lost will develop age-related diseases or symptoms. Such knockout mice were found to have decreased bone mineral content, bone mineral density and bone strength; and an increased number of osteoclasts in their bone tissues. *In vitro* examination of osteoclast-forming ability using bone marrow cells derived from these knockout mice revealed enhanced osteoclast-forming ability and an increase in osteoclast size compared to using cells derived from wildtype mice. Adding purified S1-5 protein to this *in vitro* system inhibited osteoclast-forming ability and reduced osteoclast size. Furthermore, the administration of purified S1-5 protein to osteoporotic model mice and rats showed that S1-5 protein has the effect of improving osteoporosis.

The above findings demonstrate that S1-5 protein is useful for treating and preventing age-related diseases such as bone deformation, osteoporosis, Paget's disease of bone, hyperparathyroidism, decreased bone mineral density, cancellous transformation of cortical bone, hair loss, tissue injury or necrosis, tumor, breast hypertrophy, ascites, anemia, bleeding, aging of skin (blotches, dullness of skin, flabby skin, fine wrinkles, moles, etc.), and aging of nails.

More specifically, the present invention is as follows:
[1] a non-human knockout animal showing an age-related disease or symptom, wherein all or a part of the S1-5 gene function is lost;
[2] the animal of [1], wherein the loss of all or a part of the S1-5 gene function is due to a disruption or mutation of the S1-5 gene;
[3] the animal of [1], wherein the age-related disease or symptom is at least one selected from the group consisting of bone deformation, osteoporosis, Paget's disease of bone, hyperparathyroidism, decreased bone mineral density, cancellous transformation of cortical bone, hair loss, tissue injury or necrosis, tumor, breast hypertrophy, ascites, anemia, bleeding, aging of skin, and aging of nails;
[4] the animal of [1], wherein the animal is selected from the group consisting of zebrafish, mice, rats, guinea pigs, rabbits, chickens, pigs, sheep, goats, dogs, cattle, monkeys, and chimpanzees;
[5] a cell isolated from the non-human knockout animal of any one of [1] to [4];
[6] the cell of [5], which is an osteoclast, keratinocyte epithelial cell, blood cell, cancer cell, bone marrow cell, fibroblast, vascular endothelial cell, dermal cell, muscle cell, nerve cell, lymphocyte, vascular smooth muscle cell, synoviocyte, hair papilla cell, hepatocyte, pigment cell, adipocyte, uterine endothelial cell, or alveolar epithelial cell;
[7] a method for producing a non-human knockout animal that develops an age-related disease, wherein the method comprises causing the loss of all or a part of the S1-5 gene function;
[8] the method of [7], wherein the loss of all or a part of the S1-5 gene function is caused by a disruption or mutation of the S1-5 gene;
[9] the method of [7], wherein the age-related disease or symptom is at least one selected from the group consisting of bone deformation, osteoporosis, Paget's disease of bone, hyperparathyroidism, bone mineral density, cancellous transformation of cortical bone, hair loss, tissue injury or necrosis, tumor, breast hypertrophy, ascites, anemia, bleeding, aging of skin, and aging of nails;
[10] the method of [7], wherein the animal is selected from the group consisting of zebrafish, mice, rats, guinea pigs, rabbits, chickens, pigs, sheep, goats, dogs, cattle, monkeys, and chimpanzees;
[11] a method of screening for preventive or therapeutic agents for an age-related disease or symptom, wherein the method comprises administering a candidate substance for said preventive or therapeutic agent to the non-human knockout animal of any one of [1] to [4];
[12] a method of screening for preventive or therapeutic agents for an age-related disease or symptom, wherein the method comprises contacting a candidate substance for said preventive or therapeutic agent with cells isolated from the non-human knockout animal of any one of [1] to [4];
[13] the method of [12], wherein the cells are osteoclasts, keratinocyte epithelial cells, blood cells, cancer cells, bone marrow cells, fibroblasts, vascular endothelial cells, dermal cells, muscle cells, nerve cells, lymphocytes, vascular smooth muscle cells, synoviocytes, hair papilla cells, hepatocytes, pigment cells, adipocytes, uterine endothelial cells, or alveolar epithelial cells;
[14] the method of any one of [11] to [13], wherein the age-related disease or symptom is at least one selected from the group consisting of bone deformation, osteoporosis, Paget's disease of bone, hyperparathyroidism, decreased bone mineral density, cancellous transformation of cortical bone, hair loss, tissue injury or necrosis, tumor, breast hypertrophy, ascites, anemia, bleeding, aging of skin, and aging of nails;
[15] a method of screening for agents that inhibit osteoclast function, wherein the method comprises contacting a candidate substance for said agent that inhibits osteoclast function with osteoclasts derived from the non-human knockout animal of any one of [1] to [4];
[16] an isolated protein, which is any one of (a) to (d):
   (a) a protein comprising the amino acid sequence of SEQ ID NO: 2 or 4;
   (b) a protein encoded by a DNA comprising a coding region of the nucleotide sequence of SEQ ID NO: 1 or 3;
   (c) a protein comprising an amino acid sequence with one or more amino acid substitutions, deletions, insertions, and/or additions in the amino acid sequence of SEQ ID NO: 2 or 4, wherein the protein is functionally equivalent to a protein comprising the amino acid sequence of SEQ ID NO: 2 or 4; and
   (d) a protein encoded by a DNA that hybridizes under stringent conditions with a DNA comprising the nucleotide sequence of SEQ ID NO: 1 or 3, wherein the protein is functionally equivalent to a protein comprising the amino acid sequence of SEQ ID NO: 2 or 4;
[17] a partial peptide of the protein of [16];
[18] the peptide of [17], which comprises the amino acid sequence of SEQ ID NO: 6;
[19] a preventive or therapeutic agent for an age-related disease or symptom, wherein the agent comprises the protein of [16], or the peptide of [17] or [18];
[20] the preventive or therapeutic agent of [19], wherein the age-related disease or symptom is at least one selected from the group consisting of bone deformation, osteoporosis, Paget's disease of bone, hyperparathyroidism, decreased bone mineral density, cancellous transformation of cortical bone, hair loss, tissue injury or necrosis, tumor, breast hypertrophy, ascites, anemia, bleeding, aging of skin, and aging of nails;
[21] an agent that inhibits osteoclast function, wherein the agent comprises the protein of [16], or the peptide of [17] or [18]; and
[22] an antibody that binds to the protein of [16], or to the peptide of [17] or [18].

### Brief Description of the Drawings

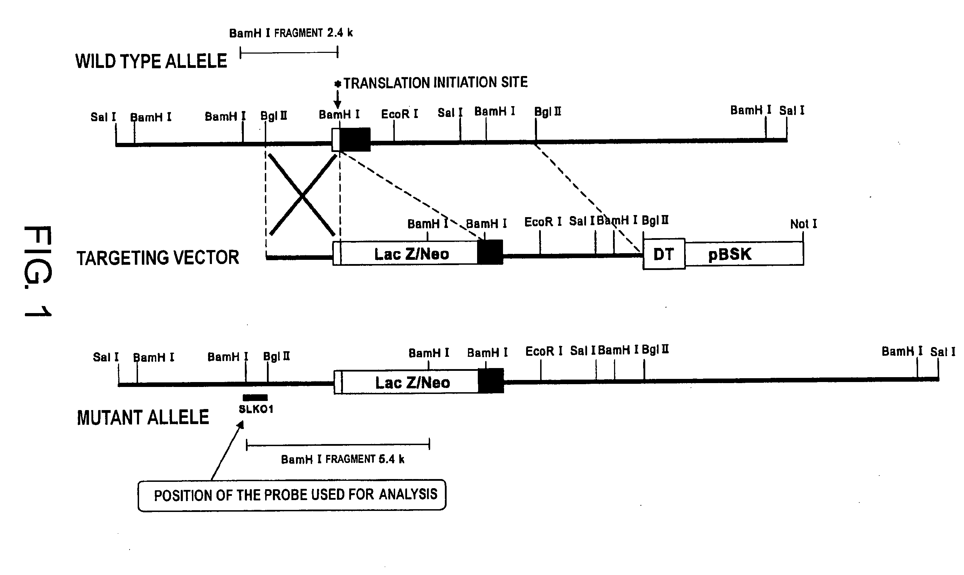

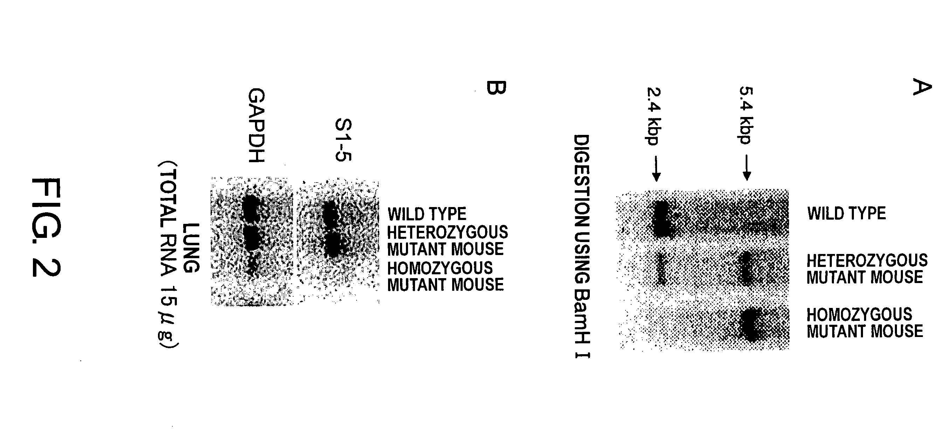

Fig. 1 shows the structure of a targeting vector that makes the S1-5 gene defective. The lacZ gene was introduced into the translation initiation site of the mouse S1-5 gene fragment (the ATG codon that is translated into the first methionine; indicated by a *), and a neomycin resistance (Neo) gene was introduced as a positive selection marker gene. In addition, diphtheria toxin A (DT-A) gene was linked as a negative marker. In individuals that undergo homologous recombination, the S 1-5 gene becomes defective and β-galactosidase is expressed instead. LacZ staining utilizing this enzyme's activity enables detection of S1-5 gene expression from the promoter. The figure also shows the position of the probe used in Southern blot analysis to confirm genotype.
Fig. 2 is photographs showing the results of analyzing the genotype of S 1-5 gene mutant mice. (A) shows the results of subjecting DNA extracted from the tail of the mice and digested with BamHI to Southern blot analysis using the probe indicated in Fig. 1. (B) shows the results of Northern blot analysis.
Fig. 3A is a set of photographs indicating the phenotype of a 26-month old S1-5 knockout mouse. S1-5-/-male (Animal No: 1101). Kyphosis, hair loss, skin injuries on the face, necrosis of the nails, breast hypertrophy, as well as ascites and liver tumors when autopsied.
Fig. 3B is a set of photographs indicating the phenotype of a 26-month old S1-5 knockout mouse. S1-5-/-female (Animal No: 1097). Kyphosis, difficulty achieving hemostasis, low hematocrit value, necrosis of the nails, blood clotting in the ocular fundus, as well as uterine hypertrophy and blood clotting in the adipose tissues when autopsied.
Fig. 3C shows photographs indicating the phenotype of a 26-month old S1-5 knockout mouse. S1-5-/-female (Animal No: 1103). Kyphosis.
Fig. 4 is a series of X-ray photographs showing bone deformation in wild type mice and S1-5 knockout mice.
Fig. 5 is a series of X-ray photographs showing bone deformation in wild type mice and S1-5 knockout mice.
Fig. 6 is a series of X-ray photographs showing bone deformation in S1-5 knockout mice.
Fig. 7: (A) shows photographs indicating the angle of backbone curvature in S1-5 knockout mice; and (B) summarizes the proportion of animals developing kyphosis in different age groups in terms of weeks, based on the angle of backbone curvature of the S1-5 knockout mice, which is accomplished by taking X-ray photographs in each age group, measuring the angle of curvature of the spine, and defining the development of kyphosis to be an angle of 95° or less.
Fig. 8 shows graphs that summarize the angle of backbone curvature of S1-5 knockout mice by age in terms of weeks.
Fig. 9 shows a photograph and graphs indicating the results of measuring the bone mineral density of the spine (thoracic vertebrae (10-12) lumbar vertebrae (1-3)) in S1-5 knockout mice. SB1-855 and such indicate Animal Nos.; the numbers in the bar graph indicate the age in weeks; and the numbers in parentheses indicate the angle of kyphosis.
Fig. 10 shows the results of measuring the bone mineral density of the spine (thoracic vertebrae (10-12) lumbar vertebrae (1-3)) in S1-5 knockout mice. Each bar in the graph represents the average value for each group, and the number in the bars indicate "n".
Fig. 11 shows a photograph and graphs indicating the result of measuring the bone mineral density of the femora of S1-5 knockout mice. SB1-855 and such indicate Animal Nos., and the numbers on the bar indicate the age in weeks.
Fig. 12 shows the results of measuring the bone mineral density of the femora of S1-5 knockout mice. The numbers in the bars indicate "n".
Fig. 13 shows a set of micro CT images of the spine (thoracic vertebrae (10-12)) of S1-5 knockout mice. SB1-855, LB594 and such indicate Animal Nos.
Fig. 14 shows graphs indicating the results of pQCT measurements on S1-5 knockout mice.
Fig. 15 shows graphs indicating the urine NTx values of S1-5 knockout mice. For each individual, a one-week pooled urine sample was measured.
Fig. 16 shows graphs that summarize the urine NTx values of S1-5 knockout mice by age in terms of weeks.
Fig. 17 is a set of bone tissue photographs of stained hard tissue samples of the femora of female S1-5 knockout mice and wild type mice. SB2-905, LB574 and such indicate Animal Nos.
Fig. 18 is a set of bone tissue photographs of stained hard tissue samples of femora which are representative examples of the female S1-5 knockout mice and wild type mice shown in Fig. 17. SB2-905, LB574 and such indicate Animal Nos.
Fig. 19 is a set of photographs showing osteoclasts in the sections of S1-5 knockout mice, and a table showing the number of TRAP-positive cells.
Fig. 20 shows a graph indicating the results of measuring the area occupied by osteoclasts in the tissue sections of S1-5 knockout mice.
Fig. 21A-C: (A) shows the experimental steps; (B) and (C) are graphs indicating the osteoclast-forming ability of bone marrow cells derived from S1-5 knockout mice. (B) shows the results of using TRAP staining to measure the number of TRAP-positive multinucleated cells. (C) shows the results of TRAP solution assays.

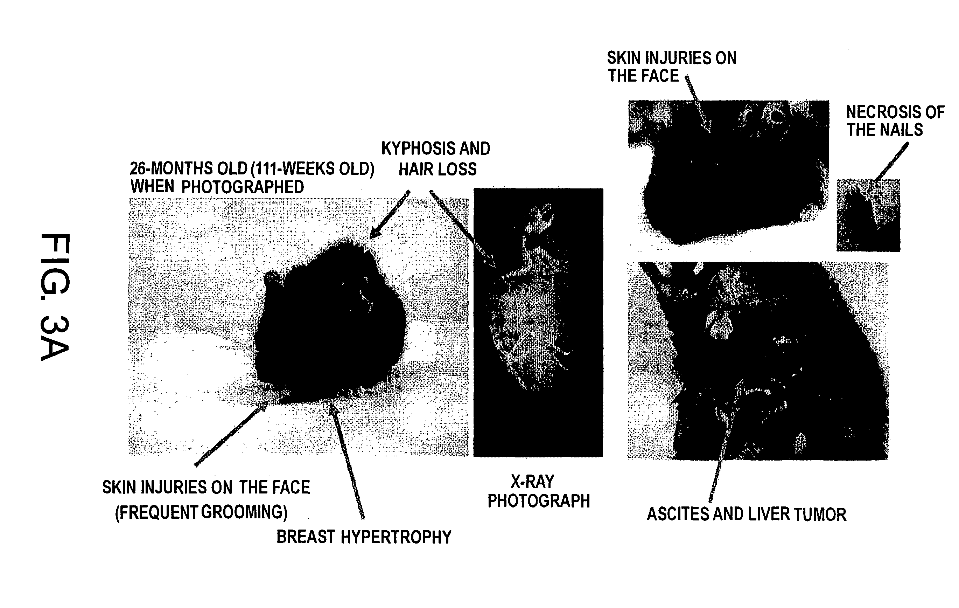

Fig. 2 1 D-E: (D) is a graph showing the results of measuring the area occupied by TRAP-positive multinucleated giant cells; (E) shows photographs demonstrating the osteoclast-forming ability of bone marrow cells derived from S1-5 knockout mice.

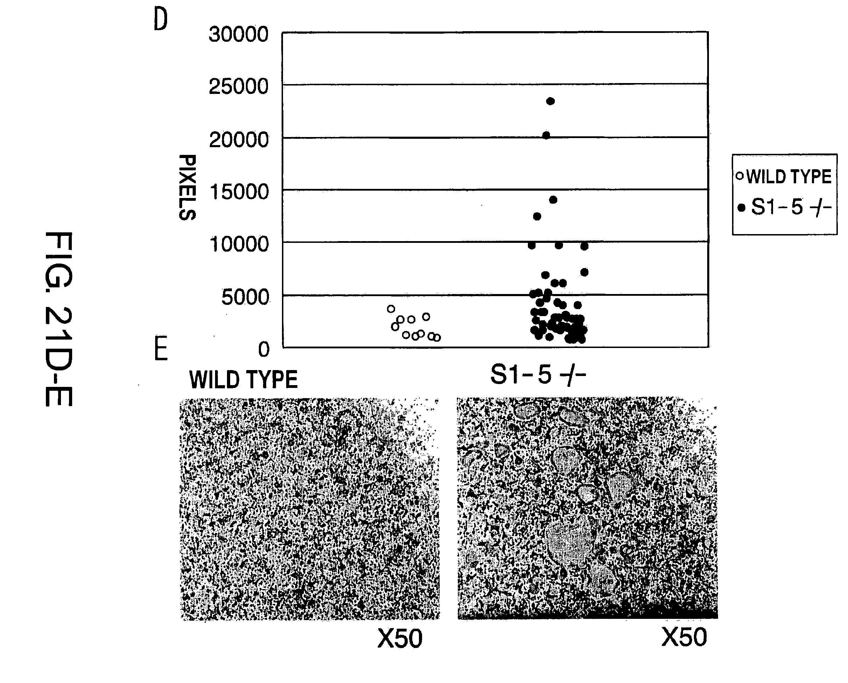

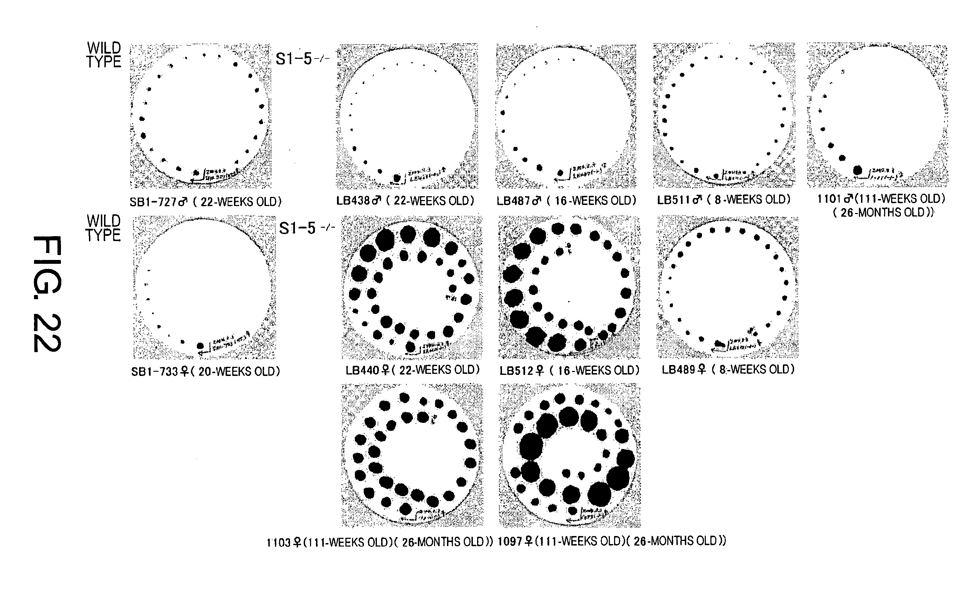

Fig. 22 is a set of photographs showing the results of making an incision on the tail of S1-5 knockout mice, and then using a filter paper to absorb the blood that flows out every ten seconds. SB1-727, LB43 8 and such indicate Animal Nos.

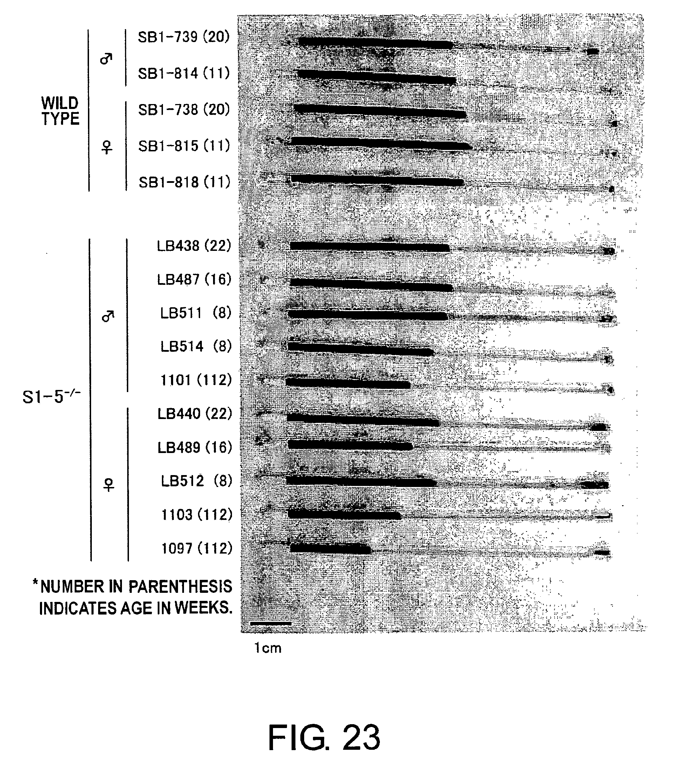

Fig. 23 is a photograph showing the hematocrit levels of S1-5 knockout mice. SB1-739, LB438 and such indicate Animal Nos.

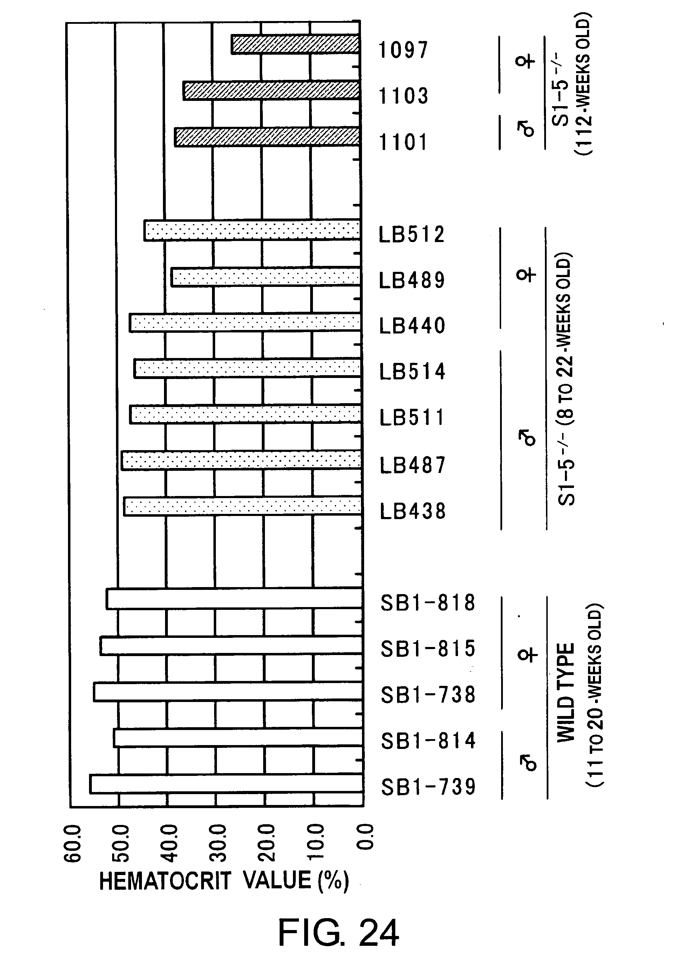

Fig. 24 indicates the hematocrit values of S1-5 knockout mice. SB1-739, LB438 and such indicate Animal Nos.

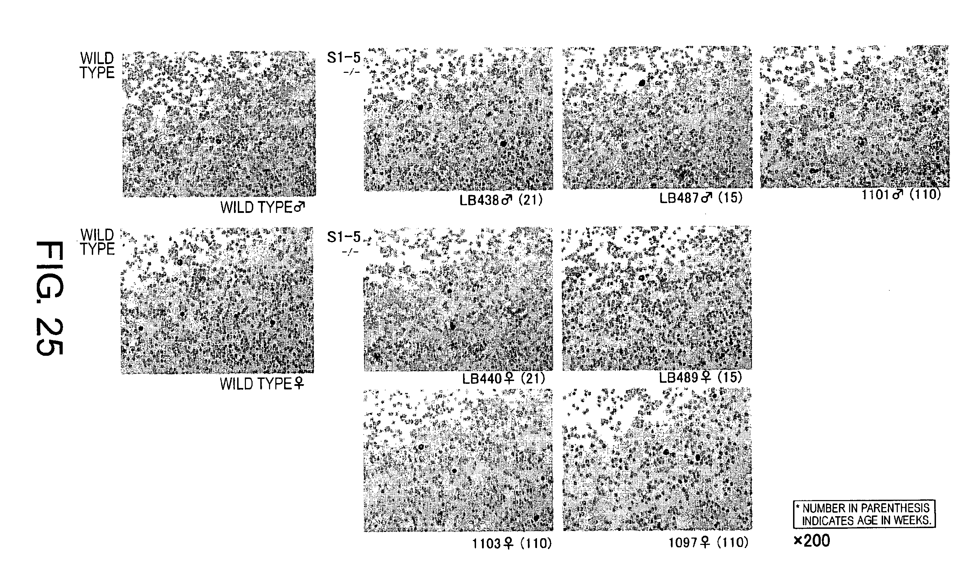

Fig. 25 shows photographs of Giemsa-stained blood smear samples produced with blood collected from the tail vein of S1-5 knockout mice. LB438, LB487 and such indicate Animal Nos.

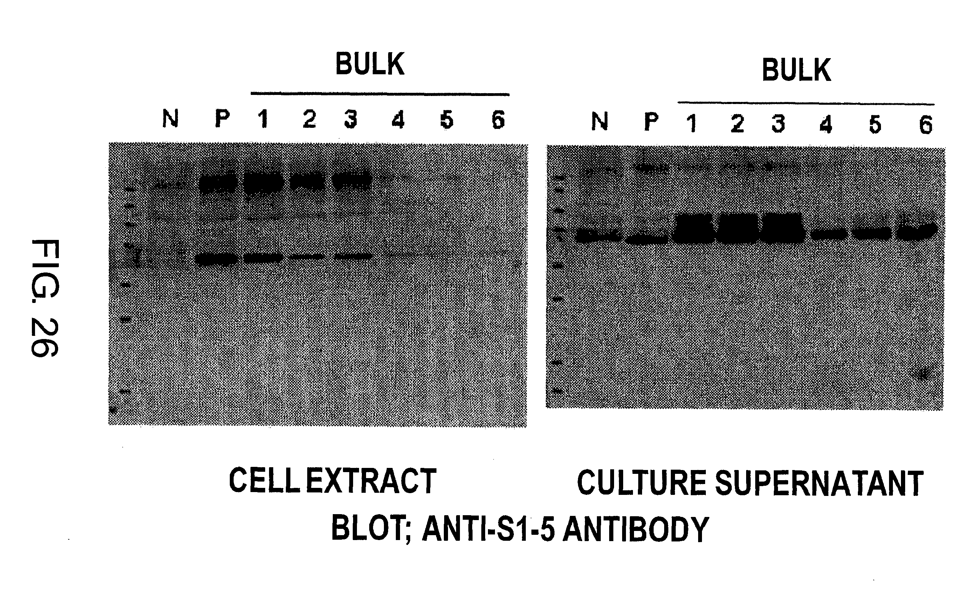

Fig. 26 shows photographs indicating the expression of S1-5-His protein in CHO-K1 cell lines (bulk) that stably express the S1-5-His protein. N indicates CHO-K1 cells that were not subjected to transfection; P indicates CHO-K1 cells that transiently expressed the S1-5-His protein.

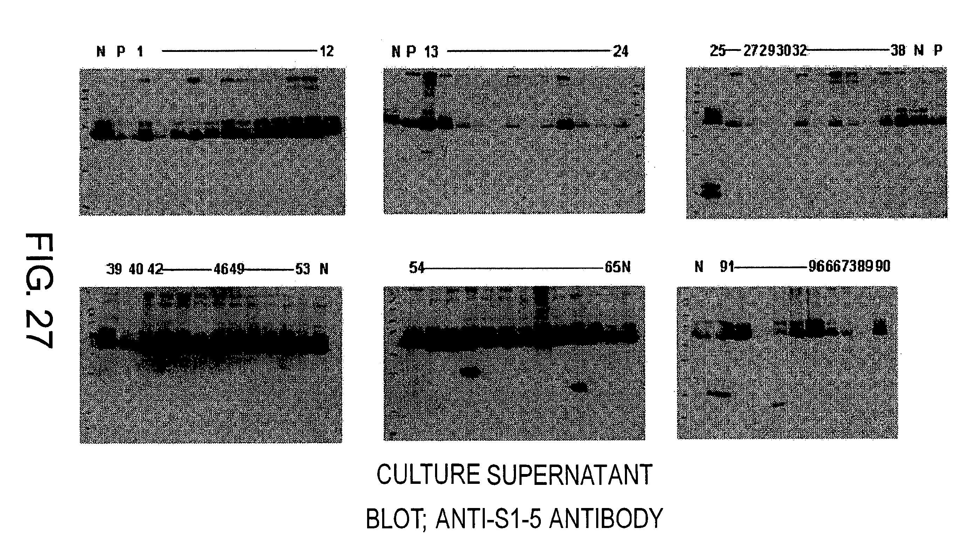

Fig. 27 is a set of photographs indicating the expression of the S1-5-His protein in a cloned CHO-K1 cell line that stably expresses S1-5-His. N indicates CHO-K1 cells that were not subjected to transfection; P indicates CHO-K1 cells that transiently expressed the S1-5-His protein.

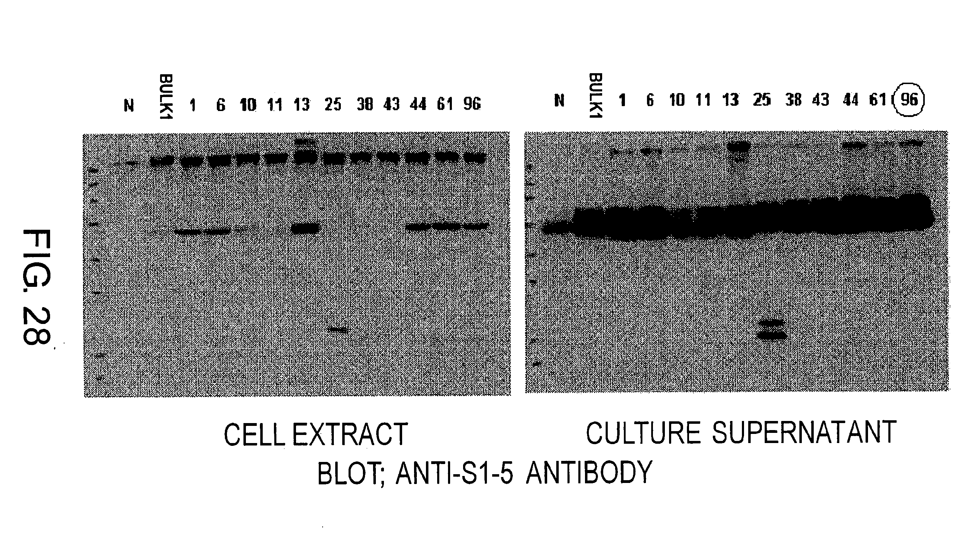

Fig. 28 shows photographs indicating the expression of the S1-5-His protein in a cloned CHO-K1 cell line that stably expresses S1-5-His. N indicates CHO-K1 cells that were not subjected to transfection.

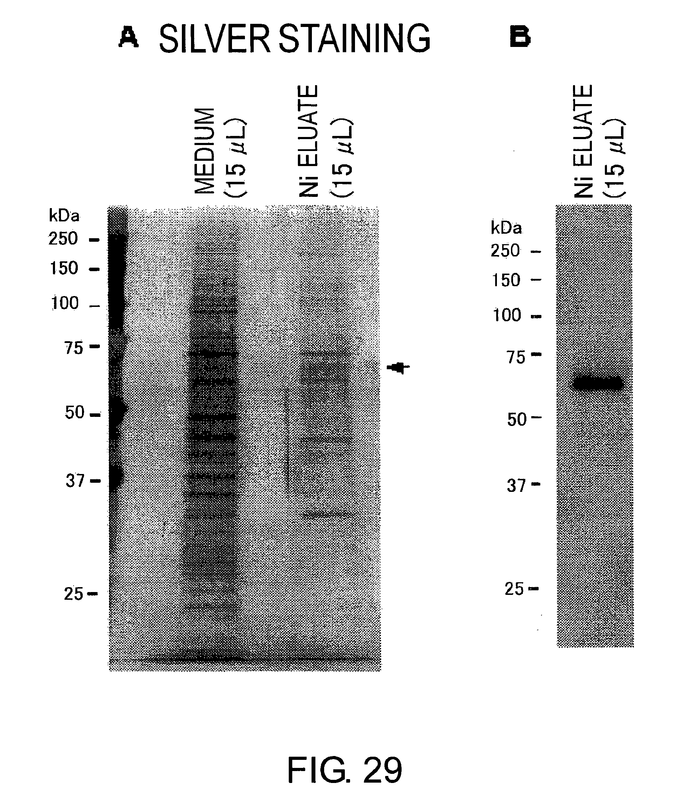

Fig. 29 shows photographs indicating the results of using 10% SDS-PAGE to separate proteins contained in the fraction eluted with 250 mM imidazole, and then detecting the proteins using (A) silver staining; and (B) Western blotting using an anti-S1-5 antibody.

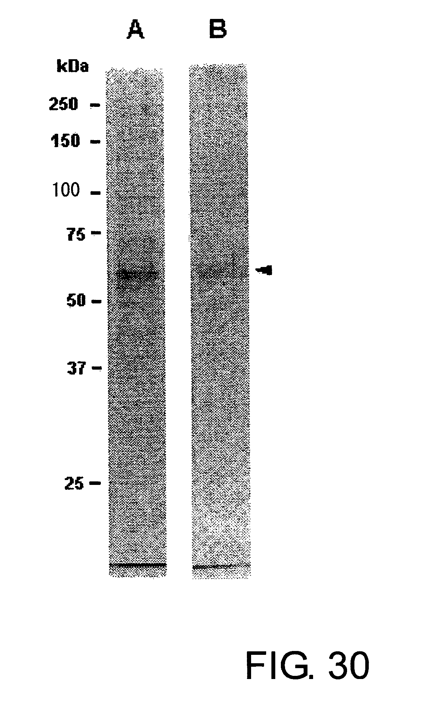

Fig. 30 is a photograph showing the result of using 10% SDS-PAGE to separate proteins contained in the fractions eluted with 50 mM (A) and 100 mM (B) imidazole, and then detecting the proteins using silver staining.

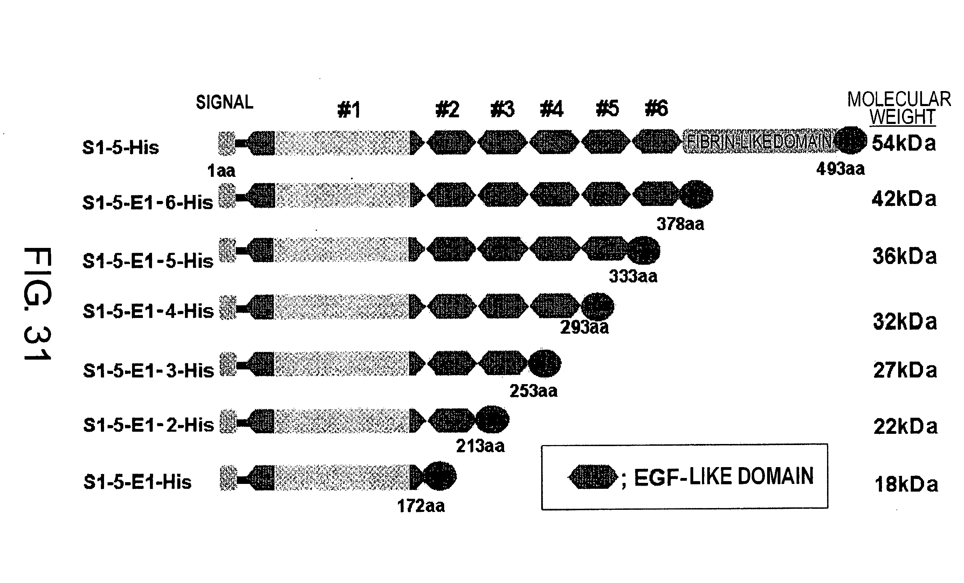

Fig. 31 is a schematic diagram of the S1-5 truncated proteins.

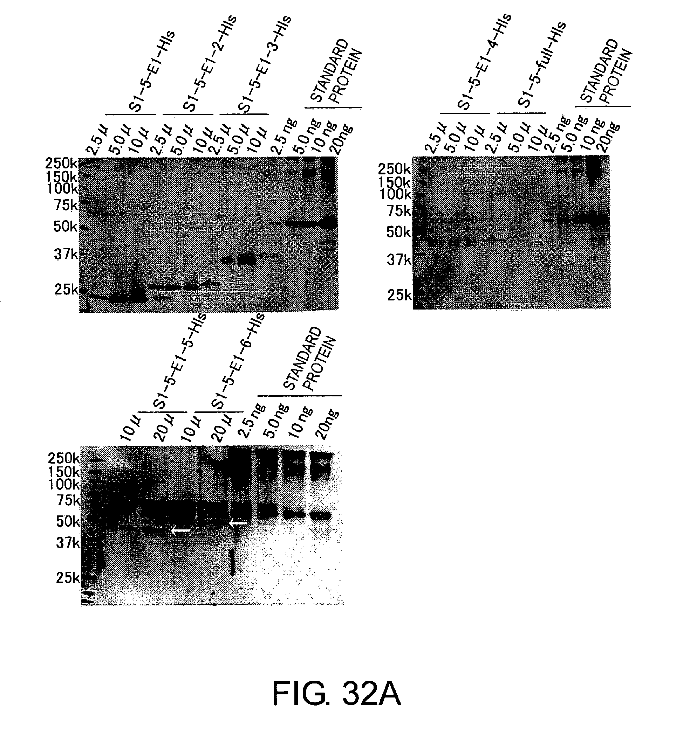

Fig. 32A is a set of photographs showing the results of preparing the S1-5-His truncated proteins.

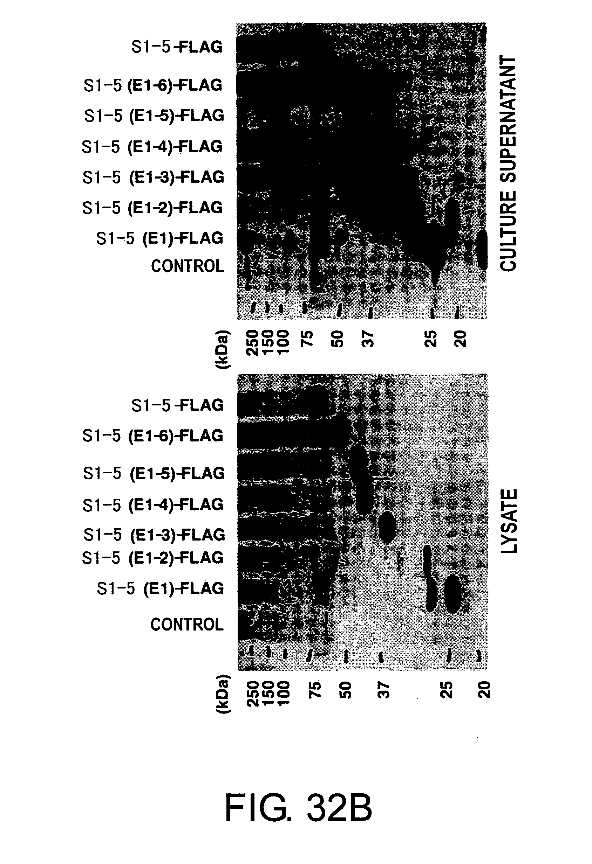

Fig. 32B shows photographs displaying the results of preparing the S1-5-FLAG truncated proteins.

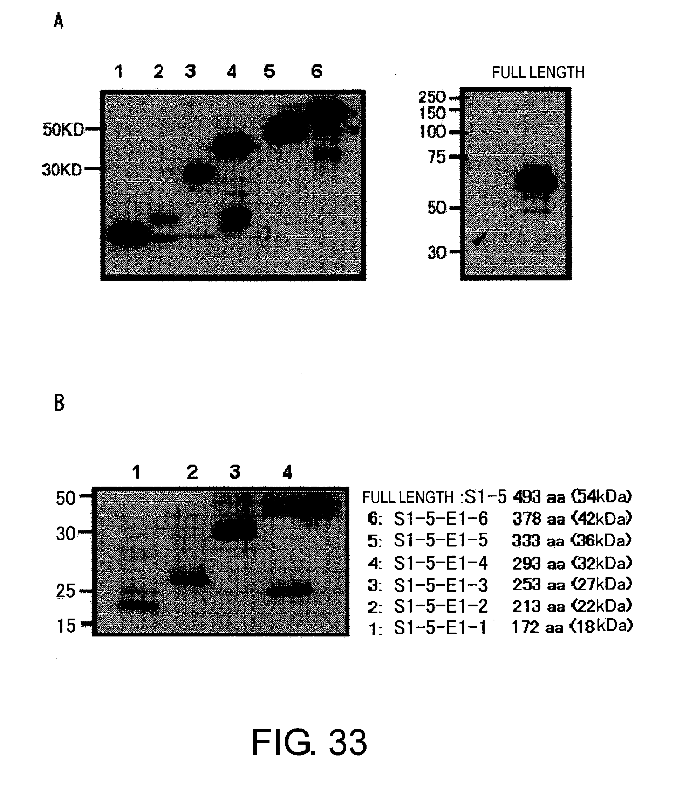

Fig. 33 is a set of photographs exhibiting the results of using Western blotting with anti-S1-5 antibody to detect (A) FLAG-S1-5 proteins purified from cells using anti-FLAG antibody, and (B) FLAG-S1-5 proteins purified from culture supernatants using anti-FLAG antibody.

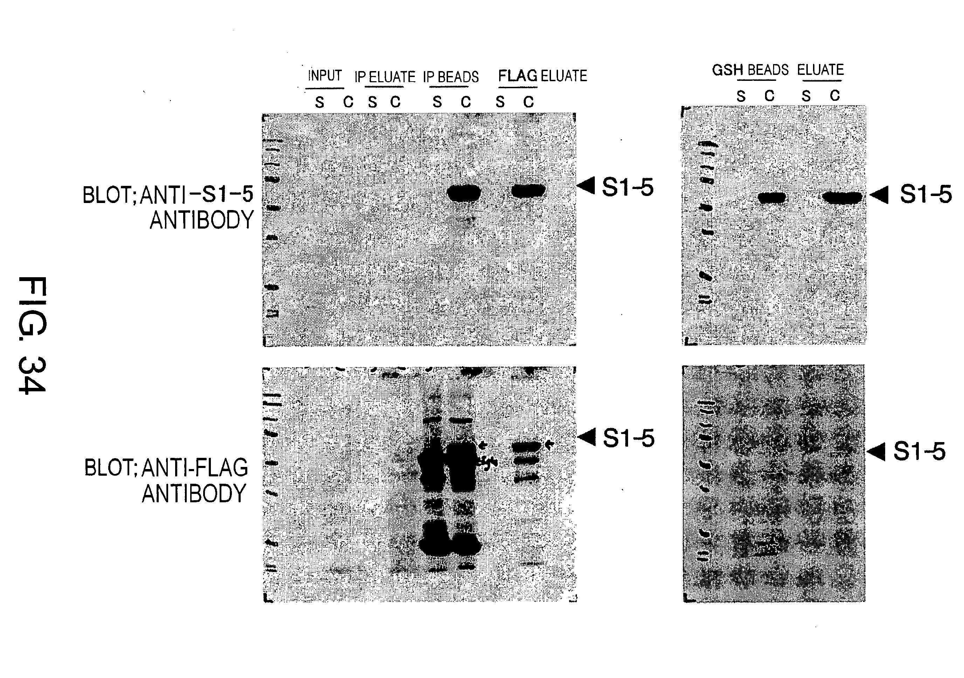

Fig. 34 is a set of photographs exhibiting the results of using Western blotting with anti-S1-5 antibody and anti-FLAG antibody to detect S1-5-FLAG protein purified from 293F non-adherent cells.

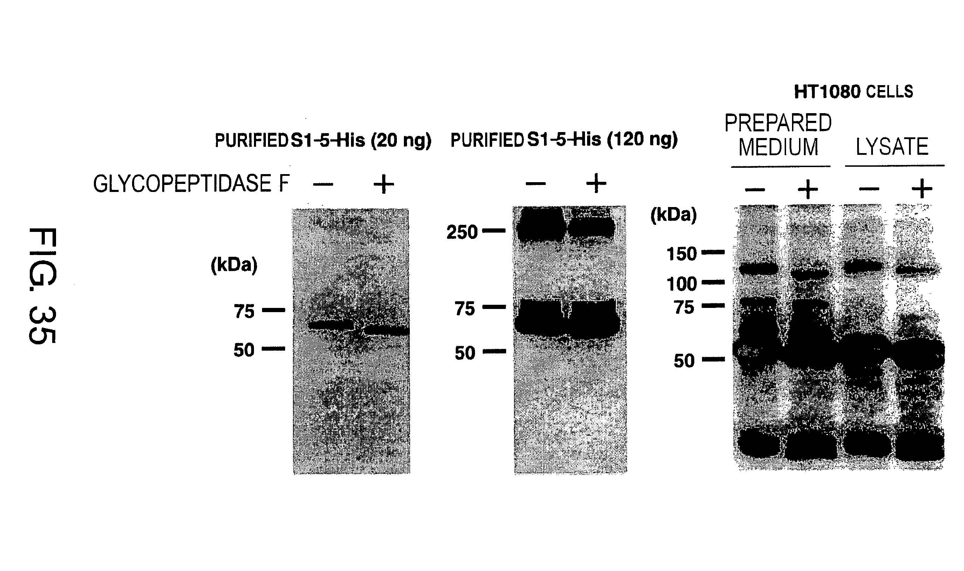

Fig. 35 is a set of photographs showing the results of using Western blotting with an anti-S1-5 antibody to detect S1-5-His protein treated with glycosylpeptidase F.

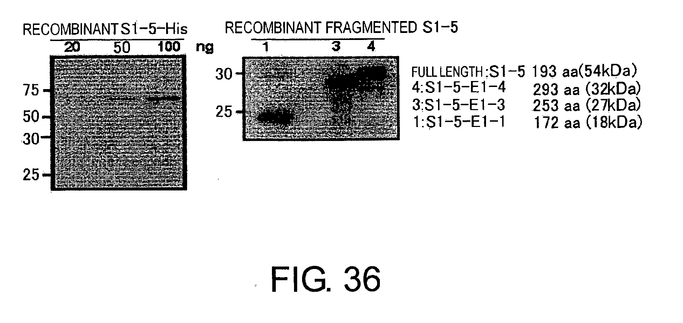

Fig. 36 shows photographs exhibiting the results of using Western blotting to detect GST-S1-5-His truncated protein purified from E. *coli* and prescission protease-treated GST-S1-5 proteins.

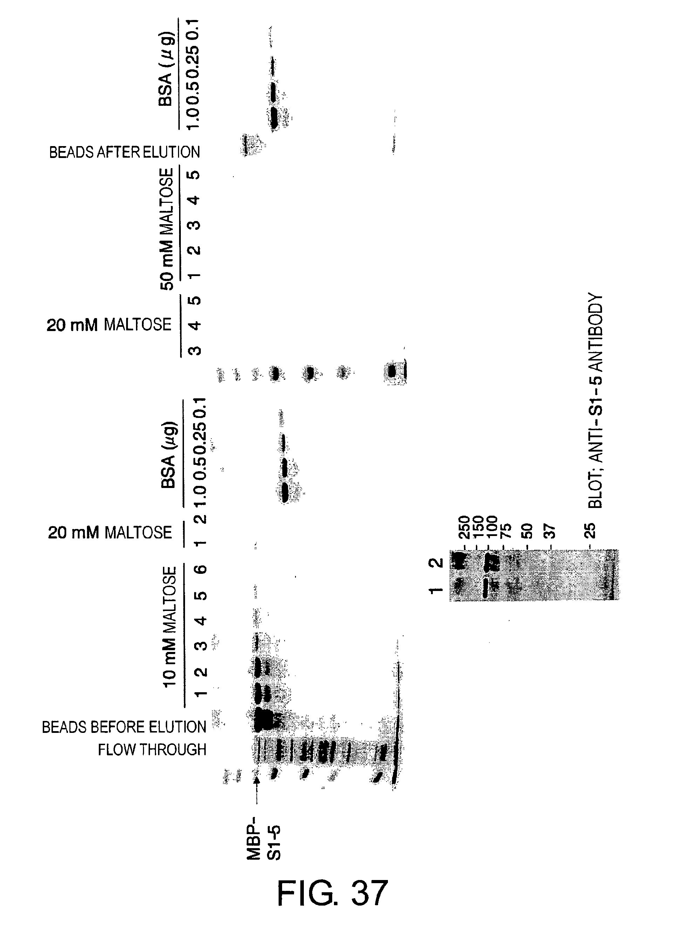

Fig. 37 shows photographs exhibiting the results of detecting the MBP-S1-5 protein purified from E. *coli* by CBB staining; and by Western blotting with an anti-S1-5 antibody. "1" indicates the GST-S1-5 protein; "2" indicates the MBP-S1-5 protein.

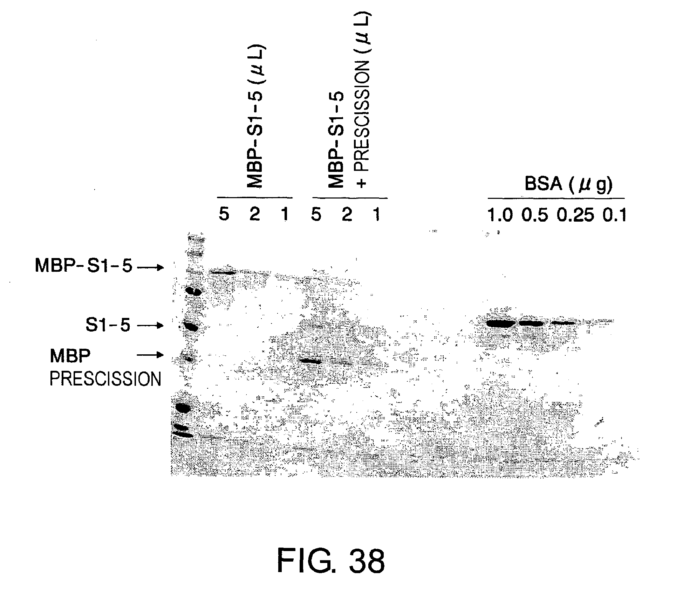

Fig. 38 is a photograph showing the results of using CBB staining to detect prescission protease-treated MBP-S1-5 protein purified from *E*. *coli.*

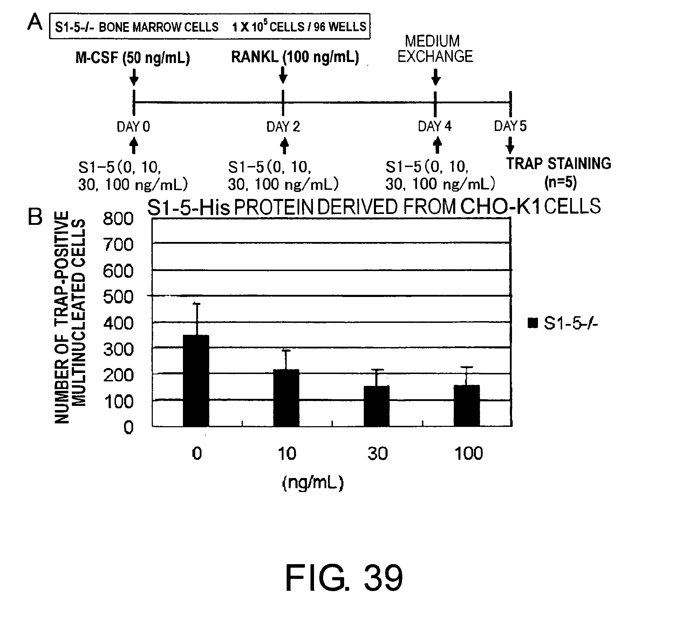

Fig. 39: (A) shows the experimental steps; and (B) is a graph showing that the osteoclast-forming ability of bone marrow cells derived from S1-5 knockout mice is suppressed by S1-5-His protein derived from CHO-K1 cells. (B) graphs the number of TRAP-positive multinuclear cells.

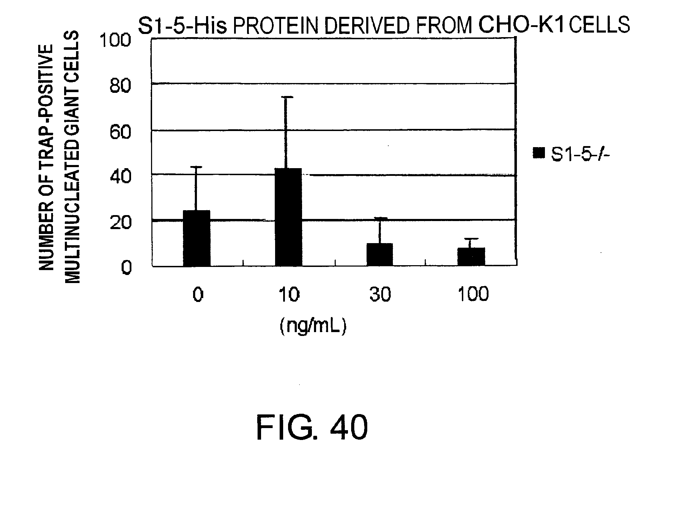

Fig. 40 is a graph indicating that the osteoclast-forming ability of bone marrow cells derived from S1-5 knockout mice is suppressed by S1-5-His protein derived from CHO-K1 cells. The graph shows the number of TRAP-positive multinucleated giant cells.

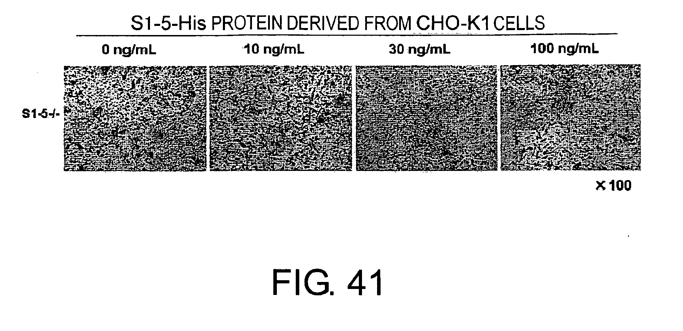

Fig. 41 is a series of photographs indicating that the osteoclast-forming ability of bone marrow cells derived from S1-5 knockout mice is suppressed by S1-5-His protein derived from CHO cells.

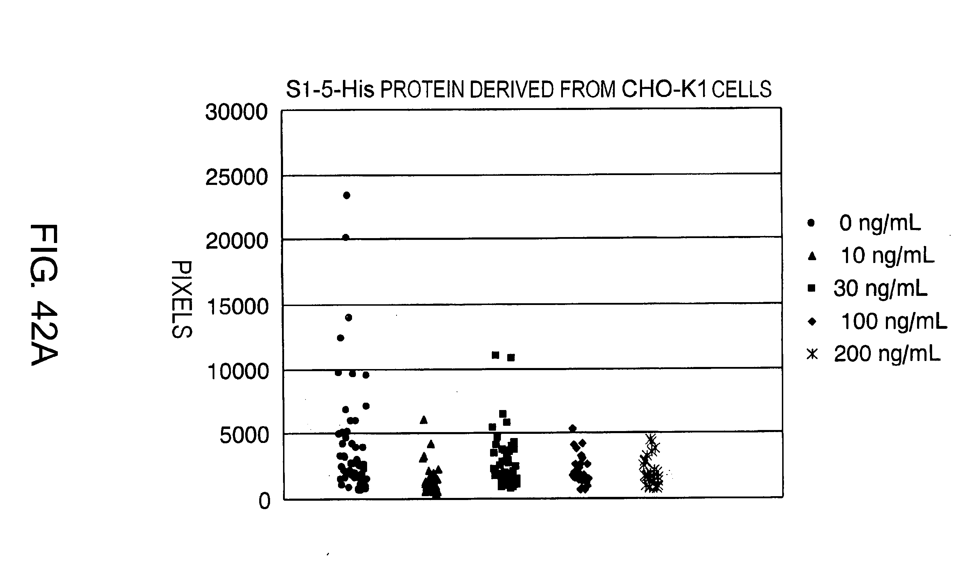

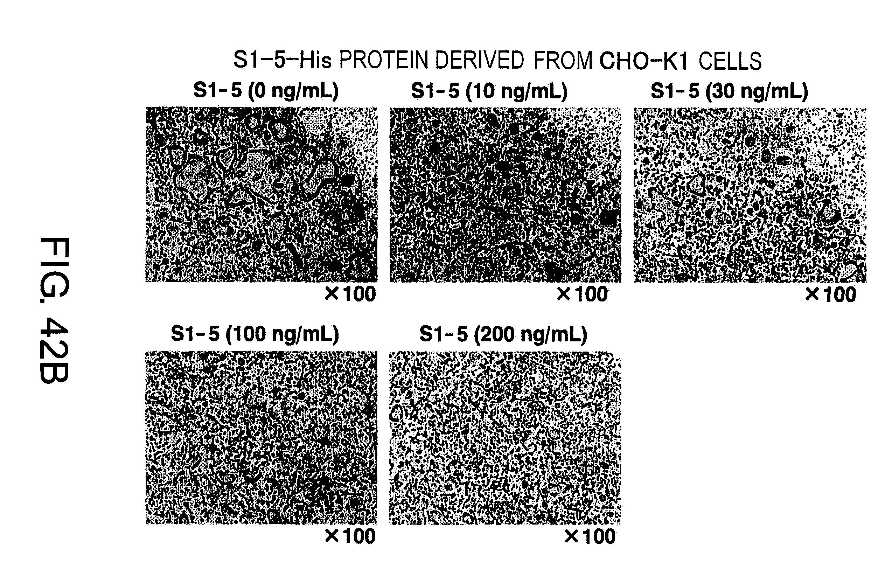

Fig. 42A is a graph showing a decrease in the area occupied by TRAP-positive multinucleated giant cells, which is caused by S1-5-His protein derived from CHO cells.
Fig. 42B is a series of photographs of stained TRAP-positive multinucleated giant cells, whose formation is suppressed in a manner dependent on the concentration of S1-5-His protein derived from CHO cells.

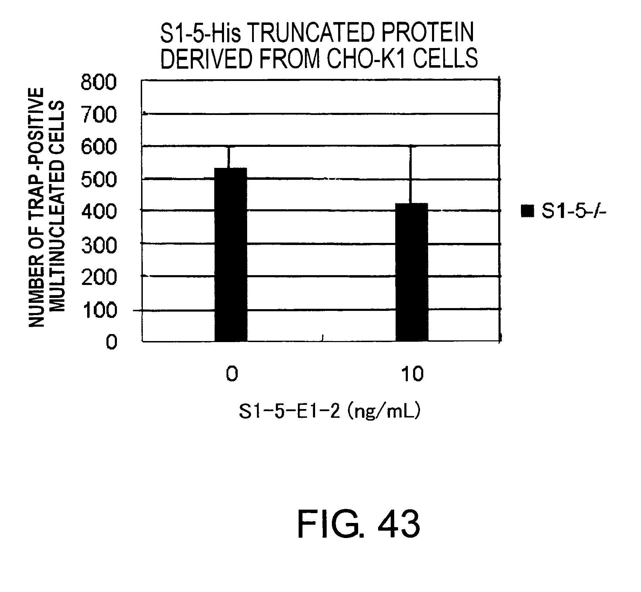

Fig. 43 is a graph indicating that the osteoclast-forming ability of bone marrow cells derived from S1-5 knockout mice is suppressed by the S1-5-His protein. The graph shows the number of TRAP-positive multinucleated cells.

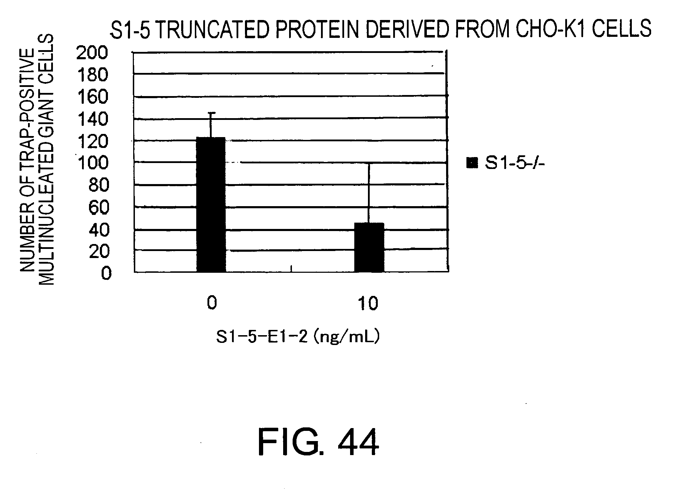

Fig. 44 is a graph indicating that the osteoclast-forming ability of bone marrow cells derived from S1-5 knockout mice is suppressed by S1-5-His protein. The graph shows the number of TRAP-positive multinucleated giant cells.

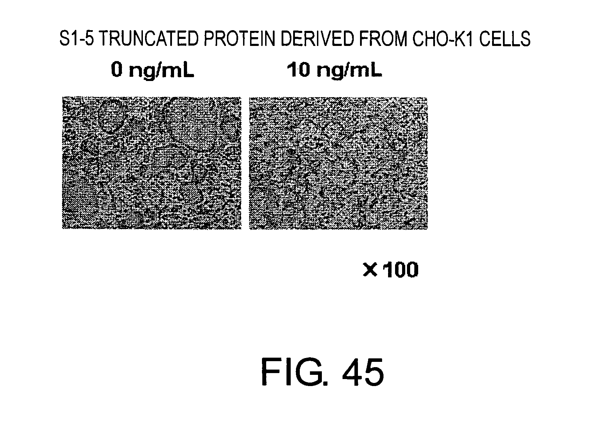

Fig. 45 shows photographs demonstrating that the osteoclast-forming ability of bone marrow cells derived from S1-5 knockout mice is suppressed by S1-5-His truncated protein.

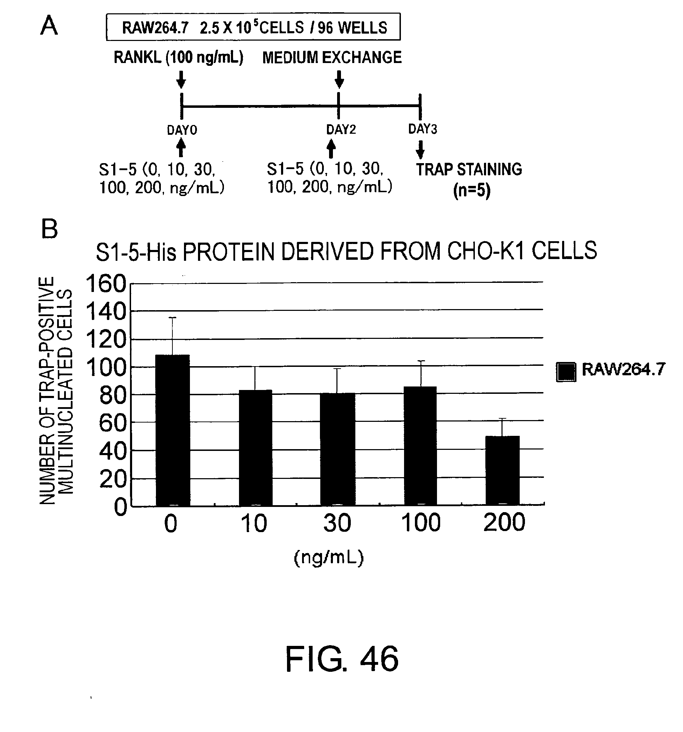

Fig. 46: (A) shows the experimental steps; and (B) is a graph showing that the osteoclast-forming ability of RAW264.7 cells is suppressed by S1-5-His protein derived from CHO-K1 cells.

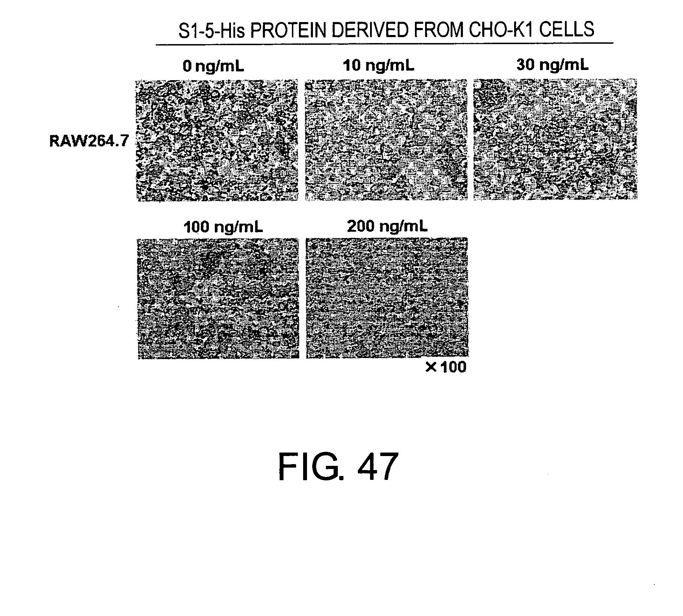

Fig. 47 is a series of photographs demonstrating that the osteoclast-forming ability of RAW264.7 cells is suppressed by S1-5-His protein.

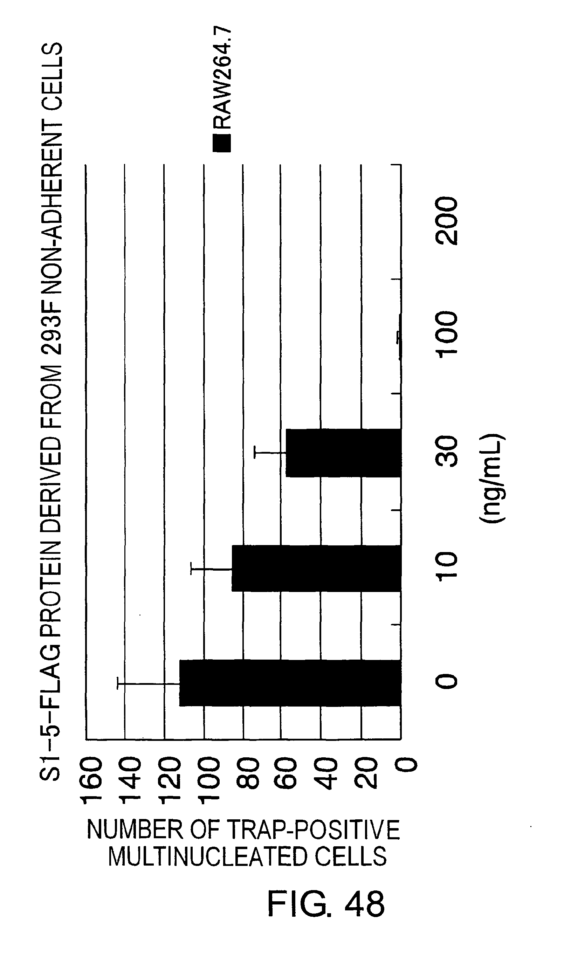

Fig. 48 is a graph indicating that the osteoclast-forming ability of RAW264.7 cells is suppressed by S1-5-FLAG protein derived from 293F non-adherent cells.

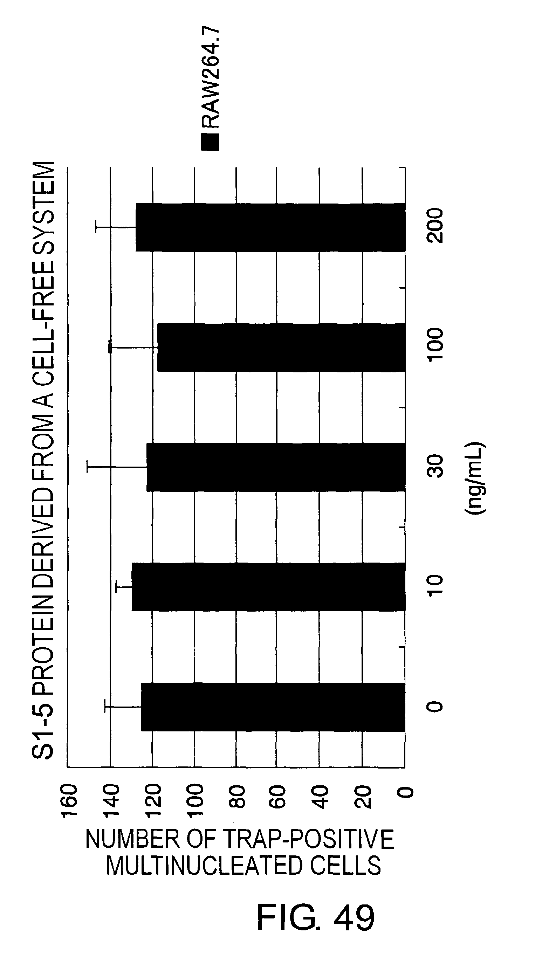

Fig. 49 is a graph indicating that the osteoclast-forming ability of RAW264.7 cells is not suppressed by S1-5 protein obtained from a cell-free expression system.

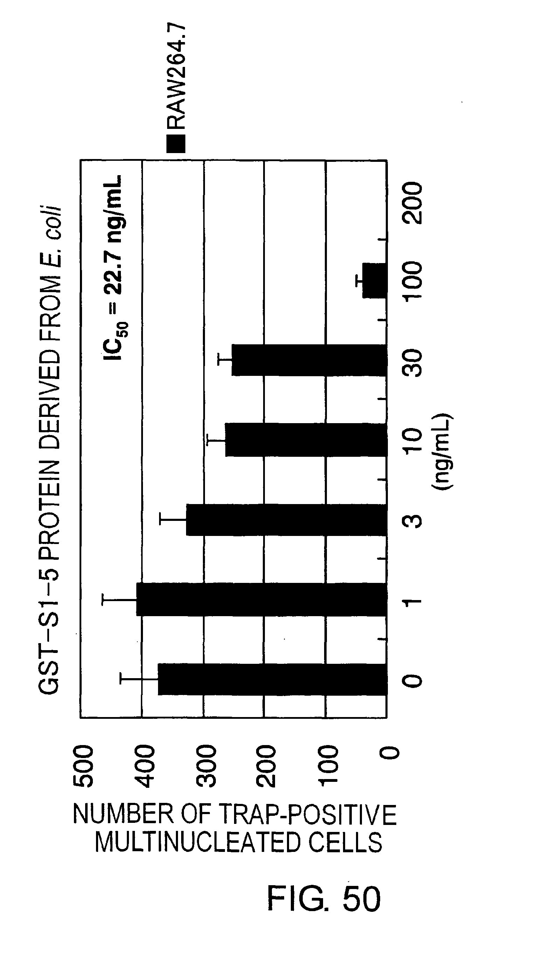

Fig. 50 is a graph indicating that the osteoclast-forming ability of RAW264.7 cells is suppressed by GST-S1-5 protein.

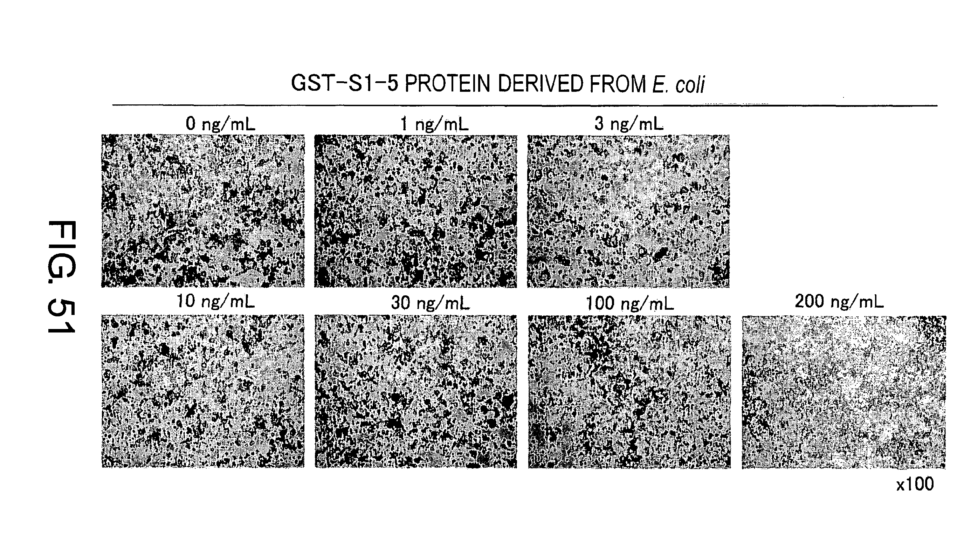

Fig. 51 is a series of photographs demonstrating that the osteoclast-forming ability of RAW264.7 cells is suppressed by GST-S1-5 protein.

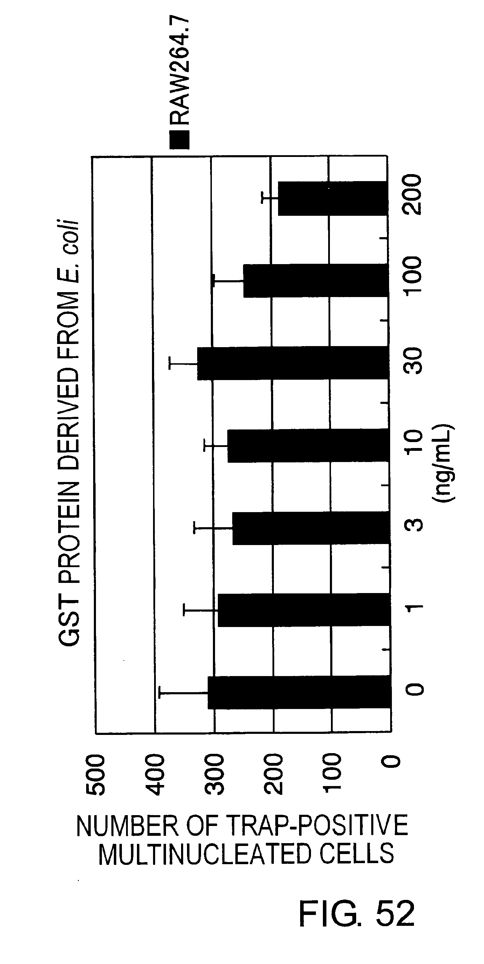

Fig. 52 is a graph indicating that the osteoclast-forming ability of RAW264.7 cells is not suppressed by GST protein.

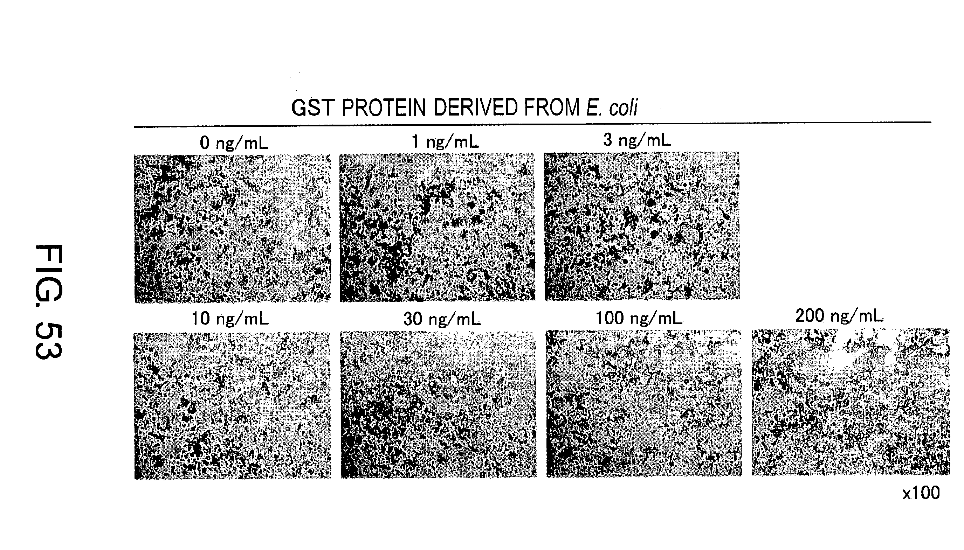

Fig. 53 is a series of photographs demonstrating that the osteoclast-forming ability of RAW264.7 cells is not suppressed by GST protein.

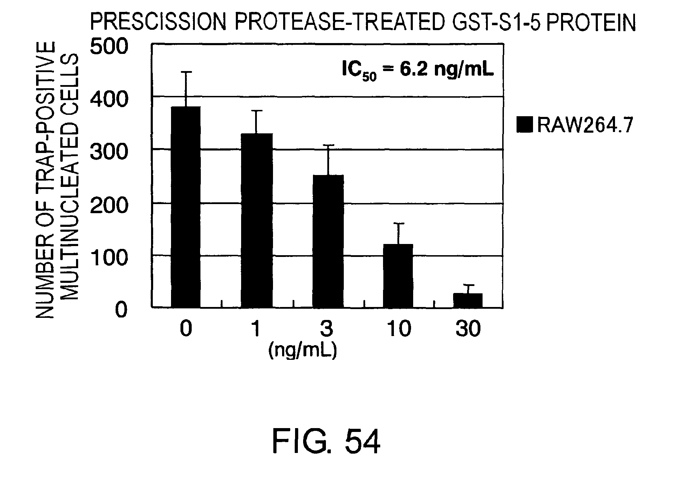

Fig. 54 is a graph indicating that the osteoclast-forming ability of RAW264.7 cells is suppressed by prescission protease-treated GST-S1-5 protein.

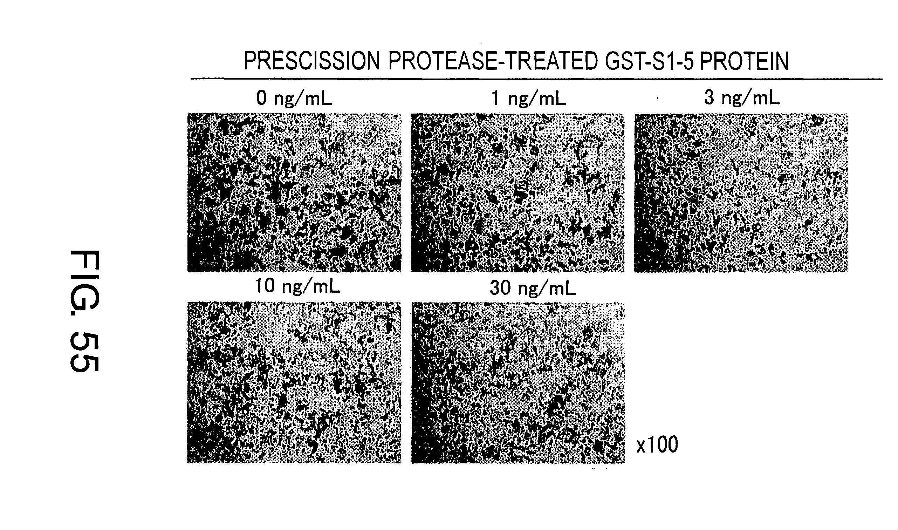

Fig. 55 is a series of photographs demonstrating that the osteoclast-forming ability of RAW264.7 cells is suppressed by prescission protease-treated GST-S1-5 protein.

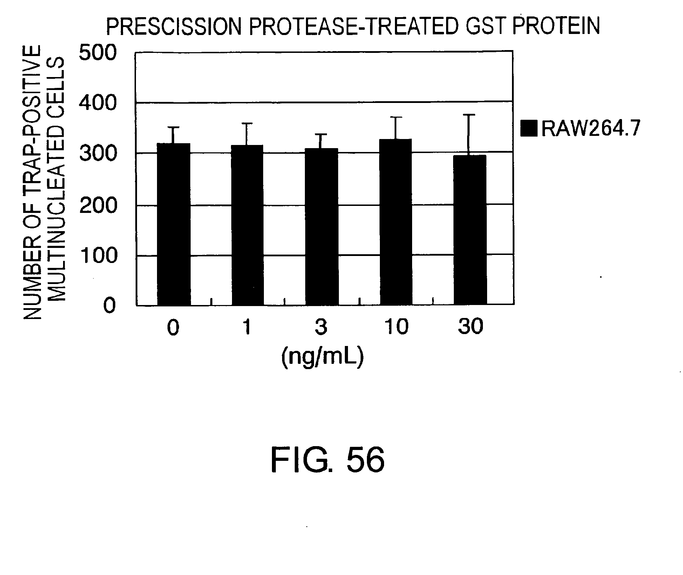

Fig. 56 is a graph indicating that the osteoclast-forming ability of RAW264.7 cells is not suppressed by prescission protease-treated GST protein.

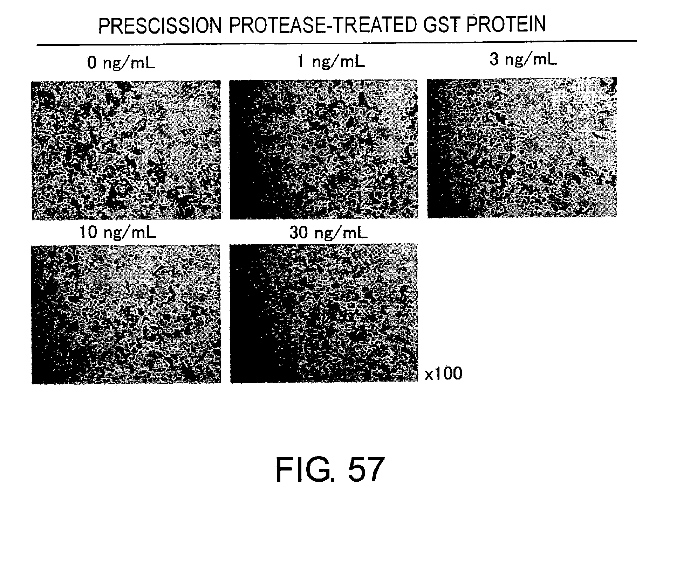

Fig. 57 is a series of photographs demonstrating that the osteoclast-forming ability of RAW264.7 cells is not suppressed by prescission protease-treated GST protein.

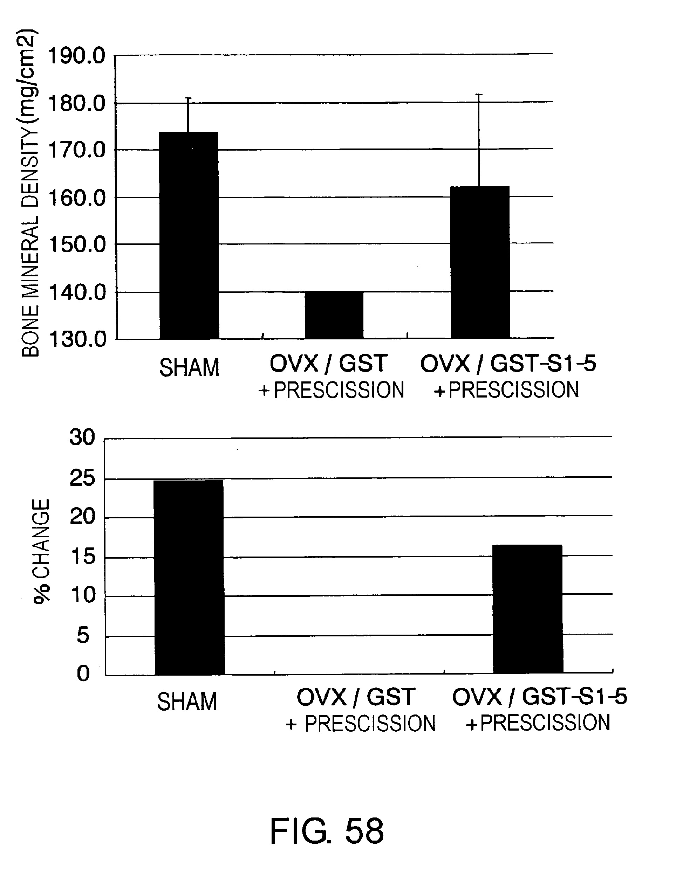

Fig. 58 shows graphs indicating that administration of S1-5 protein caused recovery of bone mineral density of the femora of ovariectomized (OVX) rats.

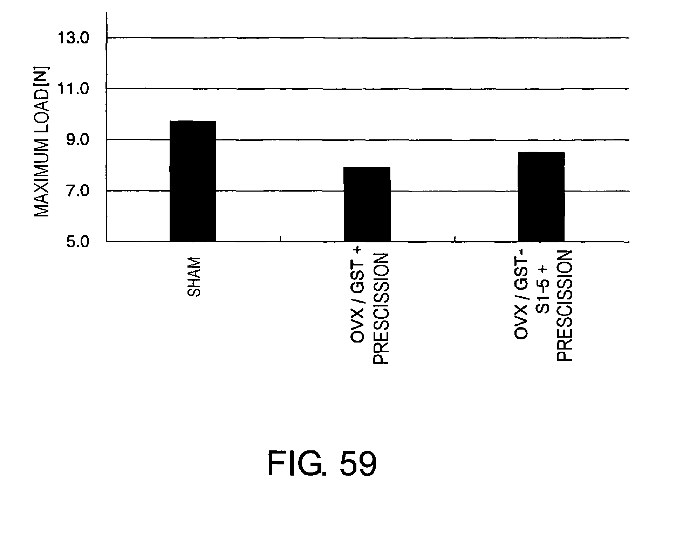

Fig. 59 shows a graph indicating that administration of S1-5 protein caused recovery of bone strength in the femora of OVX rats.

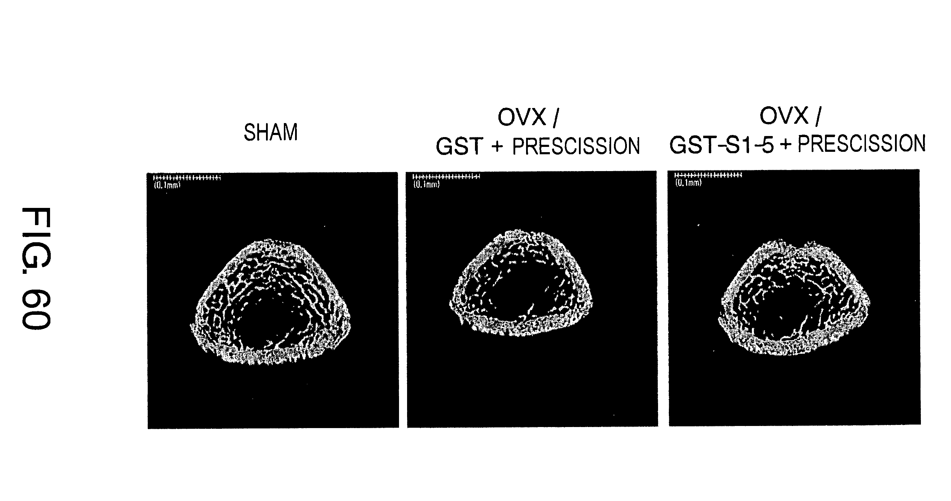

Fig. 60 is a set of photographs demonstrating that administration of S1-5 protein caused recovery of bone content in the femora of OVX rats.

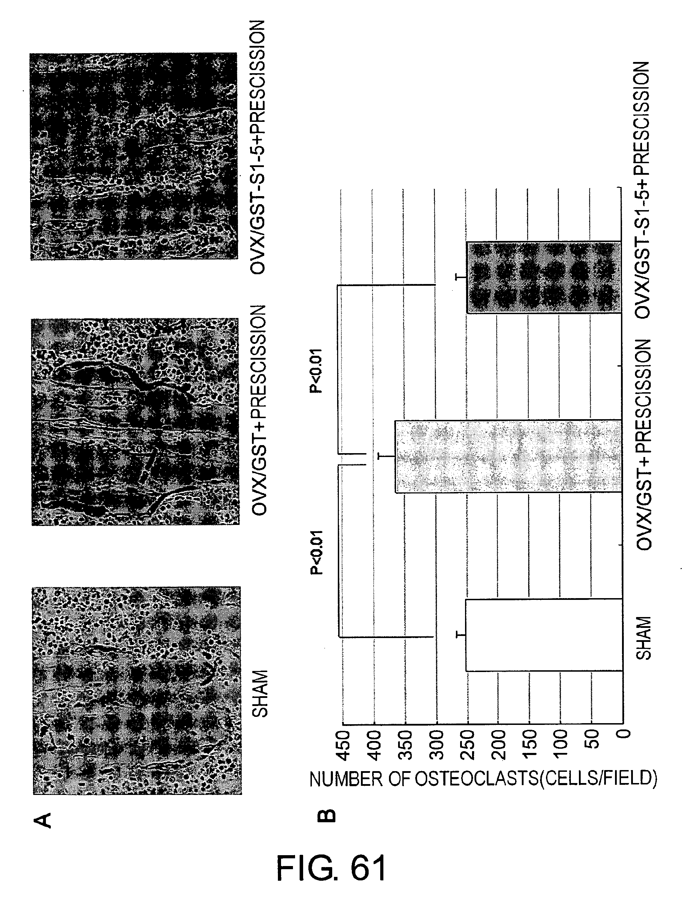

Fig. 61: (A) shows photographs demonstrating that administration of S1-5 protein suppressed the number of osteoclasts in the tissue section of OVX rats; (B) is a graph indicating the same.

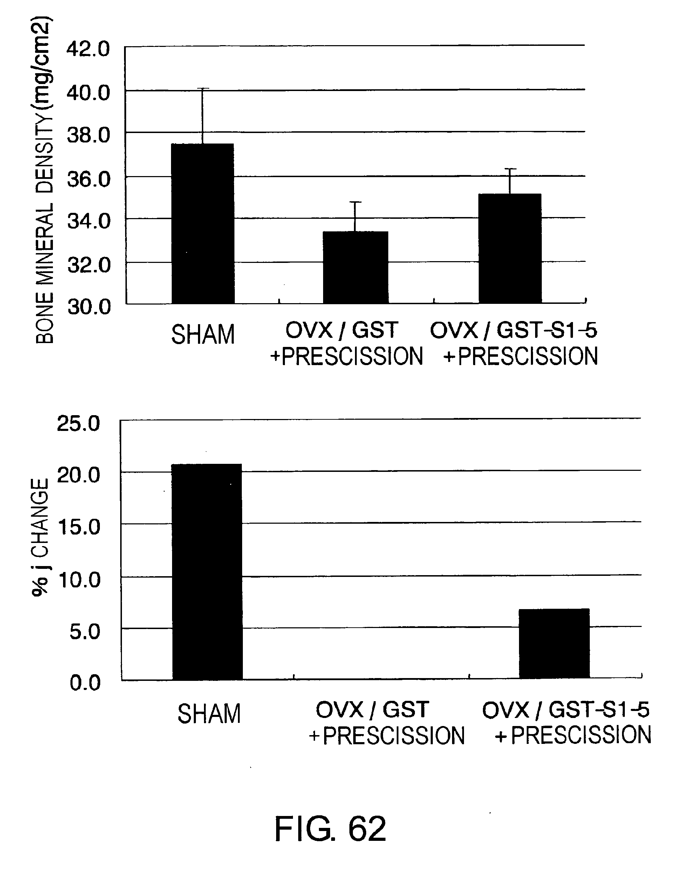

Fig. 62 shows graphs indicating that administration of S1-5 protein caused recovery of bone mineral density in the femora of OVX mice.

### Best Mode for Carrying Out the Invention

Hereinafter, the present invention will be described specifically.

### 1. S1-5 genes

The gene encoding S1-5 is publicly known, and the S1-5 protein specified by accession number AAA65590 (nucleotide accession U03877), I38449, NP_061489 (nucleotide accession NM 018894), NP_004096 (nucleotide accession NM 004105), or Q12805 and similar proteins comprising the activity of binding to human synoviolin protein may also be used (Leucka-Czernik, B. et al., Mol. Cell. Biol. 15: 120-128, 1995; Heon, E. et al., Arch. Ophthalmol. 114: 193-198, 1996; Ikegawa, S. et al., Genomics 35: 590-592, 1996; Katsanis, N. et al., Hum. Genet. 106: 66-72, 2000; Giltay, R. et al., Matrix. Biol. 18: 469-480, 1999; Stone, E. M. et al., Nat. Genet.22: 199-202, 1999).

S1-5 gene can be obtained from a genomic library of mice, rats, or such. For example, desired clones can be obtained from a bacterial artificial chromosome (BAC) library by using hybridization methods. Such clones may also be obtained using PCR methods.

### 2. Non-human knockout animals

The present invention provides 1) non-human knockout animals that have developed an age-related disease or symptom, in which all or a part of S1-5 gene function has been lost, and 2) methods for producing non-human knockout animals that develop an age-related disease,
wherein the method comprises causing the loss of all or a part of S 1-5 gene function. The loss of all or a part of S1-5 gene function may be effected by, for example, a disruption or mutation of the S1-5 gene.

In the present invention, gene targeting may be used to generate knockout animals in which all or a part of S1-5 function is lost.

An S1-5 gene targeting vector is used to cause the loss of all or a part of S1-5 gene function by disrupting the S1-5 gene. Herein, the phrase "to cause the loss of a function" means to completely lose gene function, or to produce a condition whereby gene function is reduced compared to the wildtype.

A function can be lost simply by disrupting or deleting a gene, or by making a modification, such as introducing a mutation into the gene such that a frame shift will occur during translation.

The "knockout animals" of the present invention can be produced as follows:

First, an S1-5 gene, in which all or a part of the nucleotide sequence has been modified, is introduced into totipotent cells, and those totipotent cells transfected with the modified S1-5 gene are then selected. Next, the selected genetically modified (deleted, disrupted, mutated, etc.) totipotent cells are introduced into fertilized eggs to produce chimeric individuals. Crossing the obtained chimeric individuals will produce individuals in which one or both S1-5 genes on homologous chromosomes have been knocked out.

The types of animals used in the present invention are not particularly limited. For example, with the exception of humans, the animals include zebrafish, mice, rats, guinea pigs, rabbits, chickens, pigs, sheep, goats, dogs, cattle, monkeys, and chimpanzees. Mice are preferred in the present invention since they are easy to handle and reproduce readily.

Herein, a part or all of the nucleotide sequence of the S1-5 gene can be modified to cause the loss of S 1-5 gene function. The term "modify" means to introduce a mutation that causes a deletion, substitution, or addition to a part of the DNA of the S1-5 gene. Such mutations include the use of genetic engineering techniques to delete a part or all of the nucleotide sequence, or to insert another gene or nucleotide sequence, or to substitute a gene or nucleotide sequence. For example, a defective S1-5 gene can be produced by shifting a codon reading frame, or by disrupting the function of a promoter or exon. As a result, the function of the S1-5 protein produced by expressing the S1-5 gene will not work.

The knockout mice of the present invention can be produced by known methods for gene recombination (gene targeting). Gene targeting is a technique well known in the art, and is disclosed in various laboratory manuals in the present field.

When designing a targeting vector, the part that causes the change in S1-5 gene structure is not particularly limited, so long as the function of the S1-5 gene is lost. However, considering the function and structure of the S1-5 gene, it is particularly preferable to design the vector such that a region of the EGF-like domain or fibrin-like domain of the S1-5 gene is deleted.

Recombinants carrying the vector are preferably selected by the combined use of screening using a drug-resistance gene introduced by the targeting vector, and screening using Southern blotting or PCR. Neomycin resistance gene, hygromycin B phosphotransferase gene, or such may be used as drug selection marker genes. HSV thymidine kinase gene, diphtheria toxin A gene, or such may be used as genes for negative selection.

Homologous recombination is performed using a targeting vector produced by an above-described method. As used herein, "homologous recombination" means that a modified S1-5 gene is artificially recombined into a DNA region of the S1-5 gene in a genome.

To obtain a desired recombinant, a large number of recombinants must be screened. However, screening a large number of fertilized eggs is technically difficult. Therefore, it is preferable to use cells that can be cultured *in vitro* and are multipotent, like fertilized eggs. Embryonic stem cells and such have been established as totipotent cells for mice (Nature 292:154-156, 1981), rats (Iannaccone, P.M. et al., Dev. Biol. 163(1): 288-292, 1994), monkeys (Thomson, J.A. et al., Proc. Natl. Acad. Sci. U.S.A. 92(17):7844-7848, 1995) and rabbits (Schoonjans, L. et al., Mol. Reprod. Dev. 45(4):439-443, 1996). For pigs, embryonic germ (EG) cells have been established (Shim H. et al., Biol. Reprod 57(5):1089-1095, 1997).

Therefore, in the present invention, production of knockout animals using these animal species is preferred. Mice, for which techniques relating to the production of knockout animals are well established, are particularly suitable. With regards to mouse ES cells, several ES cell lines derived from mice are currently established, and for example, TT-2 cell line, AB-1 cell line, J1 cell line, or R1 cell line may be used. A selection regarding which of these ES cell lines to use can be made appropriately according to the objectives or methods of the experiment.

When establishing ES cells, blastocysts 3.5 days after fertilization are generally used. As an alternative to this, embryos in the eight-cell stage can be collected and the blastocysts produced by culturing these embryos can be used to efficiently obtain many early stage embryos.

ES cell lines obtained this way are usually very proliferative; however, since they easily lose the regenerative capacity that enables ontogenesis, they must be subcultured carefully. For example, the methods employed involve culturing cells on appropriate feeder cells, such as STO fibroblasts, in the presence of leukemia inhibitory factor (LIF) (1 to 10000 U/ml) in a carbon dioxide incubator (preferably 5% carbon dioxide gas and 95% air; or 5% oxygen, 5% carbon dioxide gas, and 90% air) at approximately 37°C, and subculturing, for example, by separating into single cells with trypsin/EDTA solution treatment, and then plating onto freshly prepared feeder cells. Such subculturing is ordinarily carried out every one to three days, and cell morphology is preferably observed.

Genes can be transfected into ES cells using methods such as calcium phosphate coprecipitation, electroporation, lipofection, retroviral infection, agglutination, microinjection, and particle guns, but electroporation is preferred since many cells can be treated with ease.

The resultant recombinant ES cells are screened to check whether homologous recombination has taken place. More specifically, the cells are first screened using a drug resistance factor introduced with neomycin or such. Examples of drug resistance genes include neomycin resistance gene, hygromycin B phosphotransferase gene, diphtheria toxin A gene. Examples of reporter genes include β-galactosidase gene, chloramphenicol acetyltransferase gene.

Additionally, the obtained recombinant ES cells can be reliably screened to determine whether homologous recombination has taken place by performing Southern blot analysis using a DNA sequence on the S1-5 gene or in its vicinity as a probe; or by performing PCR using as primers a DNA sequence on the targeting vector and the DNA sequence of a region near but not within the mouse-derived S1-5 gene used for the targeting vector.

These assays enable the selection of cells in which correct homologous recombination has taken place between the wildtype S1-5 gene located on the chromosome and the introduced S1-5 gene fragment, such that the mutation is transferred to the S 1-5 gene on the chromosome.

Those ES cells in which the incorporation of the transgene has been confirmed are returned into embryos derived from the same type of non-human mammal, thus enabling the incorporation of the cells into the cell mass of the host embryo, and chimeric embryos are formed. Known methods for introducing ES cells into embryos such as blastocysts include microinjection and agglutination. However, any methods may be used, and those skilled in the art may appropriately modify these methods.

When using mice, female mice subjected to superovulation treatment using hormone agents (using, for example, pregnant mare's serum gonadotropin (PMSG), which has a follicle stimulating hormone (FSH)-like action, and human chorionic gonadotrophin (hCG), which has a luteinizing hormone (LH) action) are mated with male mice. Thereafter, embryos in the early stage of development are collected from the uterus 3.5 days after fertilization when using blastocysts, and 2.5 days after fertilization when using eight-cell stage embryos. ES cells that are homologously recombined using a targeting vector are injected *in vitro* into embryos collected in this manner, producing chimeric embryos. Alternatively, the zona pellucida of two-day-old mice embryos (eight-cell stage embryos) is removed and cultured with ES cells to produce an aggregate. Blastocysts are produced by cultivating this aggregate for one day, and the blastocysts are then transplanted into foster mothers, developed, and grown to produce chimeric mice. Pseudopregnant female mice for use as foster mothers can be obtained by mating female mice with a normal estrous cycle with male mice castrated by vasoligation, or such. Chimeric mice can then be produced by transplanting chimeric embryos produced by a method described above into the uterus of the pseudopregnant mice thus produced, and then causing pregnancy and delivery. To increase the certainty that implantation of the chimeric embryos and pregnancy will take place, the female mice from which the fertilized eggs are collected and the pseudopregnant mice that become the foster mothers are preferably produced from a group of female mice with the same estrous cycle.

Then, chimeric mice are selected from those babies born to the foster mothers. If individual mice derived from the ES cell-transplanted embryos are obtained, they are crossed with wildtype mice to confirm whether or not the phenotype derived from the ES cell appears in second generation individuals. If the phenotype derived from the ES cell does appear in second generation individuals, one may assume that the ES cell was introduced into the germline of the chimeric mouse. Various phenotypes can be used as indicators to verify that the ES cell was introduced into the germline; however, for ease of verification it is desirable to use hair color as an indicator. Known hair colors for mice include agouti, black, ocher, chocolate, and white. It is also possible to make selections by extracting chromosomal DNA from a part of a body (for example from the tail tip) and then performing Southern blotting or PCR. It is very likely the ES cell has been introduced into the germline of chimeric mice whose chimeric contribution is high. As described above, a chimeric mouse is selected and then crossed with a wildtype male to obtain F1 individuals and then establish a mutant mouse strain.

A chimeric animal obtained as described above is a heterozygote with a genetic defect on only one of its homologous chromosomes. F1 heterozygotes comprising a genetic defect in only one of their homologous chromosomes can be crossed with each other to obtain a homozygous knockout animal in which both S1-5 genes on homologous chromosomes are defective.

Whether or not the obtained animal is a knockout animal is verified by extracting chromosomal DNAs from its tissues and then performing Southern blot analysis or PCR on them. Also, abnormalities in the tissues and organs can be observed at autopsy. In addition, RNA can be extracted from the tissues, and the gene expression pattern can be analyzed using Northern blot analysis. Blood can also be collected as necessary to carry out blood tests and serum biochemical tests.

Homozygous knockout animals produced at this point may suffer fetal death or such, or may not develop in to adults, making them inappropriate as model animals. In such cases, the gene is preferably knocked out at a required time. Further, to investigate the function of a gene in a specific tissue *in vivo,* the gene is preferably knocked out tissue-specifically. Such animals in which a gene is knocked out at a specific time or only from a specific cell line, and animals in which a gene is knocked out only in a limited region of somatic cells are called conditional knockout animals (Bio Manual Series 8, Gene Targeting: Production of mutant mice using ES cells, Aizawa, S., Yodosha, 1995).

The Cre-loxP system (R.Kuhn. et al., Science 269: 1427-1429, 1995), which is a recombination system derived from bacteriophage P1 for gene targeting, can be used as a method for producing conditional knockout animals. Cre is a recombinase and recognizes a 34-bp sequence called loxP, which allows recombination to take place at this site. Therefore, by placing a gene to be targeted between two loxP sequences, and inserting the Cre recombinase gene downstream of a specific promoter, Cre can be produced at a specific site and at a specific time, and the gene between the loxPs can be cut out (i.e. the function of a desired gene can be obliterated at a particular site and time). When designing a targeting vector using the Cre-loxP system in the present invention, the part that changes the structure of the S1-5 gene is not particularly limited, so long as the function of the S1-5 gene is lost. Considering the function and structure of the S1-5 gene, it is particularly preferable to design a targeting vector such that a region of the EGF-like domain or fibrin-like domain of the S1-5 gene is deleted.

### 3. Cells isolated from the non-human knockout animals

The present invention also provides cells isolated from the knockout animals of the present invention. As described below, cells isolated from the knockout animals of the present invention can be used to screen for preventive or therapeutic agents for age-related diseases or symptoms.

Examples of such cells include, but not limited to osteoclasts, keratinocyte epithelial cells, blood cells, cancer cells, bone marrow cells, fibroblasts, vascular endothelial cells, dermal cells, muscle cells, nerve cells, lymphocytes, vascular smooth muscle cells, synoviocytes, hair papilla cells, hepatocytes, pigment cells, adipocytes, uterine endothelial cells, or alveolar epithelial cells.

These cells can be isolated from the knockout animals by methods well known to those skilled in the art.

### 4. Age-related diseases

The non-human knockout animals of the present invention show an age-related disease or symptom. Known mouse models of senescence are Klotho mutant mice (Kuro-o, M. et al., Nature, 6; 390(6655):18-19, 1997), senescence accelerated model mice (SAM) (Takeda, T. et al., Mech. Ageing Dev., 17(2), 183-194, 1981), and Werner's syndrome model mice (Chen, L. et al., J. Biomed. Biotechnol., 2(2), 46-54, 2002); these mice demonstrate early senescence and are short lived. A characteristic of the non-human knockout animals of the present invention (for example, S1-5 knockout mouse) is that their lifespan is not different from that of wildtype mice, but that they have a variety of age-related diseases or symptoms that develop into serious conditions. In the present invention, the phrase "age-related disease or symptom" refers to, for example, bone deformation, osteoporosis, Paget's disease of bone, hyperparathyroidism, decreased bone mineral density, cancellous transformation of cortical bone, hair loss, tissue injury or necrosis, tumor, breast hypertrophy, ascites, anemia, bleeding, aging of skin (including, but not limited to blotches, dullness of skin, flabby skin, fine wrinkles, moles), and aging of nails, and means that such diseases or symptoms appear individually or in combination.

"Bone deformation" means that the strength of the osteocartilage is decreased due to aging, RA, or osteoporosis, and that the bones and cartilage are deformed. "Osteoporosis" refers to a condition in which the osseous components generally decrease, and fracture is likely to occur. More than 90% of osteoporosis is primary osteoporosis, for which an obvious causative disease is not found, and most of it is involutional osteoporosis that develops in middle-aged and elderly people. Development of secondary osteoporosis, which differs from the above, is caused by Basedow's disease, Cushing's syndrome, severe diabetes, RA, stomach surgery, alcohol polydipsia, use of steroidal agents, or such.

"Paget's disease of bone" means a bone disease characterized by increased bone turnover and disordered bone remodeling (Merck Manual of Medical Information homepage: http://mmh.banyu.co.jp/mmhe2j/sec05/ch061/ch061a.html). In Paget's disease of bone, osteoclasts and osteoblasts are excessively activated in certain regions of the bone, occurring bone resorption and bone remodeling. Those regions of the bone where excessive activation took place are known to become relatively fragile compared to normal bones.

"Hyperparathyroidism" refers to a condition in which secretion of parathyroid hormone PTH is increased, and its action is abnormally enhanced. It is known that in hyperparathyroidism, bone content decreases, osteoclasts are activated, and such. "Hyperparathyroidism" includes, but not limited to primary hyperparathyroidism, such as adenoma or the like that increase PTH secretion from the thyroid gland, and secondary hyperparathyroidism, in which the function of the parathyroid gland is secondarily enhanced after a cause such as chronic renal failure or pregnancy.

"Tumor" means all tumorigenic cell growth and proliferation, and all precancerous and cancerous cells and tissues, regardless of whether they are malignant or benign. The tumors of the present invention may be primary tumors or metastatic tumors. The terms "cancer" and "cancerous" typically refer to a physiological condition characterized by cell growth which has become uncontrollable. In the present invention, examples of "tumors" include carcinomas, sarcomas, leukemias, and malignant lymphomas. In the present invention, examples of carcinomas include breast cancer, prostate cancer, colon cancer, squamous cell carcinoma, small cell lung cancer, non-small-cell lung cancer, gastrointestinal cancer, pancreatic cancer, glioblastoma, uterine cervical cancer, ovarian cancer, liver cancer, bladder cancer, hepatoma, colorectal cancer, endometrial cancer, salivary gland cancer, renal cancer, vulva cancer, thyroid cancer, hepatic cancer, and various types of cancers of the head and neck. In the present invention, examples of sarcomas include malignant osteoma, and malignant soft tissue sarcoma, or more specifically, osteosarcoma, chondrosarcoma, Ewing's sarcoma, liposarcoma, leiomyosarcoma, or synovial sarcoma.

### 5. Screening for therapeutic agents or preventive agents for age-related diseases

The present invention provides methods of screening for therapeutic or preventive agents for age-related diseases or symptoms, in which the methods comprise administering a candidate substance to a non-human knockout animal.

In these screening methods, substances with the activity of complementing the phenotype of a knockout animal are selected from among the candidate substances for preventive or therapeutic agents for age-related diseases or symptoms. Complementing the phenotype of the knockout animal includes not only complete but also incomplete complementation.

More specifically, a candidate substance is contacted with a knockout animal of the present invention or a part thereof, and an indicator value correlated with the targeted disease is measured in the non-human animal or the part thereof that was contacted with the candidate substance. The value is compared with that of a control, and based on this comparison, the effect of the candidate substance on the desired age-related disease is evaluated. Preventive, therapeutic, and remedial effects of the candidate substance can be tested using an increase in bone mineral density, increased bone strength, functional inhibition of osteoclasts, anti-tumor effect, increase of blood cells, improved hemostatic ability, or such as the indicator. Tests can also be carried out on animals that show aging of skin (blotches, dullness of skin, flabby skin, fine wrinkles, moles, etc.) to screen for cosmetics that yield whitening and anti-aging effects.

A "knockout animal or a part thereof' includes both the animal's entire body and specific tissues or organs. Specific tissues or organs also include those removed from the animal.

Candidate substances may be, for example, peptides, proteins, non-peptide compounds, synthetic compounds, fermentation products, cell extracts, plant extracts, animal tissue extracts, plasmas, and such, and these compounds may be novel compounds or publicly known compounds. Such candidate substances may form salts, and salts of the candidate substances that are used include physiologically acceptable acids (such as inorganic acids) and bases (such as organic acids), and in particular, physiologically acceptable acid addition salts are preferred. Examples of such salts include salts formed with inorganic acids (for example, hydrochloric acid, phosphoric acid, hydrobromic acid, and sulfuric acid), or salts formed with organic acids (for example, acetic acid, formic acid, propionic acid, fumaric acid, maleic acid, succinic acid, tartaric acid, citric acid, malic acid, oxalic acid, benzoic acid, methanesulfonic acid, and benzenesulfonic acid).

The methods that may be used for treating test animals with candidate substances include oral administration, intravenous injection, swabbing, subcutaneous administration, intradermal administration, and peritoneal administration, and can be selected appropriately depending on the symptom of the test animals, properties of the candidate substance, and such. The dose of a candidate substance can be selected appropriately according to the method of administration, properties of the candidate substance, and such.

For example, when screening for preventive, therapeutic, or remedial agents for osteoporosis, effective substances can be screened by administering a candidate substance to a knockout animal of the present invention, and then measuring bone mineral density, bone strength, X-ray photographs and so on of the animal over time. In particular, since osteoporosis and spinal kyphosis are correlated in the knockout mice, the present invention suggested that the degree of kyphosis in the knockout animal may be an indicator for measuring the progress of osteoporosis. Therefore, the efficacy of a candidate substance on osteoporosis can be determined non-invasively and efficiently by administering a candidate substance to a knockout animal of the present invention, and using X-ray photographs and such to determine the degree of kyphosis in the animal over time.

Cells isolated from the knockout animals of the present invention can be used to screen for preventive or therapeutic agents for age-related diseases or symptoms. More specifically, the present invention provides methods of screening for preventive or therapeutic agents for age-related diseases or symptoms, in which the methods comprise a step of contacting a candidate substance for the preventive or therapeutic agent with a cell isolated from a knockout animal of the present invention.

In the present invention "contact" can be carried out, for example, by adding a candidate substance to a culture medium of cells isolated from a knockout animal of the present invention. For example, if the candidate substance is a protein, "contact" can be carried out by transfecting cells isolated from the knockout animal with a vector comprising a DNA encoding this protein.

In the present invention, a substance with the activity of complementing the phenotype of a cell isolated from a knockout animal is selected from among candidate substances for preventive or therapeutic agents for age-related diseases or symptoms. Without particular limitation, the activity of complementing the phenotype of a cell isolated from a knockout animal includes, for example, the activity of suppressing osteoclast activity for osteoclasts, suppressing osteoclast formation for bone marrow cells, keratinocyte epithelial cell growth for epithelial cells, promoting blood cell differentiation for blood cells, suppressing cancer cell growth for cancer cells, fibroblast growth for fibroblasts, vascular endothelial cell growth for vascular endothelial cells, hair formation for dermal cells, correcting muscle cell degeneration for muscle cells, correcting nerve cell degeneration for nerve cells, suppressing lymphocyte activation for lymphocytes, inhibiting growth for vascular smooth muscle cells, normalizing function for synoviocytes, hair growth for hair papilla cells, normalizing function for hepatocytes, suppressing melanin production for pigment cells, correcting activation abnormality for alveolar cells, suppressing differentiation and proliferation for adipocytes, and inhibiting endothelial cell growth for uterine endothelial cells. Complementing cell phenotype includes not only complete but also incomplete complementation.

### 6. Screening for agents that inhibit osteoclast function

The present invention also provides methods of screening for agents that inhibit osteoclast function, in which the methods comprise a step of contacting a candidate substance for an agent that inhibits osteoclast function with osteoclasts isolated from a knockout animal. Examples of "osteoclast function" include, but not limited to resorption lacunae formation, TRAP producing ability, actin ring forming ability, protease (cathepsin K, MMP-9) producing ability, and acid secreting ability, but as long as the function directly or indirectly leads to bone destruction, it is included in "osteoclast function".

Agents that inhibit osteoclast function can be screened by, for example, using as an indicator the formed number and size of osteoclasts isolated from a knockout animal of the present invention. However, the screening is not limited to these methods, and for example, as indicated in the Examples, screening can be carried out by differentiating bone marrow cells isolated from a knockout animal of the present invention into multinucleated giant cells, and then using as indicators the number, size and so on of TRAP-positive multinucleated giant cells (Takahashi N. et al., Endocrinology, 123(5):2600-2, 1988; Yasuda H. et al., Proc. Natl. Acad. Sci. U S A., 31;95(7), 3597-602, 1998).

Agents that inhibit osteoclast function can be screened by using the ability to form resorption lacunae as an indicator (Hirayama, T. et al., J. Endocrinol.: Oct175(1):155-63 2002; Udagawa, N. et al., Bone.: Nov;25(5):517-23 1999).

Substances selected by this screening method can be used in the fields of research and medicine as agents that inhibit osteoclast function. The agents that inhibit osteoclast function of the present invention may find application as preventive or therapeutic agents for age-related diseases or symptoms.

When the forefront of a proliferated synovial membrane invading into the bone is examined pathologically at the site of osteolysis in RA patients, many multinucleated giant cells are found. Such cells are TRAP positive; that is, they are known to fit the phenotype of osteoclasts, and osteoclasts presumably have an important role in osteolysis in RA (Urushibara, M. et al., Arthritis Rheum. Mar;50(3):794-804, 2004; Takayanagi, H. et al. Biochem. Biophys. Res. Commun. Nov 17;240(2):279-86, 1997). Therefore, the agents that inhibit osteoclast function of the present invention can be used as preventive or therapeutic agents of RA.

### 7. Isolated S1-5 protein

The present invention provides 1) isolated S1-5 protein encoded by a DNA comprising the coding region of the nucleotide sequence of SEQ ID NO: 1 or 3; and 2) isolated S1-5 protein comprising the amino acid sequence of SEQ ID NO: 2 or 4. Herein, "isolated" refers to a condition of being taken out of the natural environment.

The above-mentioned proteins can be prepared, for example, as recombinants. For example, in the case of human S1-5 protein, a cDNA library is obtained based on mRNAs extracted from cells that express human S1-5 protein (for example, human diploid fibroblasts, and RA patient-derived synovial cells collected as synovial tissues or cultured cells) (Short, J.M. et al., Nucleic Acid Research, 16, 7583,1988). DNAs that encode the S1-5 protein can be isolated by screening this library for hybridizing clones using a probe designed on the basis of nucleotide sequence of SEQ ID NO: 1. S 1-5 protein encoded by this DNA can be obtained using a protein expression system well known to those skilled in the art. Human S1-5 protein can be collected and purified from cultures of cells that express the human S1-5 protein.

The present invention also provides proteins that are functionally equivalent to the above-mentioned S1-5 protein. The biological species from which such proteins are derived include, without limitation, humans, zebrafish, mice, rats, guinea pigs, rabbits, chickens, pigs, sheep, goats, dogs, cattle, monkeys, and chimpanzees.

Proteins functionally equivalent to the S1-5 protein include 1) isolated proteins comprising an amino acid sequence with one or more amino acid substitutions, deletions, insertions, and/or additions in the amino acid sequence of SEQ ID NO: 2 or 4, wherein the protein is functionally equivalent to a protein comprising the amino acid sequence of SEQ ID NO: 2 or 4; and 2)isolated proteins encoded by a DNA that hybridizes under stringent conditions with a DNA comprising the nucleotide sequence of SEQ ID NO: 1 or 3, wherein the protein is functionally equivalent to a protein comprising the amino acid sequence of SEQ ID NO: 2 or 4.

The proteins functionally equivalent to the S1-5 protein include proteins having the function of suppressing age-related diseases or symptoms of humans, and proteins having the function of complementing the phenotype of the knockout animals of the present invention or the cells isolated therefrom.

Proteins functionally equivalent to S1-5 protein may include proteins that are immunologically equivalent. In the present invention, "proteins that are immunologically equivalent to S1-5 protein" are not particularly limited, so long as the proteins react with antibodies that specifically recognize S1-5 protein. Examples of proteins immunologically equivalent to S1-5 protein include epitope peptides of the S1-5 protein, domains of the S1-5 protein comprising these epitopes, or proteins comprising these domains.

S1-5 protein fragments can be obtained by digestion using proteases. Alternatively, they can be obtained by randomly digesting DNAs encoding the S1-5 protein, which is shown in SEQ ID NO: 1 or 3, and then inserting these fragments into phage vectors to produce phage libraries that display domain peptides. Immunologically active domains can be determined by immunoscreening these phage libraries using antibodies that recognize S1-5 protein. The amino acid sequence of an active domain can be elucidated by sequencing the insert of the cloned phage.

The proteins functionally equivalent to S1-5 protein of the present invention are defined not only in terms of immunological characteristics, but also in terms of binding characteristics with synoviolin. More specifically, the present invention comprises S1-5 protein fragments with affinity towards synoviolin. Those skilled in the art can readily select such mutants by using synoviolin to screen candidate proteins. For example, the S1-5 protein requires 120 amino acid residues, corresponding to positions 1233-1592 in the cDNA of synoviolin, as a region necessary for binding with synoviolin. Therefore, proteins that bind to a protein consisting of an amino acid sequence that constitutes this region, or to a protein that comprises this amino acid sequence constitute the proteins functionally equivalent to S1-5 protein of the present invention.

In addition, the proteins functionally equivalent to S1-5 protein of the present invention are also defined in terms of the biochemical activity possessed by the S1-5 protein. Examples of S 1-5 protein biochemical activities include the activity of inhibiting osteoclast formation, and the activity of regulating cell growth (Leucka-Czernik, B. et al., Mol. Cell. Biol. 15: 120-128, 1995).

These proteins functionally equivalent to S 1-5 protein can be made into fusion proteins with other proteins. For example, the functionally equivalent proteins include proteins that maintain at least one characteristic of a protein functionally equivalent to S1-5 protein and to which an additional amino acid sequence, such as FLAG tag, HA tag, histidine (His) tag, glutathione-S-transferase (GST) tag, or maltose binding protein (MBP) tag, has been added. Even if the protein to be added to comprises activity different from that of S1-5 protein, if the fusion protein maintains at least one of the functions of the S1-5 protein, that fusion protein is included in the functionally equivalent proteins of the present invention.

Proteins functionally equivalent to the S1-5 protein can be isolated by methods well known to those skilled in the art (Experimental Medicine Supplementary Volume: Genetic Engineering Handbook, pp.246-251, Yodosha, 1991). For example by screening a desired library using the nucleotide sequence of SEQ ID NO: 1 or 3 (or a fragment thereof) as a probe, DNAs with a highly homologous nucleotide sequence can be cloned. Examples of such libraries include libraries in which random mutations have been introduced to the nucleotide sequence of SEQ ID NO: 1 or 3, and cDNA libraries of synovial tissues derived from human or non-human species.

Known methods for introducing random mutations to a given nucleotide sequence include substitution of base pairs by nitrous acid treatment of DNA (Hirose, S. et al., Proc. Natl. Acad. Sci. USA. 79: 7258-7260, 1982). In this method, base pair substitutions can be introduced randomly into a specific segment in which mutations are desired by performing nitrous acid treatment on that segment. Alternatively, an example of a technique for introducing a desired mutation at a discretionary site is the gapped duplex method (Keamer, W. et al., Methods in Enzymol. 154: 350-367, 1987). The gene that should incorporate the mutation is cloned into a cyclic double-stranded vector, and this vector is separated into single strands and then hybridized with a synthetic oligonucleotide that carries a mutation at a desired site. A complementary single-stranded DNA derived from a vector linearized by restriction enzyme cleavage is annealed to the cyclic single-stranded vector, the gap between the synthetic nucleotide is filled using DNA polymerase, and then further ligation gives a complete double-stranded cyclic vector.

The number of modified amino acids may be typically 50 amino acids or less, preferably 30 amino acids or less, and more preferably five amino acids or less (for example, one amino acid).

When artificially substituting an amino acid, substitution using an amino acid with similar properties should facilitate maintenance of the activity of the original protein. The proteins of the present invention include proteins in which the above-mentioned amino acid substitution was a conservative substitution, where the proteins are functionally equivalent to human S1-5 protein (SEQ ID NO: 2 or 4). Conservative substitution is considered important when substituting amino acids of domains important for protein activity. This kind of conservative amino acid substitution is well known to those skilled in the art.

Groups of amino acids suited to conservative substitution are, for example, basic amino acids (such as lysine, arginine, and histidine), acidic amino acids (such as aspartic acid and glutamic acid), uncharged polar amino acids (such as glycine, asparagine, glutamine, serine, threonine, tyrosine and cysteine), non-polar amino acids (such as alanine, valine, leucine, isoleucine, proline, phenylalanine, methionine and tryptophan), β-branched amino acids (such as threonine, valine and isoleucine), and aromatic amino acids (such as tyrosine, phenylalanine, tryptophan and histidine).

The activity of a protein may be increased (including, for example, constitutively activated proteins) or decreased (including, for example, dominant negative proteins) by non-conservative substitution.

Proteins comprising an amino acid sequence with one or more amino acid substitutions, deletions, insertions, and/or additions in the amino acid sequence of SEQ ID NO: 2 or 4, wherein the proteins are functionally equivalent to a protein comprising the amino acid sequence of SEQ ID NO: 2 or 4, include naturally existing proteins. In general, eukaryote genes show polymorphisms, as seen in interferon genes and such. There are cases where differences in nucleotide sequence due to this polymorphism may cause substitution, deletion, insertion and/or addition of one or more amino acids. Therefore, naturally existing proteins comprising an amino acid sequence with one or more amino acid substitutions, deletions, insertions, and/or additions in the amino acid sequence of SEQ ID NO: 2 or 4, wherein the proteins are functionally equivalent to a protein comprising the amino acid sequence of SEQ ID NO: 2 or 4, are included in the present invention.

Alternatively, in some cases polymorphism can cause a change the nucleotide sequence, but the amino acid sequence does not change. Such nucleotide sequence mutations are called silent mutations. Proteins encoded by DNAs comprising a nucleotide sequence with silent mutations are also included in the present invention. Herein, polymorphism means that the nucleotide sequence of a certain gene differs between individuals within a group. Polymorphism is unrelated to the proportion of different genes found.

In addition, hybridization can be included as an example of a method that can be used to obtain proteins functionally equivalent to S1-5 protein. More specifically, DNAs which encode an S1-5 protein of the present invention, and which are shown in SEQ ID NO: 1 or 3, or fragments thereof, are used as probes to isolate DNAs that hybridize with the probes. When hybridization is carried out under stringent conditions, DNAs with highly homologous nucleotide sequences will be selected, and as a result, there will be an increased possibility that the isolated proteins will comprise proteins functionally equivalent to S1-5 protein. For example, highly homologous nucleotide sequences refer to sequences with identity of 70% or more, and desirably 90% or more.

Stringent conditions specifically refer to, for example, hybridization in 6x SSC and 40% formamide at 25°C, and then washing with 1x SSC at 55°C. Stringency is influenced by conditions such as salt concentration, formamide concentration, and temperature, but those skilled in the art can obviously set these conditions to yield necessary stringencies.

The use of hybridization enables isolation of, for example, a DNA encoding a homolog of the S1-5 protein in a non-human animal species. Homologs of the S1-5 protein encoded by DNAs obtainable from non-human animal species, or more specifically, from zebrafish, mice, rats, guinea pigs, rabbits, chickens, pigs, sheep, goats, dogs, cattle, monkeys, chimpanzees, and such, constitute the functionally equivalent proteins of the present invention.

Proteins obtained by introducing a mutation into the S1-5 protein (SEQ ID NO: 2 or 4), or proteins encoded by a DNA isolated using a hybridization technique and such ordinarily share high homology in the amino acid sequences with that of S1-5 protein (SEQ ID NO: 2 or 4). High homology refers to sequence identity of at least 30% or more, preferably 50% or more, and even more preferably 80% or more (for example, 95% or more). The identity of nucleotide sequences or amino acid sequences can be determined on the internet using a homology search site. [For example, homology searches such as FASTA, BLAST, PSI-BLAST, and SSEARCH can be used through the DNA Data Bank of Japan (DDBJ) [for example, the Search and Analysis page on the website of DNA Data Bank of Japan; http://www.ddbj.nig.ac.jp/E-mail/homology-j.html]. Searches using BLAST can be performed through the National Center for Biotechnology Information (NCBI) (for example, the BLAST page on the NCBI homepage; http://www.ncbi.nlm.nih.gov/BLAST/; Altschul,S. F. et al., J. Mol. Biol. 215(3): 403-10, 1990; Altschul, S. F. et al., Meth. Enzymol. 266:460-480, 1996; Altschul, S. F. et al., Nucleic Acids. Res. 25: 3389-3402, 1997)].

For example, when calculating amino acid sequence identity using Advanced BLAST 2.1, a search is carried out using blastp as the program, setting the Expect value to 10, setting all filters to OFF, using BLOSUM62 as the Matrix, and setting the Gap existence cost, Per residue gap cost, and Lambda ratio to 11, 1, and 0.85 respectively (default values), and then obtaining a value (%) for identity (Karlin, S. et al., Proc. Natl. Acad. Sci. USA 87:2264-68 1990; Karlin, S. et al., Proc. Natl. Acad. Sci. USA 90:5873-7 1993).

The proteins of the present invention, or proteins functionally equivalent to those proteins, may be proteins subjected to various modifications, such as physiological modification by glycoside chains, labeling with fluorescent, radioactive, or such substances, or fusion with another protein. In particular, the recombinants described later may be glycosylated differently depending on the host used for expression. However, even if there are differences in glycoside modification, if the proteins show characteristics similar to those of the S1-5 protein described in the present description, they are considered to be the S1-5 protein or a functionally equivalent protein of the present invention.

The S1-5 protein can be obtained not only as a biological material but also as a recombinant protein by incorporating a gene encoding this protein into an appropriate expression system. In order to obtain the S1-5 protein by genetic engineering methods, the aforementioned DNA encoding the S1-5 protein can be incorporated into a suitable expression system, and then expressed. Host/vector systems applicable to the present invention include, for example, expression vector pGEX and *E. coli.* Since pGEX enables a foreign gene to be expressed as a fusion protein with GST (Smith DB, et al., Gene, 67:31-40, 1988), pGEX carrying a gene encoding the S1-5 protein is transfected into an E. *coli* strain such as BL21 by heat shock, and after incubating for an appropriate period of time, isopropylthio-β-D-galactoside (IPTG) is added to induce expression of the GST-fused S1-5 protein. A gene encoding the S1-5 protein can be obtained by amplification using methods such as PCR, using a synoviocyte cDNA library or such as a template. Since the GST of the present invention adsorbs onto Glutathione Sepharose 4B, expression products can be easily separated and purified by affinity chromatography.

In addition, the following may be applied as a host/vector system for obtaining a recombinant S1-5 protein. First, when using bacteria as the host, one may use commercially available vectors for expressing fusion proteins, which use a histidine tag, HA tag, FLAG tag, GST tag, MBP tag, or such. In the case of yeast, *Pichia* yeast is well known to be effective for expressing glycosylated proteins. In terms of glycosylation, expression using a baculovirus vector whose host is insect cells is also useful (Bio/Technology, 6:47-55, 1988). Furthermore, vectors that utilize promoters of CMV, RSV, SV40 or such can be transfected into mammalian cells, and all of these host/vector systems can be used as S1-5 protein expression systems. Genes can also be introduced using viral vectors such as retrovirus vectors, adenovirus vectors, or adeno-associated virus vectors.

The obtained proteins of the present invention can be isolated from the inside or outside of the host cells (the media or such), and can be purified as substantially pure and homogeneous proteins. Without limitation, separation and purification of the proteins can be carried out using the separation and purification methods used for ordinary protein purification. For example, proteins can be separated and purified by appropriately selecting and combining column chromatography, filtration, ultrafiltration, salt precipitation, solvent precipitation, solvent extraction, distillation, immunoprecipitation, SDS-polyacrylamide gel electrophoresis, isoelectric focusing, dialysis, and recrystallization.

Examples of chromatography include affinity chromatography, ion exchange chromatography, hydrophobic chromatography, gel filtration, reverse phase chromatography, and adsorption chromatography (Marshak et al., Strategies for Protein Purification and Characterization: A Laboratory Course Manual. Ed Daniel R. Cold Spring Harbor Laboratory Press, 1996). These types of chromatography can be performed as liquid phase chromatography, such as HPLC or FPLC.

For the proteins of the present invention an expression system other than a cell-free system is preferably used. The proteins of the present invention are preferably proteins modified by physiological conformation, glycosylation, disulfide bond, lipidation, methylation, or such. Examples of the glycoside chains of the present invention include N-linked glycosides and O-linked glycosides. Examples of N-linked glycosides include those with high mannose type, hybrid type, and complex type; examples of O-linked glycosides include those with a mucin (O-Ag1NAc) type, O-Fuc type, O-Man type, and O-Glc type.

The proteins of the present invention are preferably substantially purified proteins. Herein, "substantially purified" means that the purity of a protein of the present invention (the proportion of the protein of the present invention in the entire protein component) is 50% or more, 60% or more, 70% or more, 80% or more; 90% or more, 95% or more, or 100% or close to 100%. Close to 100% the upper limit depends on the purification and analysis technique used by those skilled in the art, and is 99.999%, 99.99%, 99.9%, 99%, or such.

Proteins with the above-mentioned purity are included as substantially purified proteins, regardless of the purification method used for that protein. Examples of such include, without limitation, proteins substantially purified by appropriately selecting or combining the above-mentioned column chromatography, filtration, ultrafiltration, salt precipitation, solvent precipitation, solvent extraction, distillation, immunoprecipitation, SDS-polyacrylamide gel electrophoresis, isoelectric focusing, dialysis, recrystallization, and such.

The present invention also provides partial peptides (partial fragments) of the proteins of the present invention. The length of a partial peptide is not particularly limited, as long as the partial peptide is functionally equivalent to a protein of the present invention. An example of a partial peptide of the present invention is a peptide shorter than a protein of the present invention, and comprising the amino acid sequence of SEQ ID NO: 6. Using such a peptide can prevent or treat age-related diseases or symptoms, and inhibit osteoclast activity.

### 8. Pharmaceutical agents

The present invention provides preventive or therapeutic agents for age-related diseases or symptoms, wherein the agents comprise a protein of the present invention or a partial peptide thereof (hereinafter referred to as proteins). An "age-related disease or symptom" is as described above. Furthermore, the present invention provides agents that inhibit osteoclast function, wherein the agents comprise a protein of the present invention. The proteins and such of these pharmaceutical agents are not particularly limited, as long as they are isolated, and can be used regardless of whether they are substantially or crudely purified proteins, as long as they can be used as the above-mentioned pharmaceutical agents. The proteins in the pharmaceutical agents are preferably produced in an expression system other than a cell-free system.

These pharmaceutical agents can be utilized as preventive or therapeutic agents for age-related diseases or symptoms, or as agents for inhibiting osteoclast function in humans or non-human animals (such as laboratory animals, livestock animals, and pet animals).

The proteins of the present invention may be administered after formulation by appropriate combination with pharmaceutically acceptable carriers or media, such as sterilized water or physiological saline, stabilizers, fillers, antiseptics, surfactants, chelating agents (EDTA and such), and binders.

Examples of the forms (dosage forms) of the pharmaceutical agents of the present invention include, without limitation, injections, freeze-dried agents, and solutions.

Administration to patients may be either oral or parenteral, but is preferably parenteral administration, such as administration by injection. Examples of administration by injection include systemic or local administration by intravenous injection, intramuscular injection, intraperitoneal injection, subcutaneous injection, intradermal injection, and such.

The dose varies depending on the weight and age of the patient, the method of administration, symptoms, and such, but those skilled in the art can appropriately select suitable doses. The conventional dose differs depending on the effective blood concentration and metabolism time of the pharmaceutical agent, but the daily maintenance dose may be approximately 0.1 mg/kg to approximately 1.0 g/kg, preferably approximately 0.1 mg/kg to approximately 10 mg/kg, and more preferably approximately 0.1 mg/kg to approximately 1.0 mg/kg. The dose can be administered in one to several dosages.

### 9. Antibodies

The present invention provides antibodies that recognize the S1-5 proteins. Using the S1-5 protein of the present invention or a fragment thereof as the immunogen, antibodies against the S1-5 proteins may be obtained by known methods (Harlow, E. et al., Antibodies; A Laboratory manual. Cold Spring Harbor, New York, 1988; Kohler, G. et al., Nature 256: 495-7, 1975).

For immunization, an S1-5 protein of the present invention or fragment thereof was immunized into immunization animals with an appropriate adjuvant. The S1-5 protein fragment can be conjugated to a carrier protein to produce an immunogen. Keyhole limpet hemocyanin (KLH) or bovine serum albumin (BSA) may be used as a carrier protein for obtaining the immunogen.

Rabbits, mice, rats, goats, sheep, or such are conventionally used as animals for immunization. Freund's complete adjuvant (FCA) and such are conventionally used as the adjuvant (Freund, J., Adv. Tubercl. Res., 1:130-148, 1956). Boosters are given at appropriate intervals, and when an increase of antibody titer has been confirmed, blood is collected and antiserum can be obtained. Further purification of this antibody fraction can yield purified antibodies (polyclonal antibodies).

Monoclonal antibodies can be obtained by collecting antibody-producing cells and then cloning using cell fusion or the like. Monoclonal antibodies are important tools for accomplishing high sensitivity and specificity in immunoassays. As an alternative method for obtaining antibodies that recognize S1-5 proteins, a DNA encoding synoviolin can be randomly fragmented, and the fragments can be introduced into phage vectors to produce phage libraries that display domain peptides. Domains having immunological activity can be identified by immunoscreening such libraries using antibodies that recognize synoviolin.

Cells derived from immune animals can be used as antibody producing cells. Furthermore, chimeric antibodies and humanized antibodies can be constructed from the antibody genes of monoclonal antibody producing cells derived from immune animals obtained as described above. When administering antibodies to humans, animal antibodies are undesirable since they will be eliminated as foreign substances. Therefore, it is necessary to use chimeric antibodies, in which the constant regions of highly antigenic antibodies are substituted with those of human antibodies; or to use humanized antibodies in which the frameworks of the variable regions in addition to the constant regions are substituted with those of humans.

Chimeric antibodies or humanized antibodies that recognize a S1-5 protein of the present invention are useful for drug delivery systems (DDSs). With regards to DDSs that use antibodies that recognize a S1-5 protein of the present invention, an example of a substance that may be useful when linked to an antibody is a substance that inhibits osteoclast function (bisphosphonate, osteoprotegerin).

The antibodies of the present invention can be used as immunological agents for detecting S1-5 proteins. Methods for using antibodies to immunologically detect proteins present in the tissues and blood are well known.

The antibodies of the present invention can be used to separate or detect S1-5 proteins, or cells that express S1-5 proteins. Protein isolation and purification methods using antibodies are well known to those skilled in the art. The S1-5 proteins of the present invention are used as markers for synoviocytes and human diploid fibroblast cells. More specifically, synoviocytes and human diploid fibroblasts can be detected or separated using S1-5 protein expression as an indicator. Antibodies are labeled appropriately using fluorescence and such. For example, cells expressing the S1-5 proteins can be separated by cell sorting using antibodies against the S1-5 proteins.

All prior art documents cited herein are incorporated herein by reference.

Herein below, the present invention will be specifically described using Examples. However, the present invention is not be construed as being limited thereto.

### [Example 1]

### Cloning of the S1-5 gene and production of targeting vectors

Using a cDNA expression library derived from the synoviocytes of RA patients, screening was carried out for factors that bind to synoviolin (Tadaomi Takenawa, Toshiki Watanabe, eds., Baiomanyuaru UP Shirizu "Tampakushitsu no Bunshikan Sogosayo Jikken Ho" [Bio-Manual UP Series "Experimental Methods for Protein Intermolecular Interactions"], pp. 66-67, Yodosha Co., Ltd.; Kaelin, W. G. et al., Cell 70, 351-364, 1992; Skolnik, E. Y. et al., Cell 65, 83-90, 1991; Sambrook, J. et al., Molecular Cloning, a laboratory manual second edition, Cold Spring Harbor Laboratory Press 12.16-12.20, 1989). E. *coli* (XL1-Blue MRF') was infected with the library phage by incubation for 20 minutes at 37°C, then mixed with top agarose and spread onto a plate. This was cultured for 3.5 hours at 42°C. A nitrocellulose membrane was then soaked in 10 mM IPTG, dried, and placed on the plate. Culturing was performed for an additional 3.5 hours at 37°C. The membrane was recovered, then washed five times for five minutes in a washing buffer [10 mM Tris-HCl (pH 8.0), 0.5% skim milk, 0.1% Triton X-100, 150 mM NaCl, 1 mM EDTA, 5 mM MgCl₂, 1 mM DTT, protease inhibitor (complete, Boehringer Mannheim Corporation)] and soaked for one hour in a blocking buffer [10 mM Tris-HCl (pH 8.0), 5% skim milk, 0.1% Triton X-100, 150 mM NaCl, 1 mM EDTA, 5 mM MgCl₂, 1 mM DTT, 5% glycerol, protease inhibitor (complete, Boehringer Mannheim Corporation)]. After the five-minute washing was performed five times with the washing buffer, GST-Synoviolin ³²P-labeled with protein kinase A was added as a probe (approximately 10⁶ cpm/ml), and incubation was performed. Washing was performed repeatedly while changing the washing buffer until the count per membrane was approximately 1 kcpm, and then the signal was detected by autoradiography. As a result, a clone bound to Synoviolin was obtained. The nucleotide sequence of the cDNA of this clone was determined for the 100 bp nearest its 5' end and the 100 bp nearest its 3' end. Database searches were performed based on the obtained nucleotide sequence information, and the sequences in the 100 bp end portions were found to be the same as those of a known gene: S1-5 (also called EFEMP-1, FBNL, or FBLN-3) [Lecka-Czemik, B. et al., Molecular and Cellular Biology, 15, 120-128, 1995; accession number U03877 (cDNA), AAA65590 (protein); Stone, E. M. et al., Nature Genetics 22, 199-202, 1999; accession number Q12805 (protein)]. The sizes of both genes and their translation products are roughly the same, suggesting that they are the same protein. In the present invention, the cDNA sequence and amino acid sequence of human S1-5 are shown in SEQ ID NOs: 1 and 2, respectively.

The lacZ gene was introduced into the translation initiation site of the mouse S1-5 gene fragment (the ATG codon that is translated into the first methionine) to construct a targeting vector (Fig. 1). A neomycin resistance gene was inserted as a marker gene, and the diphtheria toxin A gene was also linked to enable exclusion of cell lines in which non-homologous recombination occurs. In the present invention, the cDNA sequence, amino acid sequence, and genomic sequence of mouse S1-5 are shown as SEQ ID NOs: 3, 4, and 5 respectively.

### [Example 2]

### Preparation of S1-5 knockout mice

Electroporation was used to introduce the targeting vector of Example 1 into a mouse TT-2 cell strain, and cell lines in which homologous recombination occurred were selected. The obtained cells were injected into eight-cell stage embryos and either directly transplanted to the fallopian tubes of a surrogate mother, or transplanted to the uterus of a surrogate mother after being cultured for one day to develop into a blastocyst. The obtained heterozygous mutant mice (F1) were crossed with each other to obtain heterozygous and homozygous mutant mice. In the mutant mice thus obtained, tissues that should express S1-5 will express β-galactosidase protein instead.

Genotype was confirmed using Southern blot analysis. DNA was extracted from the mice about two weeks after birth, at a point roughly 3 mm from the tip of the tail. The obtained DNA was digested with restriction enzyme BamHI before use. Bands were detected at 2.4 kbp in the wild type, at 5.4 kbp in homozygous mutant mice, and at both positions in heterozygous mutant mice (Fig. 2A). Northern blot analysis could not confirm mRNA expression of the S 1-5 gene in the lungs (Fig. 2B).

### [Example 3]

### Analysis of S1-5 knockout animals

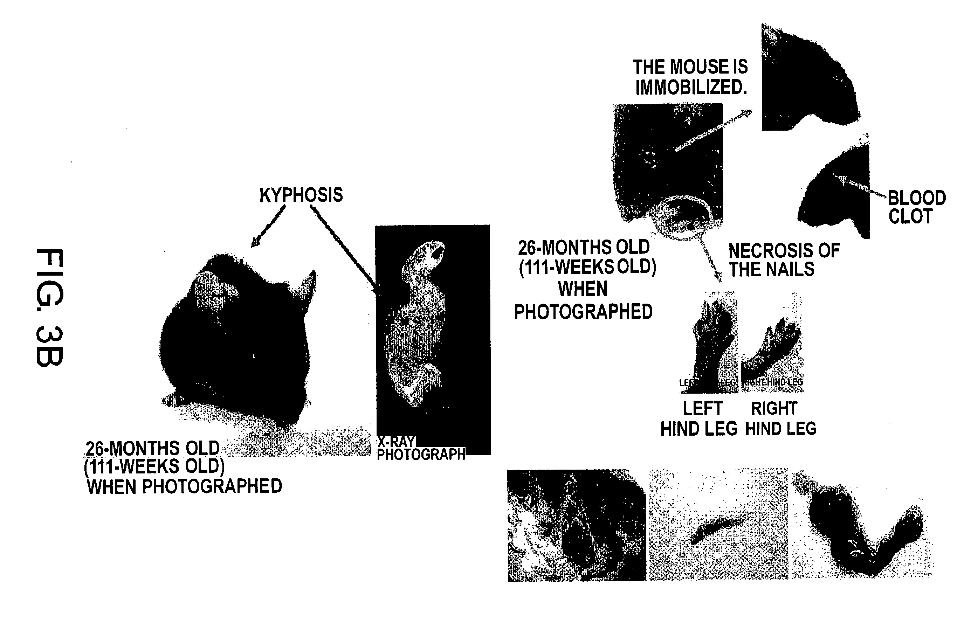

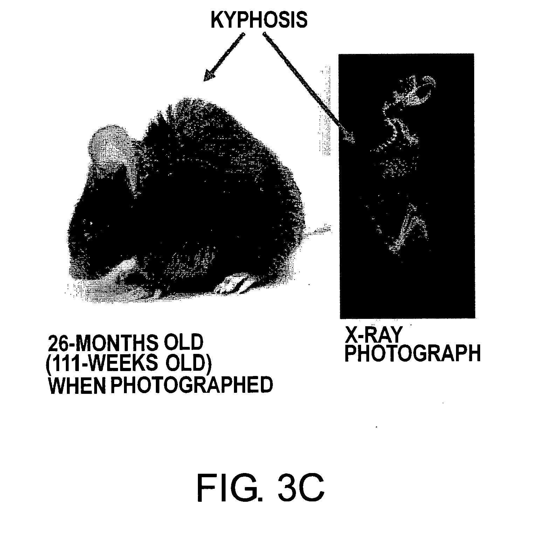

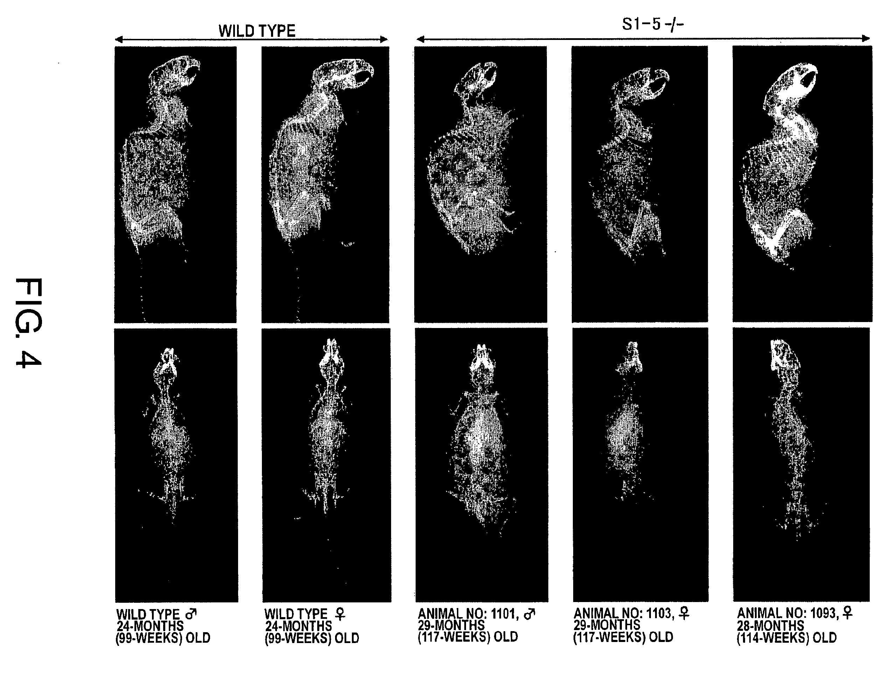

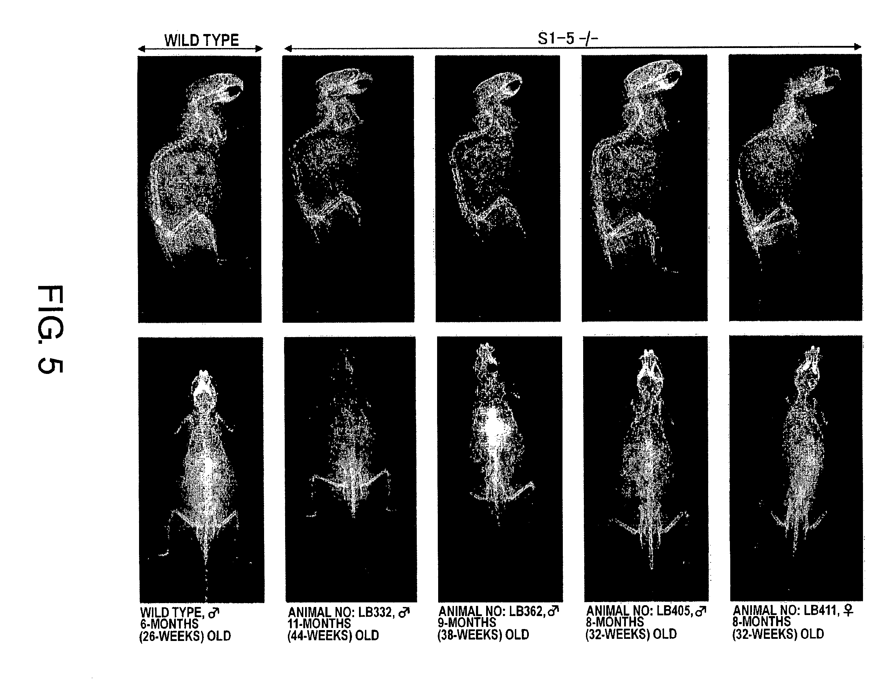

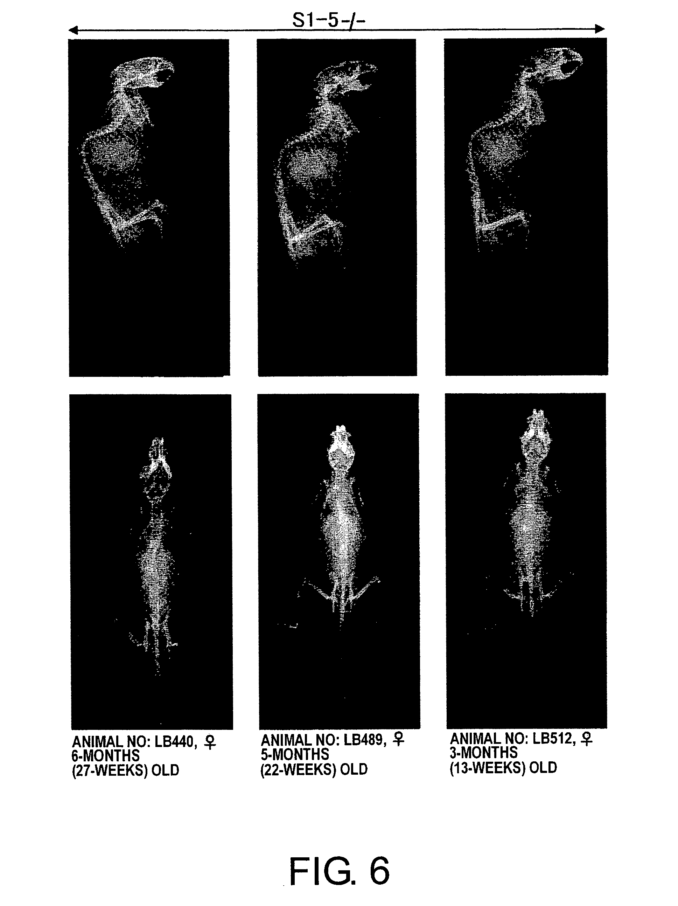

S1-5 knockout mice aged 13 to 117 weeks (three to 29 months) were analyzed using autopsy and X-ray photography. As a result, older mice (eight months or older) were observed to have kyphosis, hair loss, facial skin injuries, necrosis of the nails, breast hypertrophy, ascites, liver tumors, blood clots in the ocular fundus and adipose tissues, and uterine swelling (Figs. 3 to 6).

### [Example 4]

### Analysis of kyphosis in S1-5 knockout mice

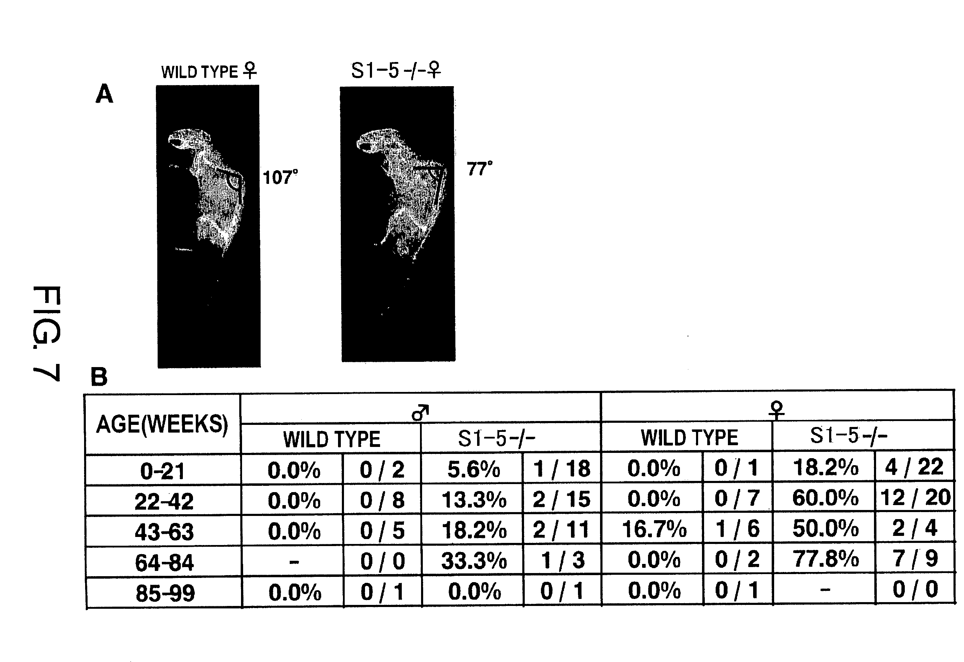

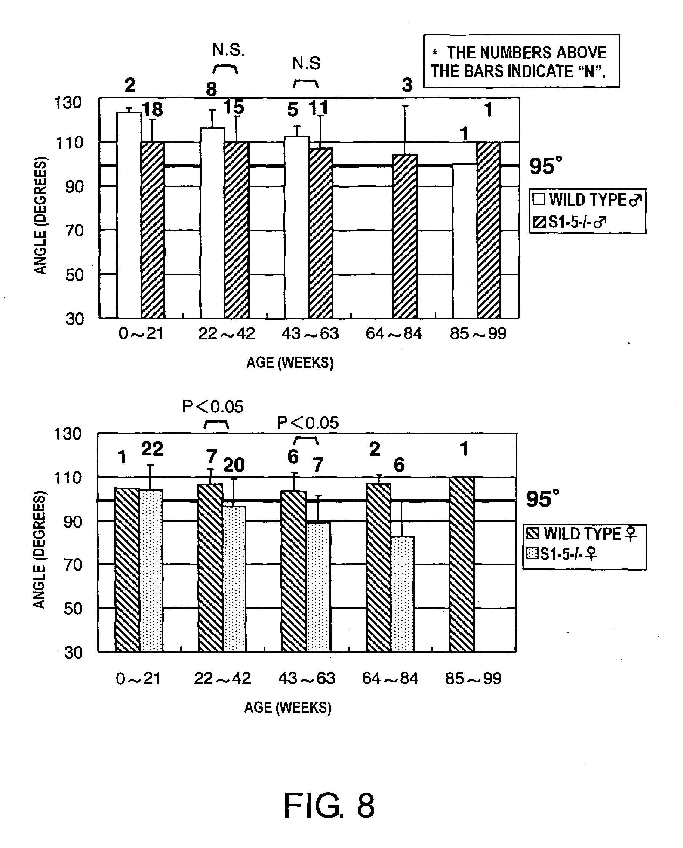

To numerically describe the spinal curvature of S1-5 knockout mice, the angle of curvature was measured using X-ray photographs. As a result, few wild type individuals had an angle of curvature of 95° or less, whereas S1-5 knockout mice with an angle of curvature of 95° or less were frequently found. Therefore, onset of kyphosis was defined as an angle of curvature of 95° or less, and the rate of onset was calculated every 21-weeks of age. Compared to males, females had a higher onset frequency, with earlier onset from about 22-weeks of age, and increasing severity with age (Figs. 7 and 8).

### [Example 5]

### Measurement of bone mineral density and micro CT analysis of S1-5 knockout mice

Since abnormalities were found in the bone tissues of S1-5 knockout mice, more detailed analyses were carried out. The spine (thoracic vertebrae (10-12) lumbar vertebrae (1-3)) and femora were removed from wild type mice and S1-5 knockout mice, and fixed in 70% ethanol. Bone mineral density was then measured. Bone mineral density was calculated by averaging the values obtained by using DCS-600EX-IIIR (Aloka) to scan 1-mm thick slices at a speed of 10 mm/s.

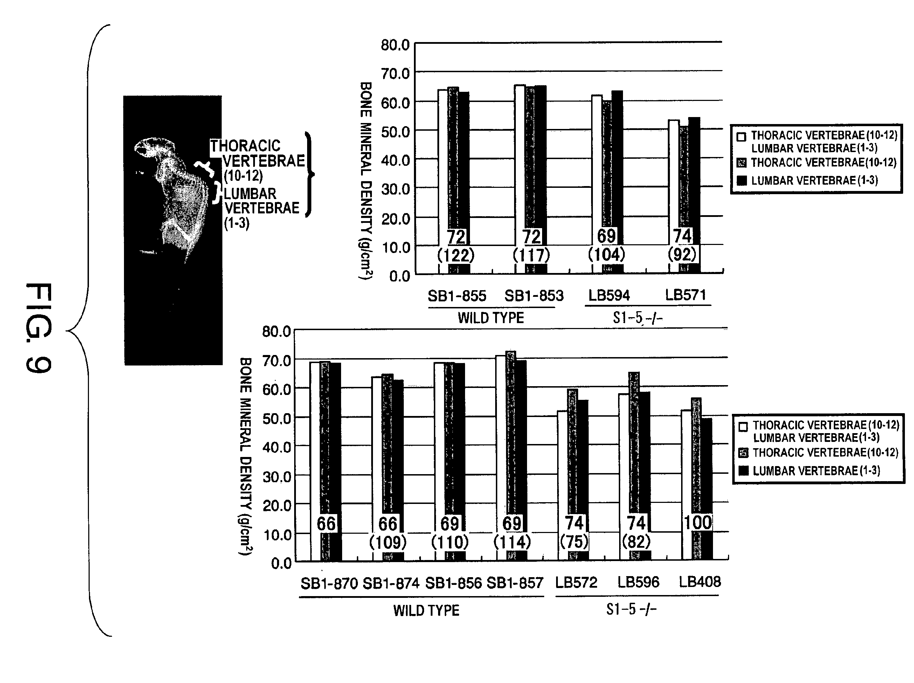

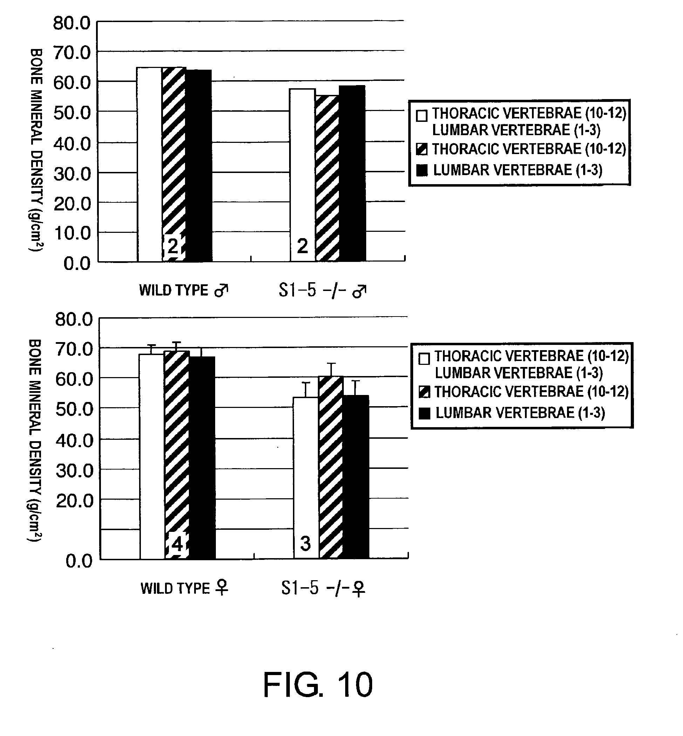

As a result, bone mineral density in the spine (thoracic vertebrae (10-12) lumbar vertebrae (1-3)) was found to be reduced in both male and female S1-5 knockout mice. Bone mineral density was measured at specific sites, with results showing a particular reduction at the thoracic vertebrae (10-12) of male S1-5 knockout mice, and the lumbar vertebrae (1-3) of female S1-5 knockout mice (Figs. 9 and 10).

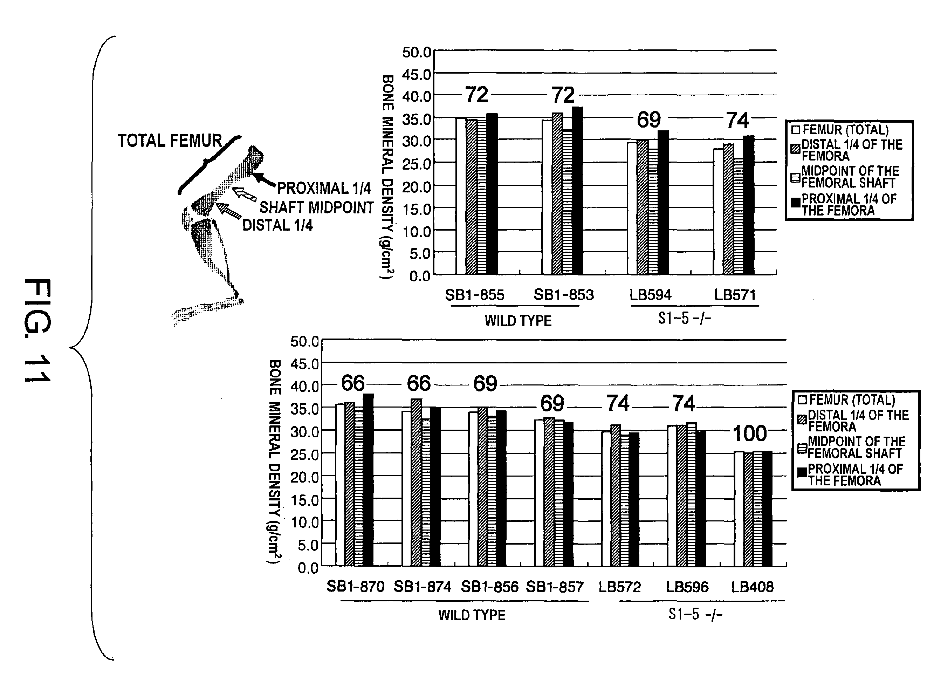

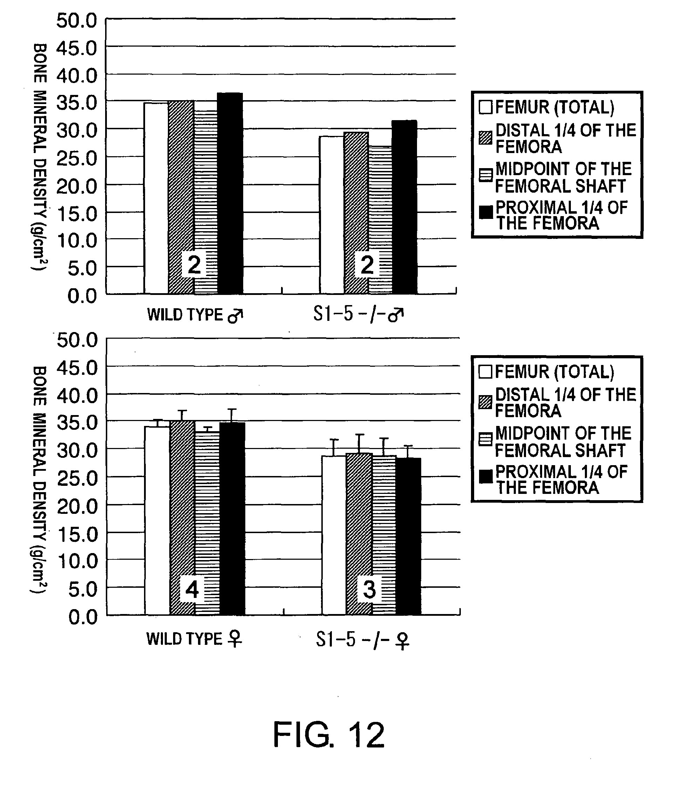

Bone mineral density was observed to be reduced in the femora of both male and female S1-5 knockout mice. Bone mineral density was also measured at specific sites, with results showing a particular reduction at the midpoint of the femoral diaphysis for male S1-5 knockout mice, and a reduction over all parts of the femur for female S1-5 knockout mice (Figs. 11 and 12).

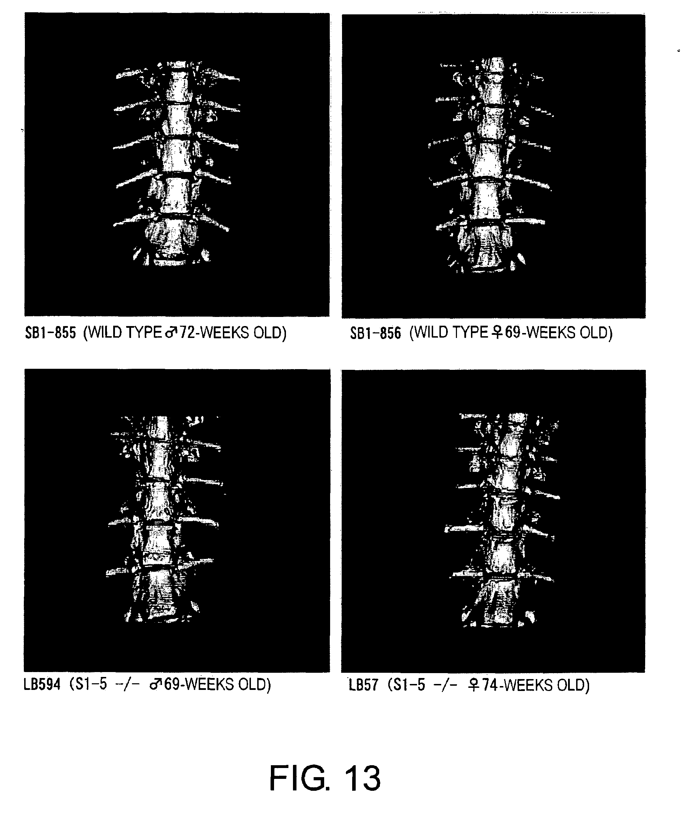

Micro CT images of S1-5 knockout mice and wildtype mice were taken, and 3D micro-CT images of the thoracic vertebrae were constructed (Fig. 13), showing that the surface of the centra was smooth in wild type mice, but noticeably uneven in the knockout mice.

### [Example 6]

### Analysis of S1-5 knockout mice by pQCT

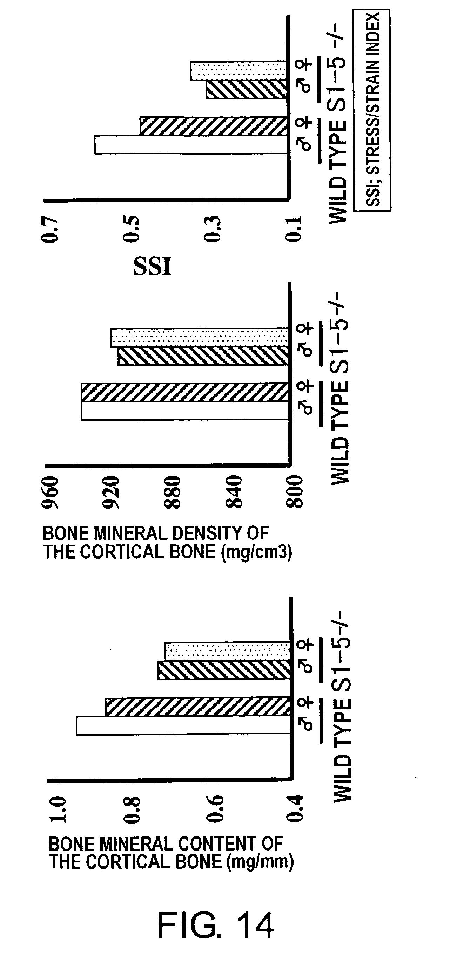

More detailed analysis was also carried out using peripheral quantitative computed tomography (pQCT) measurements. The results indicated decreased bone mineral content and bone mineral density in S1-5 knockout mice (Fig. 14). This phenomenon was particularly noticeable in female individuals. The bone strength was revealed to have decreased as shown by the noticeably reduced stress/strain index (SSI) in S1-5 knockout mice as compared to wild type mice.

### [Example 7]

### Analysis of urine NTx in S1-5 knockout mice

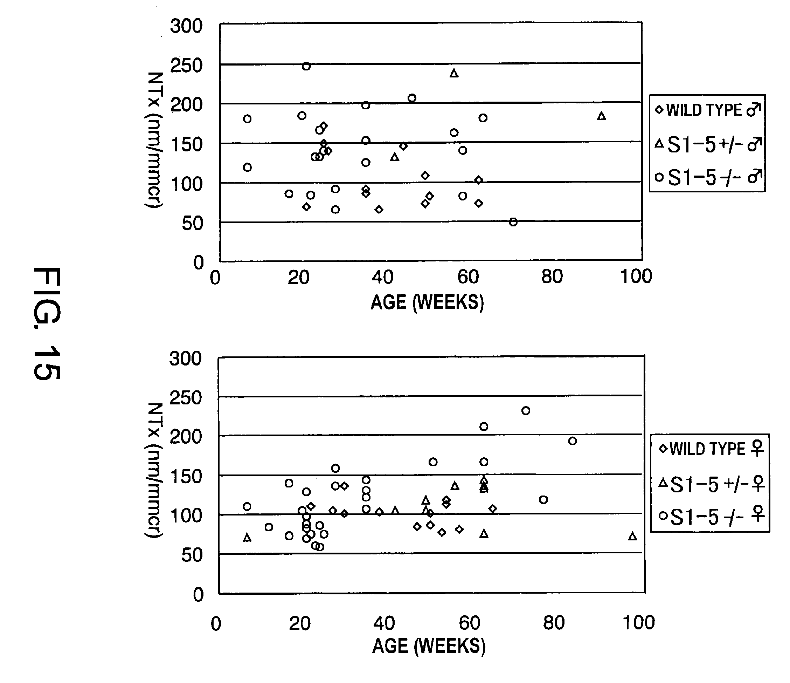

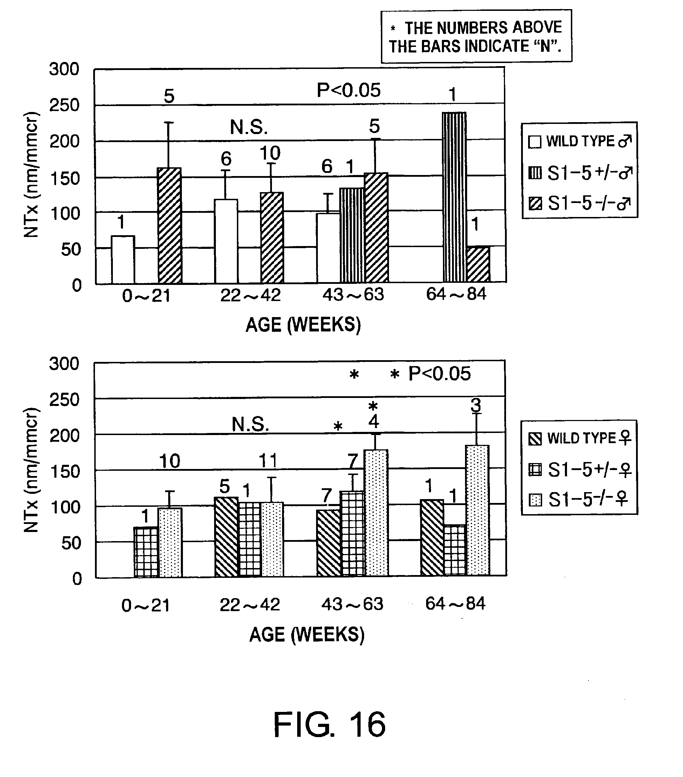

Type I collagen cross-linked N-telopeptide (NTx) in urine or serum has a negative correlation with bone mineral density, and is known to reflect enhanced bone resorption (Taguchi Y et al., Calcif. Tissue Int. 62; 395-399, 1998). Therefore, urine NTx was measured to investigate whether the reduced bone mineral density in S1-5 knockout mice was due to enhanced bone resorption. The results showed that urine NTx values were high in female S1-5 knockout mice aged 43-weeks or more (Figs. 15 and 16), suggesting that bone resorption is enhanced in S1-5 knockout mice.

### [Example 8]

### Histological analysis of the femora of S1-5 knockout mice

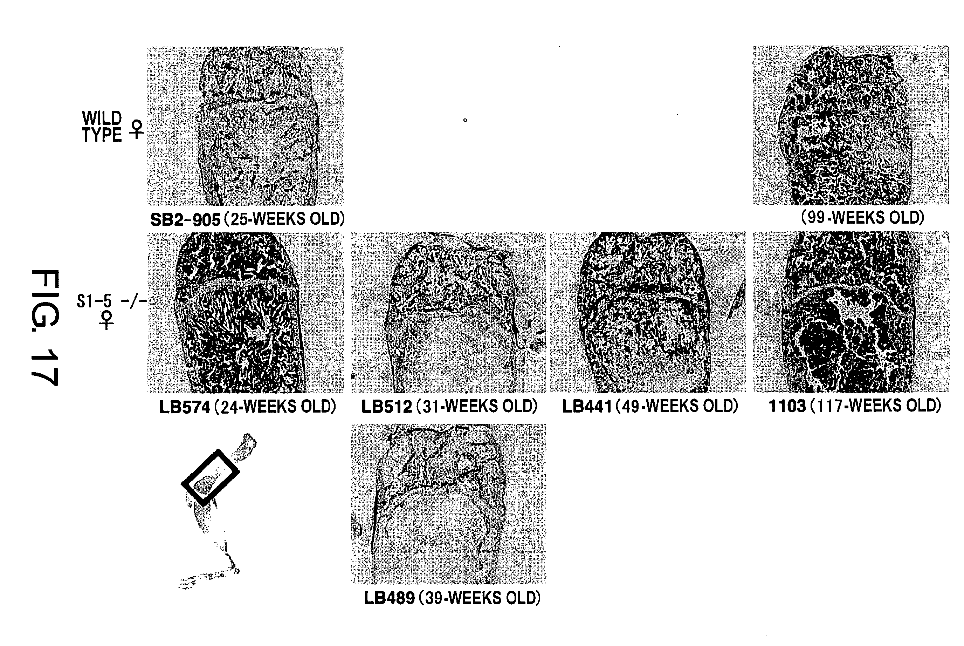

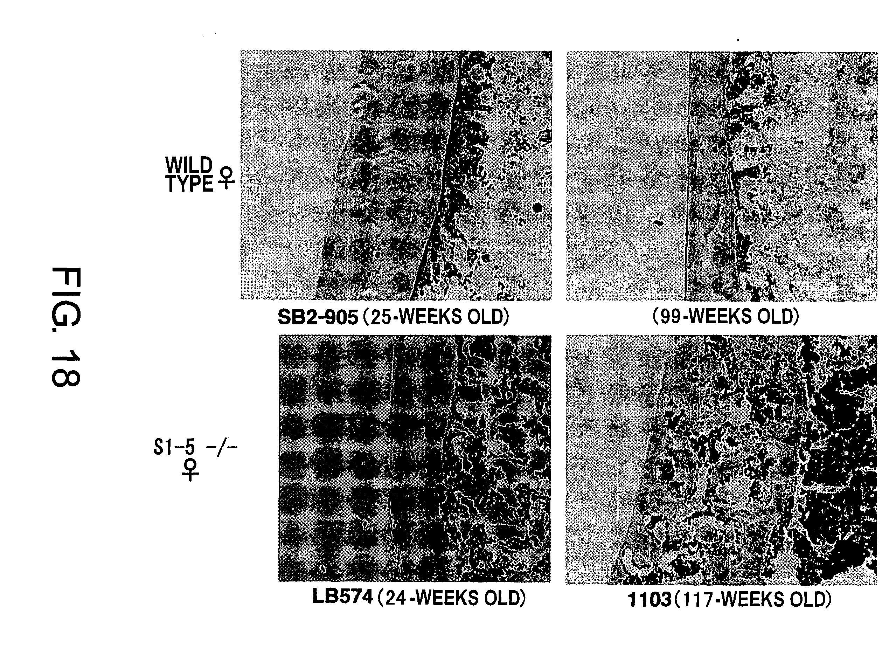

The femora of S1-5 knockout mice and wild type mice were removed and fixed in 70% ethanol after removing the soft tissue from around the bone. Tissue samples and stained hard tissue samples were prepared, and the bone tissue was examined. As a result, cancellous transformation of cortical bone was observed in the S1-5 knockout mice (Figs. 17 and 18). Hyperparathyroidism is generally known to increase blood PTH level and result in high bone-turnover, causing cancellous transformation of cortical bone. Cancellous transformation of cortical bone is also observed in Paget's disease of bone, which is characterized by increased bone-turnover and disorderly remodeling.

### [Example 9]

### Analysis of S 1-5 knockout mice by TRAP staining

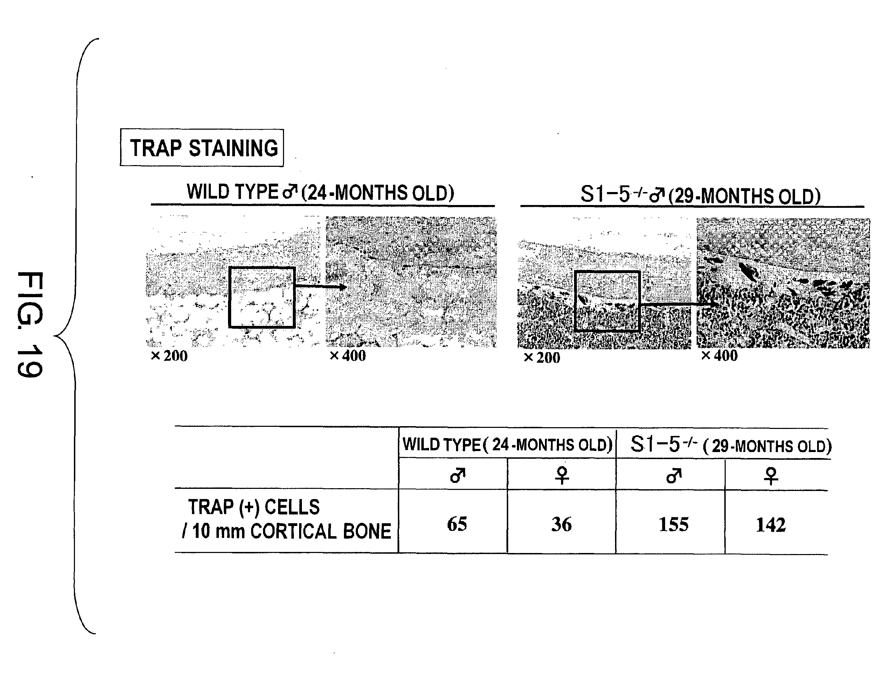

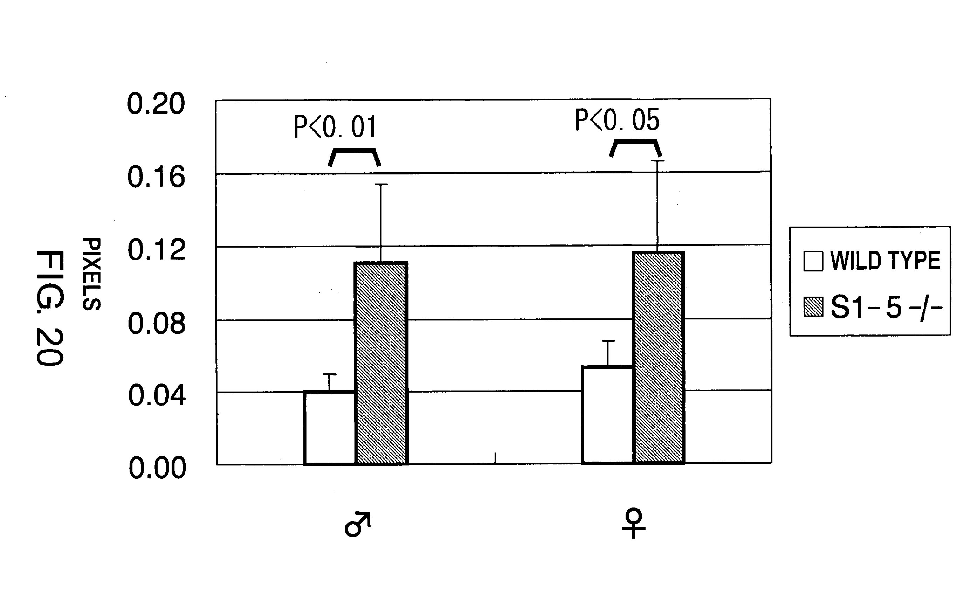

Bone tissue samples were TRAP stained to confirm the number of osteoclasts in the tissue. Images of each well were taken at equivalent magnification and captured into a computer, then Image J (distributed by NIH) was used to draw a line around parts corresponding to osteoclasts, and the area was calculated in pixels. The results showed that the number of osteoclasts (Fig. 19) and their size (Fig. 20) was increased in the S 1-5 knockout mice.

### [Example 10]

### Analysis of the osteoclast-forming ability of bone marrow cells derived from S1-5 knockout mice

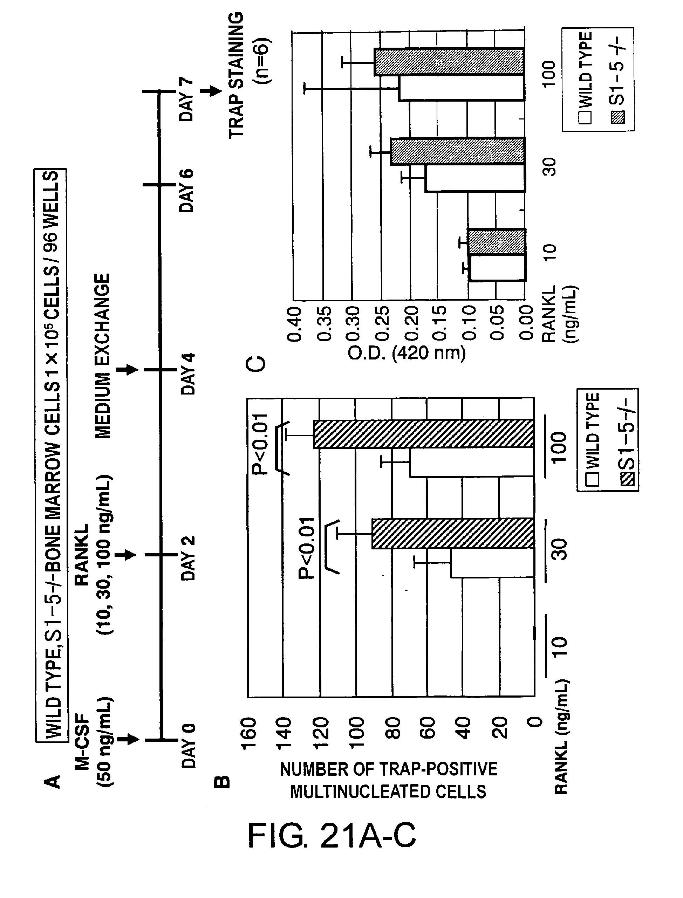

Using bone marrow cells derived from S1-5 knockout mice, the ability to form osteoclasts *in vitro* was analyzed. 1x 10⁵ bone marrow cells were plated onto a 96-well plate, and then macrophage colony stimulating factor (M-CSF) was added to a final concentration of 50 ng/mL. Two days later Receptor Activator of NF-κB Ligand (RANKL) was added to a final concentration of 10, 30, and 100 ng/mL. The cells were fixed seven days later, and then TRAP stained to examine their osteoclast-forming ability (Fig. 21A). The experiments were carried out at n=6. The results confirmed that in the presence of RANKL at final concentrations of 30 and 100 ng/mL, the number of osteoclasts formed was significantly increased (Fig. 21B). According to TRAP solution assays, TRAP activity level was increased in the S1-5 knockout mice (Fig. 21 C). TRAP staining when RANKL was added at a final concentration of 100 ng/mL showed that the size of TRAP-positive multinucleated giant cells in S1-5 knockout mice increased compared to that of the wild type (Figs. 21D and E). These results suggested that S1-5 is involved in the inhibition of osteoclast differentiation.

### [Example 11]

### Hemostatic analysis of S1-5 knockout animals

An incision was made on the tails of S1-5 knockout mice aged eight to 111 weeks (26 months), and to examine the degree of hemostasis, filter paper was used every ten seconds to absorb the blood flowing out. The results showed that hemostasis tends to be difficult to achieve in S1-5 knockout mice (Fig. 22).

### [Example 12]

### Analysis of hematocrit values in S1-5 knockout animals

One end of a capillary tube for hematocrit measurements (Capillary tubes for microhematocrits, 75 mm length, heparinized, Drummond Scientific Co.) was placed into the blood and maintained at an appropriate angle to draw the blood by capillary action up two-thirds of the full length of the capillary tube. The end from which blood was drawn was sealed by insertion of putty. The capillary, sealed at one end, was centrifuged for five minutes at 11,000 rpm, and the percentage (%) was read using a measuring plate. The results showed that S1-5 knockout mice tended to have low hematocrit values, and were in an anemic condition (Figs. 23 and 24).

### [Example 13]

### Giemsa staining of the peripheral blood of S1-5 knockout mice

Blood was collected from the tail vein of 15 to 110-week old S1-5 knockout mice. This blood was then diluted 1000 times in PBS, cytospun (800 rpm, five minutes), and then peripheral blood was stained using Giemsa. The results showed anemia in the S1-5 knockout mice: specifically, a decreased number of erythrocytes, abnormal erythrocytes (circular erythrocytes), and differences in staining were observed (Fig. 25).

### [Example 14]

### Production of CHO-K1 cells stably expressing the S1-5-His protein

CHO-K1 cells stably expressing the S1-5-His protein were prepared in order to carry out large-scale purification of the S1-5-His protein. Using Lipofectamine 2000, 20 µg of pCAGGS-S1-5-His and 0.7 µg of pcDNA3 were transfected simultaneously into CHO-K1 cells (one 10 cm dish), and 24 hours later the cells were diluted ten times and plated onto six 10-cm dishes. 24 hours later, selection was initiated by using 800 µg/mL Geneticin, and colonies were picked 11 days later. 16 colonies were picked from each 10-cm dish (a total of 96 clones) and these colonies were plated into a 96-well plate, then scaled up into 24-well plates, and then to 6-well plates. At the same time, 6 types of bulk samples were individually plated onto 10-cm dishes, and when they reached confluency, the media were exchanged for serum-free media. 24 hours later, Western blotting was used to check S1-5-His expression in the cell extract and in the culture supernatant. Antibodies were produced by immunizing rabbits using the S1-5 peptide (42-56: 15-amino acid CKDIDECDIVPDACK: the underlined portion is the predicted T cell epitope) as the immunogen, and then those confirmed by Western blotting to recognize the S1-5 protein were used. The expression of S1-5-His protein was similarly checked using those cloned cells that reached confluency on 6-well plates. 1 mL of serum-free medium was used for each well of a 6-well plate, and half of the TCA precipitates were used to check expression. The results confirmed S1-5-His protein expression in the cell extract and culture supernatant of all six types of bulk sample, with strong expression observed in bulk sample Nos. 1, 2 and 3 in particular. Furthermore, S1-5 protein endogenous to CHO-K1 cells was confirmed to be secreted into the culture supernatant (Fig. 26). Meanwhile, of the 96 clones selected, 90 clones in which growth had been confirmed were checked for expression of the S1-5-His protein in the culture supernatant (Fig. 27). Of these, Clone Nos. 1, 6, 10, 11, 13, 25, 38, 43, 44, 61, and 96 were again checked for S1-5-His protein expression (Fig. 28). As a result, S1-5-His protein expression was found to be stronger in Clone Nos. 1, 6, 44 and 96 than in bulk sample 1. Ultimately, Clone No. 96 was chosen for subsequent experiments.

### [Example 15]

### Purification of the S1-5-His protein from the cell culture supernatant of CHO-K1 stably expressing the same

60 mL of the cell culture supernatant of CHO-K1 stably expressing the S1-5-His protein was passed through a filter, and then concentrated to approximately 10 mL using Centriplus. The concentrated sample was then dialyzed against a purification buffer (20 mM Tris-HCl (pH 7.5), 500 mM NaCl, 10% glycerol). The dialyzed sample was loaded onto a 1-mL HisTrap column, and the column was washed with 10 mL of purification buffer. The adsorbed protein was then eluted using the purification buffer supplemented with 10 mM and 250 mM imidazole (1 mL/fraction). The proteins contained in the fraction eluted with the 250 mM imidazole solution were separated by 10% SDS-PAGE, and then detected using silver staining (Fig. 29A) or Western blotting using anti-S1-5 antibody (Fig. 29B). As a result, one band was detected when using the anti-S1-5 antibody.

280 mL of the culture supernatant of CHO-K1 cell stably expressing the S1-5-His protein was passed through a filter (0.22 µm) and then loaded onto a 1-mL HisTrap column. The column was washed with 10 mL of purification buffer (20 mM Tris-HCl(pH7.5), 500 mM NaCl, 10% glycerol) and then with the purification buffer supplemented with 10 mM imidazole. The adsorbed protein was then eluted with the purification buffer supplemented with 50 mM and 100 mM of imidazole (1 mL/fraction). The eluted fractions were collected (approximately 4 mL), and dialyzed overnight against 1 L of PBS, and the sample was then passed through a filter (0.22 µm). The proteins included in the fractions eluted with 50 mM and 100 mM imidazole were separated by 10% SDS-PAGE, and then detected using silver staining (Fig. 30). The results showed that fractions eluted with the purification buffer containing 100 mM imidazole had lost unnecessary bands compared to the fractions eluted with the purification buffer containing 50 mM imidazole, indicating that a more purified S1-5 protein was obtained in the former case.

### [Example 16]

### Preparation of S1-5-His truncated proteins from CHO-K1 cells

The S1-5 sequence comprises six EGF-like domains. To investigate the function of S1-5, the EGF-like domains were deleted one at a time from the C-terminal end, producing six constructs (Fig. 31).

S1-5-E1-His/pME18S, S1-5-E1-2-His/pME18S, S1-5-E1-3-His/pME18S, S1-5-E1-4-His/pME18S, S1-5-E1-5-His/pME18S, S1-5-E1-6-His/pME18S and S1-5 full-His/pME18S were transfected into CHO-K1 cells using FuGENE6 (Roche). Eight hours after transfection the media were exchanged for serum-free media, and the cells were then cultured for 24 hours. Culture supernatants were collected, Ni-agarose was added to them, and they were mixed for eight hours at 4°C. The Ni-agarose was washed three times with a washing buffer (20 mM Tris (pH7.5), 500 mM NaCl, 10% Glycerol, and 10 mM Imidazole), and then the S1-5 proteins were eluted using an elution buffer (20 mM Tris (pH7.5), 500 mM NaCl, 10% Glycerol, 250 mM imidazole). The eluted fractions were dialyzed to exchange the buffer for PBS, and then the concentration of S1-5 proteins were determined by Western blotting (Fig. 32A).

S1-5-FLAG truncated proteins and the S1-5 FLAG protein were similarly produced (Fig. 32B), and their respective expression vectors were transfected into CHO-K1 cells (6-well plate). Four hours later, the media were exchanged for serum-free media (1 mL), and after incubating for 24 hours, the culture supernatants (900 µL) were subjected to TCA precipitation. Thereafter, the TCA precipitates and cell extract solutions were used to perform Western blotting using anti-FLAG antibody. The results showed that the expression levels of all truncated proteins were higher than that of the full length S1-5 protein, and that the expression level of S1-5(E1-3)-FLAG protein was particularly high.

### [Example 17]

### Preparation of FLAG-S1-5 truncated proteins from HEK293 cells

The FLAG-S1-5 truncated proteins expressed in HEK293 cells (Dainippon Pharmaceutical) were purified. HEK293 cells were plated to 80% to 90% confluency in a 150-mm dish (IWAKI). The following day, transfection was carried out using FuGENE6. The procedure was carried out as per the attached manual. The cells were collected two days later, and 300 µL of lysis buffer (50 mM Tris-HCl (pH 7.4), 420 mM NaCl, 1 mM EDTA, 1 mM MgCl₂, 0.5 mM DTT, and 1 mM PMSF) was added. This was incubated on ice for 20 minutes. 100 µL of anti-FLAG antibody (SIGMA) bound to agarose beads was added and incubated overnight while rotating at 4°C. The beads were washed five times with washing buffer (50 mM Tris-HCl (pH7.4), 150 mM NaCl, 0.5 mM DTT, and 1 mM PMSF), then 100 µL of 100 µg/mL FLAG peptide (SIGMA) was added, and this was incubated for two hours while rotating at 4°C. The supernatants were collected by centrifugation, and 10 µL of each were subjected to SDS-PAGE. The FLAG-S1-5 truncated proteins purified from the cells were identified by Western blotting (Fig. 33A), and their concentrations were determined using CBB staining.

The culture supernatants were collected 48 hours after transfection, at which point 100 µL of anti-FLAG antibody bound to agarose beads was added. These were then incubated overnight while rotating at 4°C. Thereafter, the same procedure as described above was carried out, and the FLAG-S1-5 proteins purified from the culture supernatants were identified and their concentrations were determined (Fig. 33B). The results showed that S1-5 proteins were purified both from inside the cells and from the culture supernatants (Fig. 33AB).

### [Example 18]

### Purification of S1-5-FLAG protein from 293F non-adherent cells, and purification of S1-5-FLAG protein subjected to prescission protease treatment

S1-5-FLAG protein, forcibly expressed in 293F non-adherent cells, was purified. First, the S1-5-FLAG protein expressed in the cytoplasm of 293F cells was purified by the following steps: 293F cells transfected with pcDNA3-S1-5-prescission-FLAG plasmid was suspension-cultured at a volume of 90 mL, and cells were collected 48 hours later. 1 mL of lysis buffer (50 mM Tris-HCl (pH 7.4), 420 mM NaCl, 1 mM EDTA, 1 mM MgCl₂, 0.5 mM DTT, 1 mM PMSF, 0.5 % NP-40) was added, and this was incubated on ice for 20 minutes. At this point, 100 µL of anti-FLAG antibody (SIGMA) bound to agarose beads was added, and this was incubated overnight while rotating at 4°C. The beads were washed five times with a washing buffer (50 mM Tris-HCl (pH 7.4), 150 mM NaCl, 0.5 mM DTT, 1 mM PMSF, 0.5% NP-40), then transferred to a mini-column and eluted three times with 100 µL of 200 µg/mL FLAG peptide (SIGMA). The eluate was dialyzed overnight against a prescission buffer (50mM Tris-HCl (pH7.0), 150 mM NaCl, 1 mM EDTA, 1 mM DTT). 20 U of prescission protease was added, and the reaction was allowed to take place for five hours at 4°C. 50 µL of glutathione sepharose beads were added, and the mixture was incubated for one hour while rotating at 4°C. The supernatant was collected, and this was subjected to SDS-PAGE along with samples obtained at each stage of purification, and the proteins were identified by Western blotting. Next, the S1-5-FLAG protein obtained from the culture supernatant of transfected 293F cells was purified. The culture supernatant was collected 48 hours after transfection, 100 µL of anti-FLAG antibody bound to agarose beads was added, and this was incubated overnight while rotating at 4°C. Thereafter, purification was carried out by the same procedure as described above, followed by SDS-PAGE and Western blotting. The results are shown in Fig. 34. The S1-5-FLAG protein in the cytoplasm was purified using anti-FLAG antibody bound to agarose beads. In contrast to the case when using anti-S1-5 antibody, hardly any S1-5 could be detected in the final eluate when using anti-FLAG antibody, suggesting that prescission protease digestion had taken place. Also, Western blotting did not detected a band from the culture supernatant at any stage of purification. These results indicated that this method can purify S1-5 protein without a FLAG tag from the cytoplasm.

### [Example 19]

### Examination of glycosylation of the S1-5 protein

Whether the S1-5 protein is in fact glycosylated was examined. Glycosylpeptidase F treatment (37°C, 17 hours) was performed on S1-5-His protein purified from the culture supernatant of CHO-K1 cells stably expressing the S1-5-His protein, cell extract of CHO-K1 cells, and S1-5 protein immunoprecipitated from the culture supernatant of HT1080 cells, and then Western blotting using anti-S1-5 antibody was performed (Fig. 35). As a result, glycosylpeptidase F treatment was found to cause a band shift of the pure S1-5-His protein (20 ng) (Fig. 35, left figure). Similarly, band shift of the S1-5 protein was also observed in the S1-5 protein immunoprecipitated from the culture supernatant of HT1080 cells (Fig. 35, right figure). These results suggested that S1-5 undergoes N-linked glycosylation. In fact, the S1-5 proteins share a common sequence (NX(S/T)X≠P) at one site. When a broad band that was difficult to detect at a low concentration was examined by increasing the amount of protein (120 ng), an overall band shift was observed; however, the broad band did not disappear, suggesting that the S1-5 protein may also have undergone O-linked glycosylation (Fig. 35, center figure).

### [Example 20]

### Preparation of S1-5-His truncated proteins from E. coli

ApGEX vector that expresses the GST-fused S1-5-His protein was prepared, *E. coli* was transformed using this vector, and colonies were isolated. The isolated colonies were inoculated into 10 mL of LB-ampicillin medium (50 mg/mL ampicillin), and cultured overnight at 37°C (pre-culture). The pre-cultured *E. coli* was added to 200 mL of LB-ampicillin medium and cultured for 2.5 hours at 37°C (main culture). IPTG was then added at a final concentration of 0.5 mM, and the mixture was cultured for another three hours at 30°C. The bacterial cells were collected by centrifugation, then 5 mL of BC500 (20 mM Tris-HCl (pH 8.0), 0.5 mM EDTA, 500 mM KCl, 20% glycerol, 1% NP-40, 1 mM DTT, 0.5 mM PMSF, 1 µg/mL of aprotinin, pepstatin, and leupeptin) and 100 µL of 100 µg/mL lysozyme was added to produce a suspension. The bacteria were then homogenized by sonication. The supernatant was collected by centrifugation, 500 µL of glutathione S transferase-conjugated agarose beads (GSH) (Amersham Pharmacia) GSH resin was added, and the mixture was incubated overnight while rotating at 4°C. The GSH resin was washed five times with BC500, and then applied to a micro-column (Biorad). The column was capped after adding 500 µL of prescission protease solution (10U prescission protease, 50 mM Tris-HCl (pH7.0), 150 mM NaCl, 1 mM EDTA, 1 mM DTT), and was left to stand for 24 hours of incubation at 4°C. The column eluate was collected as a digested S1-5-His protein solution, identified using Western blotting (Fig. 36) and then CBB stained to determine concentration. The results showed that the S1-5 protein was purified from *E. coli* (Fig. 36).

### [Example 21]

### Purification of MBP-S1-5 protein and MBP-S1-5 protein subjected to prescission protease treatment from E. coli

Purification of the MBP-S1-5 protein was also carried out by an extraction method similar to that of Example 20 except that the pGEX vector that expresses the MBP-fused S1-5 protein was used. The results showed that the most efficient elution was achieved when using 10 mM maltose. MBP-S1-5 was obtained more efficiently than GST-S1-5 protein at 450 µg/500 mL culture (Fig. 37). In addition, prescission protease treatment in solution was confirmed to cleave MBP, just as in the prescission protease treatment of GST-S1-5 (Fig. 38).

### [Example 22]

### Effect of S1-5-His protein derived from CHO-K1 cell culture supernatant on osteoclast-forming ability in bone marrow cells derived from S1-5 knockout mice

The effect of purified S1-5-His protein on *in vitro* osteoclast-forming ability was examined using bone marrow cells derived from S1-5 knockout mice and the protein purified in Example 15. 1x10⁵ bone marrow cells were plated onto a 96-well plate, M-CSF was added at a final concentration of 50 ng/mL, and two days later RANKL was added to a final concentration of 100 ng/mL. S1-5 was diluted in PBS, and was added continuously to the culture medium from the time of M-CSF addition, such that the final concentrations were 0, 10, 30, and 100 ng/mL. Five days later the cells were fixed and the effect of S1-5-His protein on osteoclast-forming ability was examined using TRAP staining. The experiments were carried out at n=5. The results confirmed that the number of TRAP-positive multinucleated giant cells was significantly suppressed depending on S1-5-His protein concentration (Fig. 39 and 41). Suppression of the number of TRAP-positive multinucleated giant cells formed was also dependent on the concentration of S1-5-His protein (Figs. 40 and 41). The experiments were carried out as described above, except that S1-5-His protein was added at a final concentration of 0, 10, 30, 100, 200, and 500 ng/mL. The area occupied by TRAP-positive multinucleated giant cells was measured, indicating a reduction in the size of TRAP-positive multinucleated giant cells dependent on the concentration of S1-5-His protein (Figs. 42A, 42B and 42C).

### [Example 23]

### Effect of S1-5-His truncated proteins on osteoclast-forming ability in bone marrow cells derived from S1-5 knockout mice

The effect of purified S1-5-E1-2 protein on *in vitro* osteoclast-forming ability was examined using bone marrow cells derived from S1-5 knockout mice and the protein purified in Example 16. The method was as for Example 22, except that S1-5-E1-2 protein was added at a final concentration of 0 and 10 ng/mL. The results confirmed that the S1-5 truncated protein suppressed the number of TRAP-positive multinucleated cells (Figs. 43 and 45). The number of TRAP-positive multinucleated giant cells formed was even more noticeably suppressed (Figs. 44 and 45). The amino acid sequence of S1-5-E1-2 is shown in SEQ ID NO: 6.

### [Example 24]

### Effect of S1-5-His protein derived from the culture supernatant of CHO-K1 cells on osteoclast-forming ability in RAW264.7 cells

Using RAW264.7 cells (mouse macrophage-derived cells) and the protein purified in Example 15, the effect of purified S1-5-His protein derived from the culture supernatant of CHO-K1 cells on *in vitro* osteoclast-forming ability was examined. 2.5x 10⁵ RAW264.7 cells were plated onto a 96-well plate, and RANKL was added to a final concentration of 100 ng/mL. The S1-5-His protein was diluted in PBS, and was continuously added to the culture medium to final concentrations of 0, 10, 30, 100, 200, and 500 ng/mL. Three days later, the cells were fixed, and the effect of the S1-5-His protein on osteoclast-forming ability was examined using TRAP staining. The experiments were carried out at n=5. The results confirmed S1-5-His protein concentration-dependent suppression of the number of TRAP-positive multinucleated cells (Figs. 46 and 47).

### [Example 25]

### Effect of S1-5-FLAG protein derived from 293F non-adherent cells on osteoclast-forming ability in RAW264.7 cells

The effect of purified S1-5-FLAG protein on osteoclast-forming ability was examined using RAW264.7 cells and the protein purified in Example 18. The method was as in Example 24, except that the S1-5-FLAG protein derived from 293F non-adherent cells, and the S1-5 protein produced by cell-free expression (Abnova, Cat. No. H00002202-P01) were added to final concentrations of 0, 10, 30, 100, and 200 ng/mL. The results indicated that when the S1-5-FLAG protein purified in Example 18 was added, osteoclast-forming ability was suppressed dependent on the concentration of S1-5-FLAG protein (Fig. 48). However, this suppressive effect was not observed when the S1-5 protein obtained by cell-free expression was added (Fig. 49).

### [Example 26]

### Examination of the effect of the GST-S1-5 protein derived from E. coli, and GST-S1-5 protein subjected to prescission protease treatment, on osteoclast-forming ability in RAW264.7 cells

The effects of purified GST-S1-5 protein and prescission protease-treated GST-S1-5 protein on osteoclast-forming ability were examined using RAW264.7 cells and the protein purified in Example 20. The method was as for Example 24, except that *E. coli*-derived GST-S1-5 protein and GST protein were added to final concentrations of 0, 1, 3, 10, 30, 100, and 200 ng/mL, and prescission protease-treated GST-S1-5 protein and prescission protease-treated GST protein were added to final concentrations of 0, 1, 3, 10, and 30 ng/mL. As a result, when GST-S1-5 protein and prescission protease-treated GST-S1-5 protein were added, the suppressive effect was found to be concentration-dependent (Figs. 50, 51, 54, and 55); however, GST protein and prescission protease-treated GST protein did not affect osteoclast-forming ability (Figs. 52, 53, 56, and 57). The S1-5 protein whose GST tag was removed by prescission protease treatment not only maintained but enhanced its activity as a result of tag removal.

### [Example 27]

### Examination of the effect of S1-5 protein on the lowering of bone mineral density in osteoporotic model rats

Following standard procedures, 7-week-old Wister rats were ovariectomized (OVX), and then the protein purified in Example 20 was administered subcutaneously. The protein was administered five times a week at a dose of 1.0 mg/kg per day following ovariectomy. The rats were sacrificed four weeks after the operation, their left and right femora were removed and fixed in 70% ethanol, and then bone mineral density was measured. Bone mineral density was measured on a DCS-600EX-IIIR Model (Aloka) by taking 1-mm thick slices of the distal quarter of the femur, and taking 20 scans at a speed of 10 mm/s from the distal position to the proximal position. Compared to sham rats, OVX rats given GST and prescission protease showed an approximately 10% reduction in bone mineral density. An OVX model is considered to have been made if its bone mineral density is reduced 10% or more (Modder, UI. et al., Endcrinology 145: 913-921, 2004). In contrast, OVX rats given prescission protease-treated GST-S1-5 protein showed an increase in bone mineral density. These results suggested that S1-5 protein improved osteoporosis (Fig. 58).

### [Example 28]

### Examination of the effect of S1-5 protein on the decrease of bone mineral density in osteoporosis model rats

Analysis of bone strength was carried out on the femora of the rats examined in Example 27. Bone strength was measured using a three-point bending test carried out on a MZ-500S (Maruto) with a 500 N load cell, support span of 13 mm, and head speed of 10 mm/min.

The results showed that OVX rats given GST protein and prescission protease had reduced bone strength compared to sham rats, but that bone strength improved when the protein purified in Example 20 was administered (Fig. 59).

### [Example 29]

### Analysis of the effect of S1-5 protein on osteoporotic model rats using micro CT

Femora were removed from the rats examined in Example 27 and fixed in 70% ethanol after removing the soft tissues from around the bone. Micro CT images were then taken at a position 10% down the diaphysis from the growth plate cartilage. Compared to sham rats, OVX rats given GST protein and prescission protease showed a decrease of cancellous bone; however, in OVX rats given the protein purified in Example 20, the decrease of cancellous bone was improved (Fig. 60). These images suggested that S1-5 protein demonstrates the effect of suppressing osteoclasts.

### [Example 30]

### Histological analysis of the effect of S1-5 protein on osteoporotic model rats

Tibias were removed from the rats examined in Example 27 and fixed in 70% ethanol after removing the soft tissues from around the bone. Thereafter, bone tissue samples were TRAP stained and the number of osteoclasts in the tissues was measured. The results showed that, compared to sham rats, OVX rats given GST protein and prescission protease showed an increase in the number of osteoclasts in the proximal metaphysis of the tibia, but in OVX rats given the protein purified in Example 20, this increase in the number of osteoclasts was suppressed (Fig. 61).

### [Example 31]

### Examination of the effect of S1-5 protein on osteoporotic model mice

Following standard procedures, 7-week old C57BL/6j mice were ovariectomized, and the protein purified in Example 20 was administered subcutaneously. The protein was administered five times a week at a dose of 1.0 mg/kg/day following ovariectomy. The mice were sacrificed eight weeks after the operation, their femora were removed and fixed in 70% ethanol, and then their bone densities were measured. The bone mineral density was measured using a DCS-600EX-IIIR Model (Aloka) by taking 1-mm thick slices of the distal one quarter of the femur, and taking 20 scans at a speed of 10 mm/s from the distal position to the proximal position. As was the case for the rats, the results showed that bone mineral density in OVX mice was approximately 10% less than in sham mice. In contrast, OVX mice given the protein purified in Example 20 showed an increase in bone mineral density. These results suggested that S1-5 protein improves osteoporosis (Fig. 62).

### Industrial Applicability

The present invention provides knockout animals in which the S1-5 gene has been made defective, where these animals develop age-related diseases, as well as methods for producing such animals. Since the animals of the present invention develop various age-related diseases and symptoms, such as bone deformation, hair loss, tissue injury and tumor development, they may be used as model animals to screen for pharmaceutical compositions with therapeutic or remedial effects by administering substances effective for the treatment or prevention of age-related diseases to these animals. The present invention also provides cells isolated from the knockout animals. The use of these cells enables screening for pharmaceutical agents for the treatment or prevention of age-related diseases. Since the bone marrow cells isolated from the knockout animals have a greater ability to form tartrate-resistant acid phosphatase (TRAP)-positive multinucleated giant cells when differentiated into osteoclasts than the bone marrow cells of wild type mice, one can screen for agents that inhibit osteoclast function using the number and/or size of TRAP-positive multinucleated giant cells as an indicator.

The present invention also provides isolated S1-5 protein and antibodies that bind to the S1-5 protein. The use of the isolated S1-5 protein enables treatment or prevention of age-related diseases and inhibition of osteoclast function.

## Claims

1. A non-human knockout animal showing an age-related disease or symptom, wherein all or a part of the S1-5 gene function is lost.

2. The animal of claim 1, wherein the loss of all or a part of the S1-5 gene function is due to a disruption or mutation of the S 1-5 gene.

3. The animal of claim 1, wherein the age-related disease or symptom is at least one selected from the group consisting of bone deformation, osteoporosis, Paget's disease of bone, hyperparathyroidism, decreased bone mineral density, cancellous transformation of cortical bone, hair loss, tissue injury or necrosis, tumor, breast hypertrophy, ascites, anemia, bleeding, aging of skin, and aging of nails.

4. The animal of claim 1, wherein the animal is selected from the group consisting of zebrafish, mice, rats, guinea pigs, rabbits, chickens, pigs, sheep, goats, dogs, cattle, monkeys, and chimpanzees.

5. A cell isolated from the non-human knockout animal of any one of claims 1 to 4.

6. The cell of claim 5, which is an osteoclast, keratinocyte epithelial cell, blood cell, cancer cell, bone marrow cell, fibroblast, vascular endothelial cell, dermal cell, muscle cell, nerve cell, lymphocyte, vascular smooth muscle cell, synoviocyte, hair papilla cell, hepatocyte, pigment cell, adipocyte, uterine endothelial cell, or alveolar epithelial cell.

7. A method for producing a non-human knockout animal that develops an age-related disease, wherein the method comprises causing the loss of all or a part of the S1-5 gene function.

8. The method of claim 7, wherein the loss of all or a part of the S1-5 gene function is caused by a disruption or mutation of the S1-5 gene.

9. The method of claim 7, wherein the age-related disease or symptom is at least one selected from the group consisting of bone deformation, osteoporosis, Paget's disease of bone, hyperparathyroidism, bone mineral density, cancellous transformation of cortical bone, hair loss, tissue injury or necrosis, tumor, breast hypertrophy, ascites, anemia, bleeding, aging of skin, and aging of nails.

10. The method of claim 7, wherein the animal is selected from the group consisting of zebrafish, mice, rats, guinea pigs, rabbits, chickens, pigs, sheep, goats, dogs, cattle, monkeys, and chimpanzees.

11. A method of screening for preventive or therapeutic agents for an age-related disease or symptom, wherein the method comprises administering a candidate substance for said preventive or therapeutic agent to the non-human knockout animal of any one of claims 1 to 4.

12. A method of screening for preventive or therapeutic agents for an age-related disease or symptom, wherein the method comprises contacting a candidate substance for said preventive or therapeutic agent with cells isolated from the non-human knockout animal of any one of claims 1 to 4.

13. The method of claim 12, wherein the cells are osteoclasts, keratinocyte epithelial cells, blood cells, cancer cells, bone marrow cells, fibroblasts, vascular endothelial cells, dermal cells, muscle cells, nerve cells, lymphocytes, vascular smooth muscle cells, synoviocytes, hair papilla cells, hepatocytes, pigment cells, adipocytes, uterine endothelial cells, or alveolar epithelial cells.

14. The method of any one of claims 11 to 13, wherein the age-related disease or symptom is at least one selected from the group consisting of bone deformation, osteoporosis, Paget's disease of bone, hyperparathyroidism, decreased bone mineral density, cancellous transformation of cortical bone, hair loss, tissue injury or necrosis, tumor, breast hypertrophy, ascites, anemia, bleeding, aging of skin, and aging of nails.

15. A method of screening for agents that inhibit osteoclast function, wherein the method comprises contacting a candidate substance for said agent that inhibits osteoclast function with osteoclasts derived from the non-human knockout animal of any one of claims 1 to 4.

16. An isolated protein, which is any one of (a) to (d):
(a) a protein comprising the amino acid sequence of SEQ ID NO: 2 or 4;
(b) a protein encoded by a DNA comprising a coding region of the nucleotide sequence of SEQ ID NO: 1 or 3;
(c) a protein comprising an amino acid sequence with one or more amino acid substitutions, deletions, insertions, and/or additions in the amino acid sequence of SEQ ID NO: 2 or 4, wherein the protein is functionally equivalent to a protein comprising the amino acid sequence of SEQ ID NO: 2 or 4; and
(d) a protein encoded by a DNA that hybridizes under stringent conditions with a DNA comprising the nucleotide sequence of SEQ ID NO: 1 or 3, wherein the protein is functionally equivalent to a protein comprising the amino acid sequence of SEQ ID NO: 2 or 4.

17. A partial peptide of the protein of claim 16.

18. The peptide of claim 17, which comprises the amino acid sequence of SEQ ID NO: 6.

19. A preventive or therapeutic agent for an age-related disease or symptom, wherein the agent comprises the protein of claim 16, or the peptide of claim 17 or 18.

20. The preventive or therapeutic agent of claim 19, wherein the age-related disease or symptom is at least one selected from the group consisting of bone deformation, osteoporosis, Paget's disease of bone, hyperparathyroidism, decreased bone mineral density, cancellous transformation of cortical bone, hair loss, tissue injury or necrosis, tumor, breast hypertrophy, ascites, anemia, bleeding, aging of skin, and aging of nails.

21. An agent that inhibits osteoclast function, wherein the agent comprises the protein of claim 16, or the peptide of claim 17 or 18.

22. An antibody that binds to the protein of claim 16, or to the peptide of claim 17 or 18.
